# EUROPEAN PATENT SPECIFICATION

(11) **EP 4 225 128 B1**
(45) Date of publication and mention of the grant of the patent: **07.01.2026**
(21) Application number: 21798945.8
(22) Date of filing: 07.10.2021
(51) Int. Cl.: A61B 3/08, A61B 3/113, A61B 5/16, A61B 3/00, A61B 5/11

(54) **FUNCTIONAL DIFFERENTIAL EXPLORATION AND REHABILITATION OF BINOCULAR MOTOR COORDINATION IN INDIVIDUALS SUFFERING FROM LEARNING DISORDERS**
FUNKTIONALE DIFFERENZIELLE EXPLORATION UND REHABILITIERUNG VON BINOKULARER BEWEGUNGSKOORDINATION BEI PERSONEN MIT LERNSTÖRUNGEN
EXPLORATION DIFFÉRENTIELLE FONCTIONNELLE ET RÉHABILITATION DE LA COORDINATION MOTRICE BINOCULAIRE CHEZ DES PERSONNES SOUFFRANT DE TROUBLES DE L'APPRENTISSAGE

(30) Priority: 07.10.2020 EP 20306164
(43) Date of publication of application: 16.08.2023
(73) Proprietor: CENTRE NATIONAL DE LA RECHERCHE SCIENTIFIQUE (CNRS), 75016 Paris (FR); Université Paris Cité, 75006 Paris (FR)
(72) Inventor: KAPOULA, Zoi, 75015 PARIS (FR)
(74) Representative: Hautier IP
(86) International application number: PCT/EP2021/077718
(87) International publication number: WO 2022/074130

(56) References cited:
- WO-A1-2014/083281
- WO-A1-2015/120438
- US-A1- 2017 295 353
- BUCCI M P ET AL: "Binocular coordination of saccades in children with vertigo: Dependency on the vergence state", VISION RESEARCH, ELSEVIER, AMSTERDAM, NL, vol. 46, no. 21, 1 October 2006 (2006-10-01), pages 3594 - 3602, XP025010124, ISSN: 0042-6989, [retrieved on 20061001], DOI: 10.1016/J.VISRES.2006.06.001
- STEPHANIE JAINTA ET AL: "Dyslexic Children Are Confronted with Unstable Binocular Fixation while Reading", PLOS ONE, 6 April 2011 (2011-04-06), pages 1 - 9, XP055022200, Retrieved from the Internet <URL:www.plosone.org/article/fetchObjectAttachment.action?uri=info%3Adoi%2F10.1371%2Fjournal.pone.0018694&representation=PDF> [retrieved on 20120319], DOI: 10.1371/journal.pone.0018694

## Description

### TECHNICAL FIELD OF THE INVENTION

The present invention is in the field of learning disorders such as dyslexia, and more particularly to the functional exploration and rehabilitation of ocular motor coordination in such individuals. It relates to a method for measuring binocular motricity parameters in ecologic conditions (both eye viewing targets displaced in the real 3D space) as well as during reading for persons suffering from a learning disorder comprising multiple ocular motor tests (at least a reading test, a vergence test and a saccade test), wherein the vergence test and the saccade test are performed using a device physically stimulating vergences or saccades in the real world in three dimensions and contain multiple trials of vergence or saccade stimulation. It further relates to a method for treating such intrinsic ocular motor abnormalities in an individual suffering from a learning disorder.

### BACKGROUND ART

Dyslexia is a reading disorder that emerges in childhood. The primary deficit involves problems with word recognition, decoding words, and spelling, which translates into slower reading, decreased comprehension, and even difficulty writing (Raghuram, A. et al. JAMA Ophthalmol 136, 1089-1095, doi:10.1001/jamaophthalmol.2018.2797 (2018)). Traditionally, dyslexia has been viewed as a primary problem related to a linguistic deficit in phonological processing. The traditional therapy has therefore been educational rehabilitation and learning accommodation (e.g. use of computers for home or school work). However, there have been many studies that have shown dyslexic adolescents do have evidence of abnormal visual processing, including abnormal saccadic and vergence eye movements (Raghuram, A. et al. JAMA Ophthalmol 136, 1089-1095, doi:10.1001 /jamaophthalmol.2018.2797 (2018) ; Bucci, M. P., et al. PLoS One 7, e33458, doi:10.1371/journal.pone.0033458 (2012) ; Bucci, M. P., et al. Exp Brain Res 188, 1-12, doi:10.1007/s00221-008-1345-5 (2008) ; Bucci, M. P., et al. Graefes Arch Clin Exp Ophthalmol 246, 417-428, doi:10.1007/s00417-007-0723-1 (2008); Jainta, S. & Kapoula, Z. PLoS One 6, e18694, doi:10.1371/journal.pone.0018694 (2011); Seassau, M., et al. Front Integr Neurosci 8, 85, doi:10.3389/fnint.2014.00085 (2014) ; Tiadi, A., et al. Res Dev Disabil 35, 3175-3181, doi:10.1016/j.ridd.2014.07.057 (2014)).

However, these studies are mainly clinically based and do not provide objective measurements of eye movements, but rather subjective evaluation of vergence and moreover using haploscopic conditions in which the two eyes are dissociated (via polarizers, prisms, or intermittent spectacles, virtual reality etc.). Other research studies using eye movement recording have identified deficits with binocular coordination during reading in dyslexics. Yet, there have been no differential studies that provide objective binocular measurements of saccades and vergences eye movements in the real space vs binocular eye movements in reading in the dyslexic population to determine if these deficits are due to a motor problem or if they are the consequence of poor reading.

Moreover, conclusions found in the prior art concerning abnormal saccades during reading for dyslexic individuals are conflicting, maybe due to the use of variable and inappropriate testing methods.

The same is true of learning disorders related to other developmental disorders, attention deficits/hyperactivity disorders, or any neurologic disorder affecting reading in which ocular motor abnormalities are clinically noticed, but it is not possible to conclude whether or not the ocular motor abnormalities are intrinsic.

Bucci MP, Kapoula Z, Brémond-Gignac D, Wiener-Vacher S. Binocular coordination of saccades in children with vertigo: dependency on the vergence state. Vision Res. 2006 Oct;46(21):3594-602, describes an evaluation of the binocular coordination of saccades in children with symptoms of vertigo headache and equilibrium disorder.US2017/295353 A1 discloses the impact of 3D stereoscopic image capture and display on dynamic depth distortion in stereo image motion, as well as systems and methods to reduce such distortions and related instabilities.

Jainta S, Kapoula Z. Dyslexic Children Are Confronted with Unstable Binocular Fixation while Reading. PLoS ONE 2011, 6(4): e18694 discuss binocular instability in dyslexic children.

There is thus a need for methods in which the intrinsic ocular motor abnormalities of dyslexic individuals, and more generally of individuals suffering from a learning or neurologic disorder, may be detected and treated in order to improve learning reading and quality of life. When no intrinsic ocular motor abnormality is detected, the clinical management of the individual will also be improved, since other types of treatment will be prescribed.

### SUMMARY OF THE INVENTION

In the context of the present invention, the inventors have measured various binocular coordination parameters, including those of vergence and saccade eye movement parameters, for dyslexic adolescent individuals one third of them presenting other comorbidities, e.g. they were diagnosed by reference medical centers in Paris for dyslexia and many of them presenting other comorbidities such as dysorthographia, dyscalculia, and/or dyspraxia. Seventy eight per cent of them had been following orthoptic clinical visual treatment and continuous speech therapy since many years; those persons were submitted to several ocular motor tests, including a reading test, at least a vergence test and at least a saccade test, the vergence test(s) and the saccade test(s) being performed using the REMOBI^{®} device (see WO2011073288A1), which is a binocular motor stimulation device configured to specifically and physically stimulate vergences and saccades, comprising visual or audiovisual targets placed in real space at eccentricities and depths of a surface, and allows the measurement of intrinsic ocular motor abnormalities.

They found that, compared to non-dyslexic individuals, dyslexic individuals showed ocular motor abnormalities during reading (see Example 1), and also during vergence and saccade tests (see Example 2).

During reading dyslexics performed many more backward saccades fixating the same word several times, the speed of reading (number of words measured per minute) was significantly lower and fixation durations were longer. Reading saccades also had abnormal velocity profile, e.g., lower average velocity, longer duration and the two eyes were poorly coordinated, showing increased disconjugacy during the saccade (see Example 1).

During vergence and saccade tests, the results showed statistically significant abnormalities in vergence and saccades. In vergence, dyslexics displayed a reduced amplitude of the 160 ms component of the convergence that follows an initial phasic component and a longer duration in the initial phasic component of divergence. In saccades, dyslexic adolescents demonstrated execution slowness' e.g. longer duration for both left and right saccades. They also showed increased deconjugate drift (convergent or divergent drifts of the eyes) in the first 80 or 160 ms of the fixation following saccades to the right, suggesting poor binocular coordination. For all movements, convergence, divergence, saccades to right or saccades to the left, the peak velocity which is achieved early during the acceleration phase of the movement, was significantly higher in dyslexics. In contrast, the average velocity was slower for dyslexics meaning that the deceleration phase of the movements was slower in dyslexics. Thus, these results indicate peculiar velocity profiles in dyslexics, particularly a slow deceleration phase in both vergence and saccades (see Example 2).

Moreover, using several distinct machine learning algorithms, they found in preliminary experiments that a general population of individuals comprising both healthy (non-dyslexic) and dyslexic individuals could be classified into two groups of non-dyslexic and dyslexic individuals based on vergence and saccade parameters measured in saccade and vergence tests using the REMOBI device with significant sensitivity and specificity, further confirming the relevance of measuring saccade and vergence parameters in saccade and vergence test as an aid for diagnosis and management of dyslexic individuals (see Example 3).

They further found that, contrary to healthy individuals, dyslexic individuals do not perform better in vergence and saccade tests when audiovisually stimulated rather than when being stimulated only visually (see Example 4).

When the individual further had an accelerometer fixed to his/her head, they further found increased head motion accompanying the eye movements in saccades and vergence tests or during reading or (see Example 5).

They also found that submitting dyslexic individuals to vergence training sessions with the use of audio-visual double step vergence paradigm and the REMOBI device permits to improve abnormal intrinsic vergence and saccade parameters, which further results in improved reading speed (see Example 6).

Finally, the same approach was tested on other individuals suffering from distinct learning disorders (dyspraxia, dysphasia) and found to be also useful in these individuals (see Example 7).

On this basis, the invention provides new methods improving the clinical management of binocular eye movement problems in dyslexic individuals, and more generally individuals suffering from learning disorders, by the measure of binocular motricity parameters specifically altered in most dyslexic individuals in ocular motor tests including not only a reading test but also vergence and saccade tests, the detection of intrinsic vergence and/or saccade abnormalities and the rehabilitation of individuals with such intrinsic vergence and/or saccade abnormalities using vergence training sessions with the double step vergence method.

In a first aspect, the present invention thus relates to a method for measuring binocular motricity parameters in an individual suffering from a learning disorder, comprising:
a1) submitting the individual to ocular motor tests comprising at least a reading test, a vergence test and a saccade test;
b1) recording the eye movements of the left eye and the right eye of the individual during each of the ocular motor tests; and
c1) calculating, using a digital processing device, the value of at least one binocular coordination parameter from the records obtained in step b1);

wherein the vergence test and the saccade test are conducted using a binocular motor stimulation device configured to specifically and physically stimulate vergences and saccades, comprising visual or audiovisual targets placed in real space at eccentricities and depths of a surface;
wherein the vergence test comprises 30 to 50 trials of:
   - visually and/or audiovisually stimulating the individual at a point located at the eyes level, in the central axis between the left eye and the right eye, at a first distance, and
   - visually and/or audiovisually stimulating the individual at another point located at the eyes level, in the central axis between the left eye and the right eye, at another distance calling for a convergence movement or a divergence movement, half of the trials calling for a convergence movement, the other half for a divergence movement, the trials calling for convergence and divergence movements being interleaved; and
wherein the saccade test comprises 30 to 50 trials of:
   - visually and/or audiovisually stimulating the individual at a point located at the eye's level, in the central axis between the left eye and the right eye, and
   - visually and/or audiovisually stimulating the individual at another point located at the eye's level, on the left or on the right of the previous point, half of the trials being on the left, the other half on the right, the trials on the left and on the right being interleaved.

The present invention also relates to a method for detecting intrinsic ocular motor abnormalities in an individual suffering from a learning disorder, comprising:
a2) providing previously obtained recordings of the eye movements of the left eye and the right eye of the individual during ocular motor tests comprising at least a reading test, a vergence test and a saccade test,
b2) calculating, using a digital processing device, the value(s) of at least one binocular coordination parameter from the records provided in step a2);
c2) comparing the value(s) calculated in step b2) to one or more reference value(s) obtained in one or more individual(s) not suffering from a learning disorder; and
d2) concluding to the presence of intrinsic ocular motor abnormalities in the individual suffering from a learning disorder if at least one binocular coordination parameter calculated in step b2) is significantly different from the one or more reference value(s) obtained in one or more individual(s) not suffering from a learning disorder;

wherein the vergence test and the saccade test from which the recordings are provided in step a2) have been conducted using a binocular motor stimulation device configured to specifically and physically stimulate vergences and saccades, comprising visual or audiovisual targets placed in real space at eccentricities and depths of a surface;
wherein the vergence test which recording is provided comprises 30 to 50 trials of:
   - visually and/or audiovisually stimulating the individual suffering from a learning disorder at a point located at the eyes level, in the central axis between the left eye and the right eye, at a first distance, and
   - visually and/or audiovisually stimulating the individual suffering from a learning disorder at another point located at the eyes level, in the central axis between the left eye and the right eye, at another distance calling for a convergence movement or a divergence movement, half of the trials calling for a convergence movement, the other half for a divergence movement, the trials calling for convergence and divergence movements being interleaved; and
wherein the saccade test which recording is provided comprises 30 to 50 trials of:
   - visually and/or audiovisually stimulating the individual suffering from a learning disorder at a point located at the eyes level, in the central axis between the left eye and the right eye, and
   - visually and/or audiovisually stimulating the individual suffering from a learning disorder at another point located at the eyes level, on the left or on the right of the previous point, half of the trials being on the left, the other half on the right, the trials on the left and on the right being interleaved.

This method may be used by clinicians as a diagnostic aid.

The present invention also relates to a method for treating intrinsic ocular motor abnormalities in an individual suffering from a learning disorder, comprising:
a3) submitting the individual to ocular motor tests comprising at least a reading test, a vergence test and a saccade test;
b3) recording the eye movements of the left eye and the right eye of the individual during each of the ocular motor tests; and
c3) calculating, using a digital processing device, the value of at least one binocular coordination parameter from the records obtained in step b);
d3) comparing the value(s) calculated in step b3) to one or more reference value(s) obtained in one or more individual(s) not suffering from a learning disorder;
e3) concluding to the presence of intrinsic ocular motor abnormalities in the individual if at least one binocular coordination parameter calculated in step b3) is significantly different from the one or more reference value(s) obtained in one or more individual(s) not suffering from a learning disorder,
f3) submitting the individual to vergence training sessions once or twice a week between 2 to 6 weeks; and
g3) repeating steps a3) to e3), and optionally step f3), until some improvement in the intrinsic ocular motor abnormalities of the individual suffering from a learning disorder is found;

wherein the vergence test and the saccade test are conducted using a binocular motor stimulation device configured to specifically and physically stimulate vergences and saccades, comprising visual or audiovisual targets placed in real space at eccentricities and depths of a surface;
wherein the vergence test comprises 30 to 50 trials of:
   - visually and/or audiovisually stimulating the individual suffering from a learning disorder at a point located at the eyes level, in the central axis between the left eye and the right eye, at a first distance, and
   - visually and/or audiovisually stimulating the individual suffering from a learning disorder at another point located at the eyes level, in the central axis between the left eye and the right eye, at another distance calling for a convergence movement or a divergence movement, half of the trials calling for a convergence movement, the other half for a divergence movement, the trials calling for convergence and divergence movements being interleaved; and
wherein the saccade test comprises 30 to 50 trials of:
   - visually and/or audiovisually stimulating the individual suffering from a learning disorder at a point located at the eyes level, in the central axis between the left eye and the right eye, and
   - visually and/or audiovisually stimulating the individual suffering from a learning disorder at another point located at the eyes level, on the left or on the right of the previous point, half of the trials being on the left, the other half on the right, the trials on the left and on the right being interleaved; and
wherein each vergence training session comprises 1 or 2 repetitions of 1 or 2 block(s) of divergence followed by 3 to 5 blocks of convergence, each block comprising 30 to 50 trials all using the double step vergence paradigm of:
   - visually and/or audiovisually stimulating the individual suffering from a learning disorder at a first point located at the eyes level, in the central axis between the left eye and the right eye for a period varying from 800 to 1800 ms, preferably 1000 to 1600 ms, at a first distance,
   - visually and/or audiovisually stimulating the individual suffering from a learning disorder at a second point located at the eyes level, in the central axis between the left eye and the right eye for a period of 150 to 250 ms , preferably 175 to 225 ms, such as 200 ms, at a second distance calling for a convergence movement in convergence blocks or a divergence movement in divergence blocks, and
   - visually and/or audiovisually stimulating the individual suffering from a learning disorder at a third point located at the eyes level, in the central axis between the left eye and the right eye for a period varying from 800 to 1800 ms, preferably 1000 to 1600 ms, at a third distance calling for a further convergence movement in convergence blocks or a further divergence movement in divergence blocks.

### DESCRIPTION OF THE FIGURES

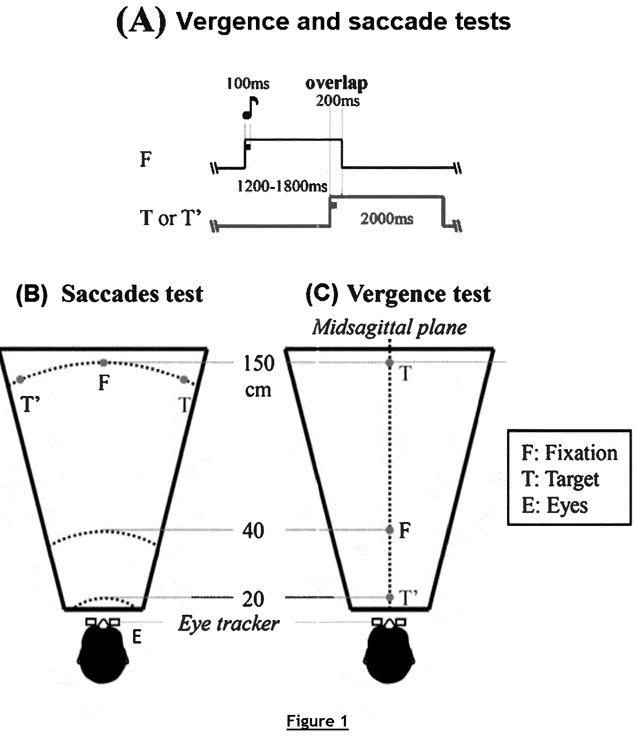

**Figure 1A, 1B & 1C****:** Vergence and saccade tests experiment Set-Up. Temporal (A) and spatial **(B-C)** arrangements of the REMOBI vergence (A, B) and saccade (A, C) tests. The targets were randomly interleaved in order to test vergence (B) or saccades (C) eye movements in different blocks using the temporal paradigm as shown in (A). Individuals looked successively at different LEDs; from the initial (40 cm) fixation LED (F), to the target LED for divergence (150 cm, T) or the target LED for convergence (20 cm, T') (B); and from the initial (40 cm) fixation LED to the target LED for saccades (20° to left to target T or to the right to target T') (C). Each trial starts with the fixation target that appears for a variable period of 1200 to 1800 ms; following this period the target LED lights are on for 2000ms together with a paired buzzer preceding 50 ms and lasting only 100ms.

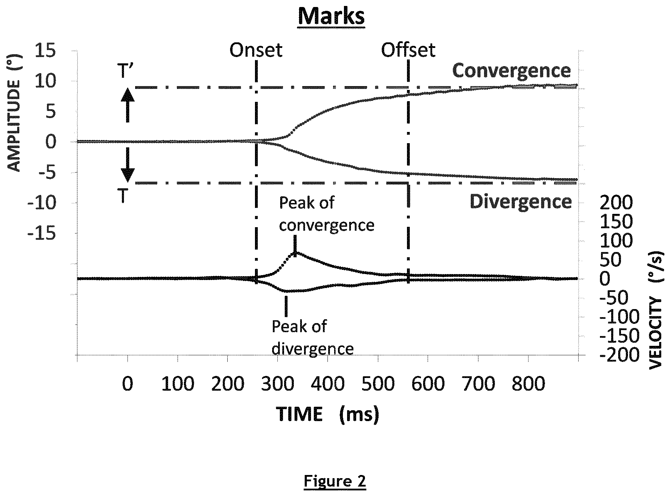

**Figure 2****.** Method for vergence analysis: convergence and divergence were obtained by subtraction of right eye (RE) position from the left eye (LE) position (LE - RE). The corresponding velocity trace is shown *below.* Mark of onset and offset of vergence is based on velocity criteria: the time point when the eye velocity respectively exceeded or dropped below 5° /s. The *horizontal dotted lines* indicate the targets' (T for divergence, T' for convergence) position.

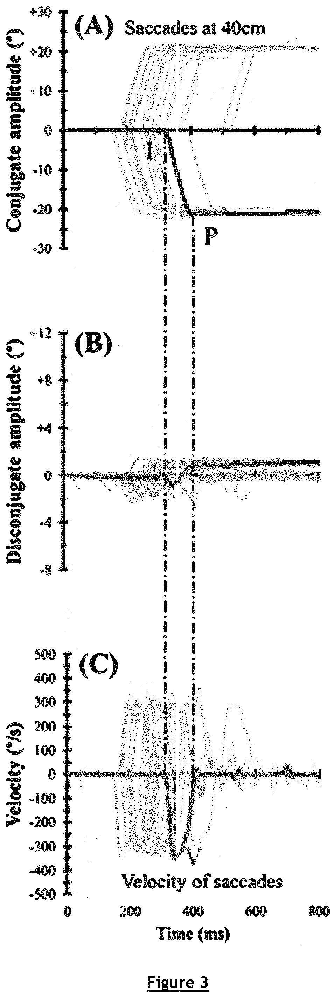

**Figure 3****.** Method for saccade analysis, illustrated for saccades of 20° in an individual with vergence disorders (TC). All trials are superimposed at t = 0, the instant of target appearance. The bold lines show an individual trial of saccade to the left (A, B): Onset (I) and offset (P) of eye movements were defined according to velocity threshold (V), traces of velocity being illustrated at the bottom (C). Note that coordination of saccades may be evaluated from the disconjugate amplitude (B).

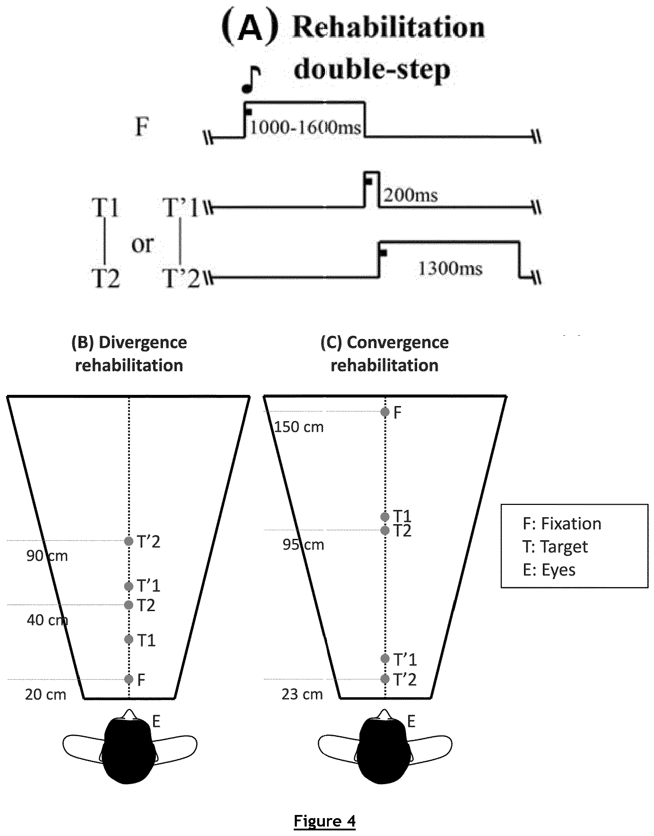

**Figure 4****.** Vergence rehabilitation experiment Set-Up. Temporal (A) and spatial (B, C) arrangements of REMOBI divergence (B) and convergence (C) training blocks. Double-step targets used for divergence and convergence rehabilitation are shown in (B, C) from F to T1 or T2, the first target location; then to T'1 or T'2, the final target location.

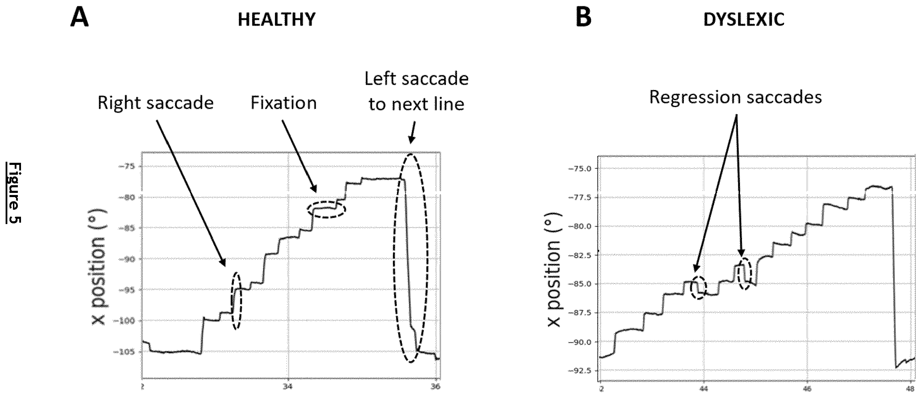

**Figure 5****.** Exemplary trajectories of the eye movements of a healthy (non-dyslexic, A) and a dyslexic (B) individual during reading of one line of text. In this figure, on the x-axis we have time; in y the conjugate signal of both eyes in degrees, showing the succession of saccades to the right followed by fixations fixing the words one after the other, then the large saccade to the left to start reading the next line. Regression saccades are further present for the dyslexic individual.

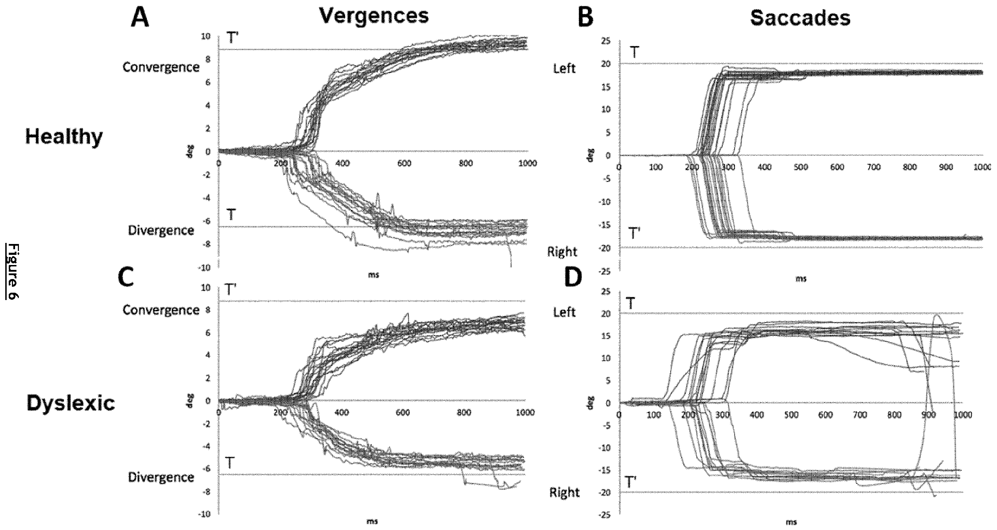

**Figure 6****. Example of Individual Vergence and Saccade Trials in healthy and dyslexic individuals.** Trajectories of vergence and saccades in an individual healthy control (A,B) and dyslexic (C,D) individual in a vergence and a saccade test. Despite initiating vergence movements quickly towards the target (grey line marked T or T' above and below the x-axis), the dyslexic individual takes longer to reach the vergence target (indeed, longer than measured). Similar difficulties can be seen in the saccade trial (B,D).

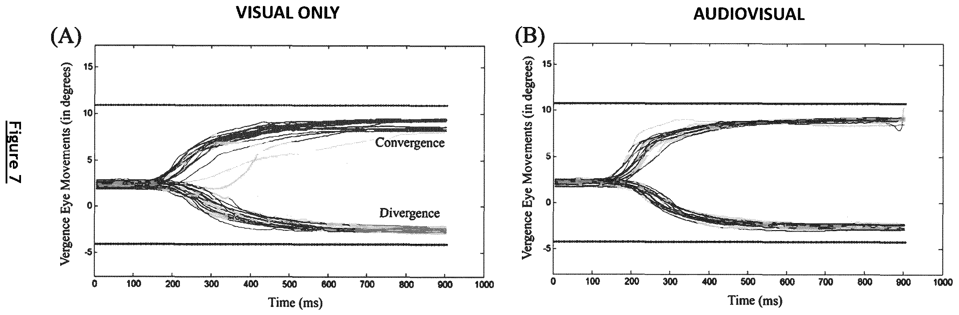

**Figure 7****.** Exemplary vergence trajectories of a non-dyslexic individual after visual stimulation only (A) or audiovisual stimulation (B).

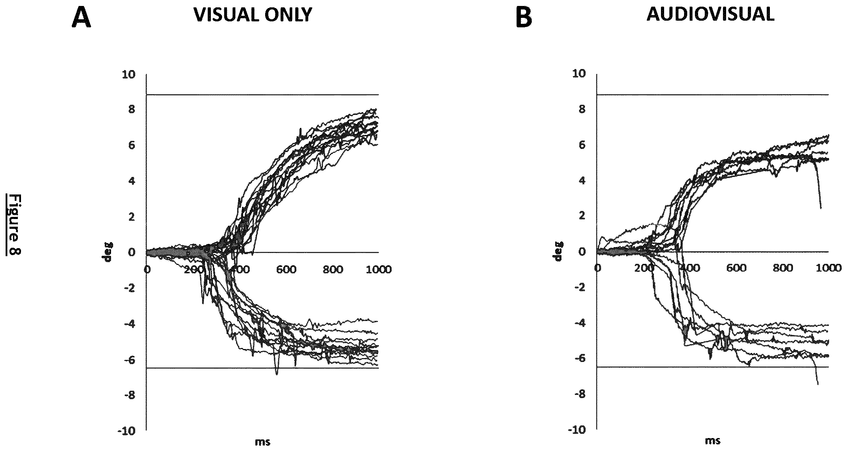

**Figure 8****.** Exemplary vergence trajectories of a dyslexic individual after visual stimulation only (A) or audiovisual stimulation (B).

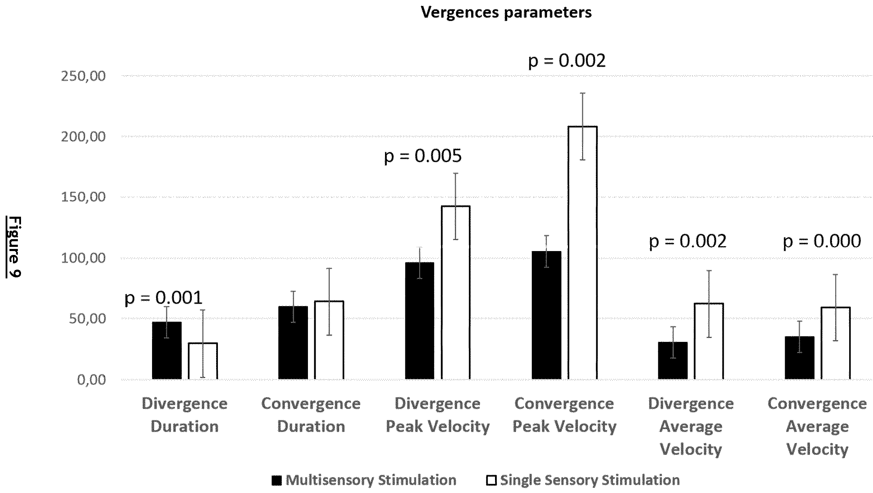

**Figure 9****.** Vergence parameters after visual stimulation only (single sensory stimulation) or audiovisual stimulation (multisensory stimulation) in dyslexic individuals.

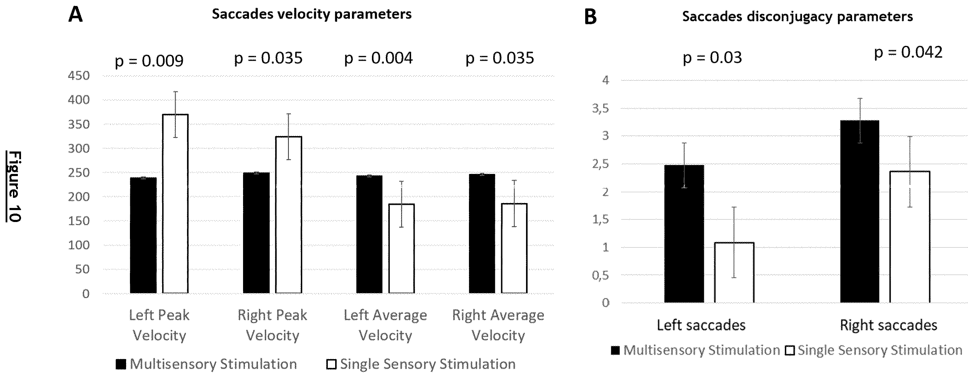

**Figure 10****.** Saccade parameters after visual stimulation only (single sensory stimulation) or audiovisual stimulation (multisensory stimulation) in dyslexic individuals. (A) Saccades velocity parameters. (B) Saccades disconjugacy parameters.

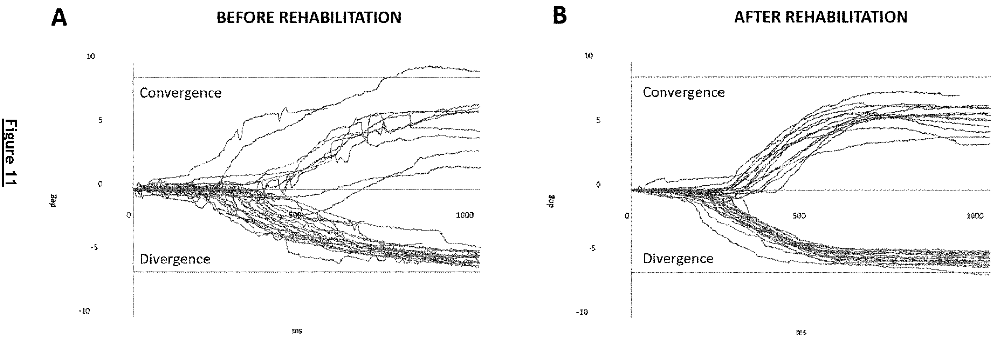

**Figure 11****.** Exemplary vergence trajectories of a first dyslexic individual before (A) and after (B) two vergence training sessions using REMOBI and the vergence double step paradigm.

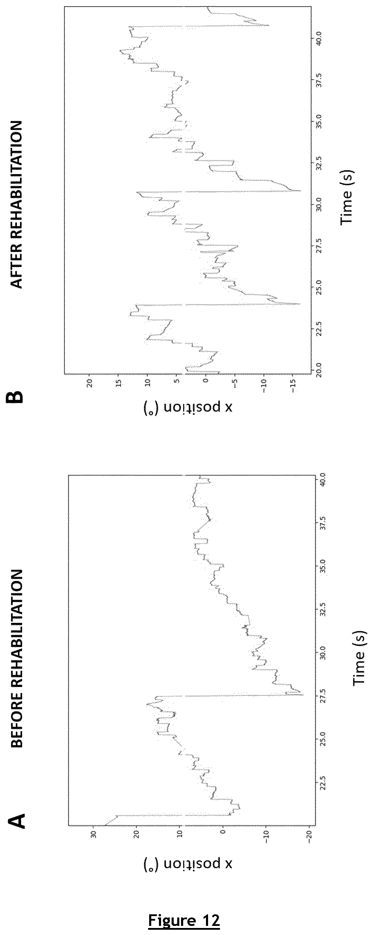

**Figure 12****.** Exemplary curves of the eyes movements of a dyslexic individual before (A) and after (B) two vergence training sessions using REMOBI and the vergence double step paradigm.

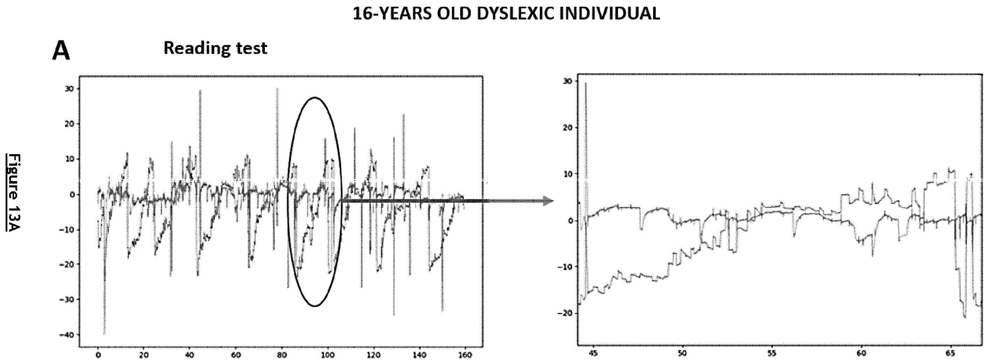

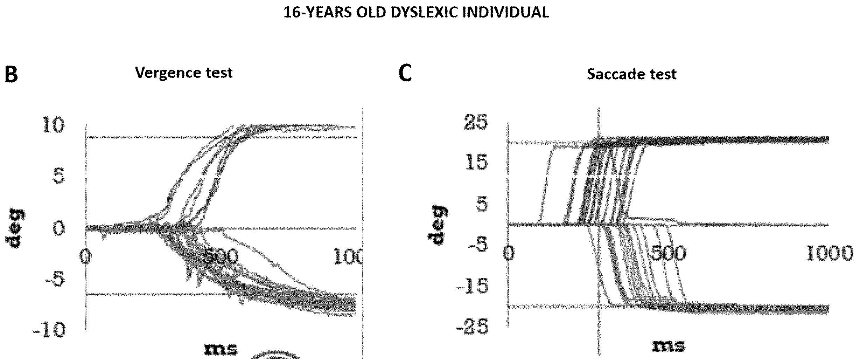

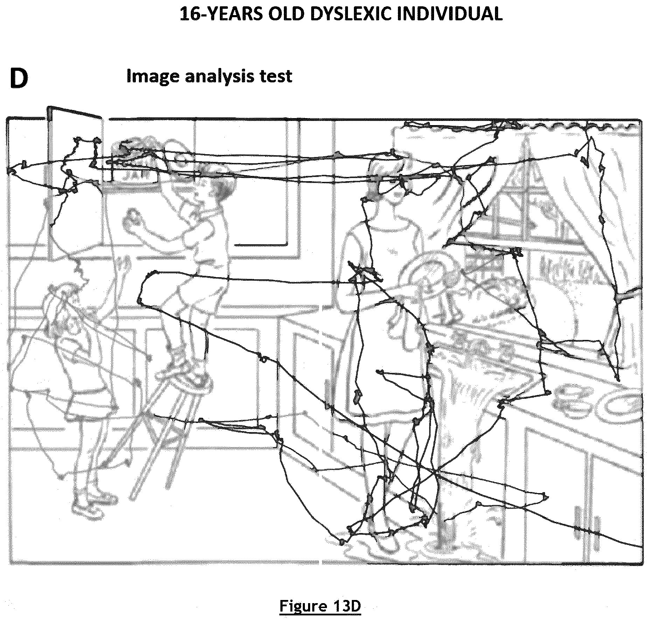

**Figure 13****.** Functional exploration of a 16-years old dyslexic individual using a vergence test, a saccade test, a reading test and an image analysis test. (A) Curves of the eyes movements during reading. In this figure, on the x-axis we have time; in y the conjugate signal of both eyes in degrees, showing the succession of saccades to the right followed by fixations fixing the words one after the other, then the large saccade to the left to start reading the next line. (B) Vergence trajectories during a vergence test. (C) Saccades trajectories during a saccade test. (D) Eyes movements during an image analysis test.

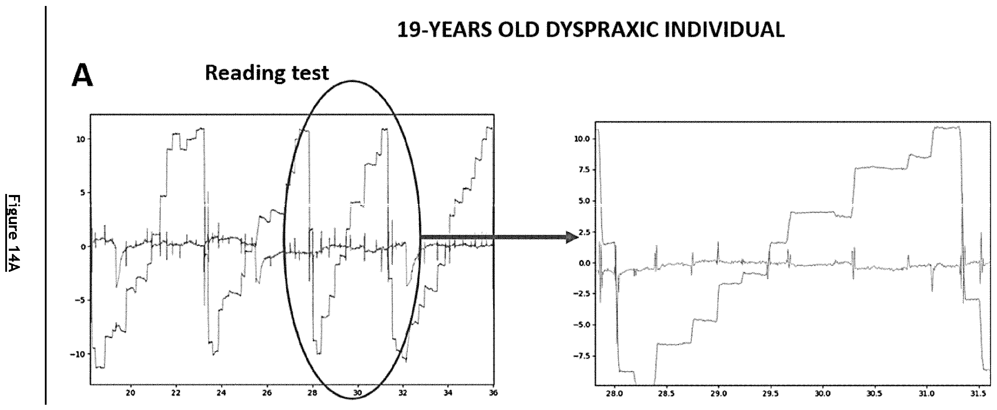

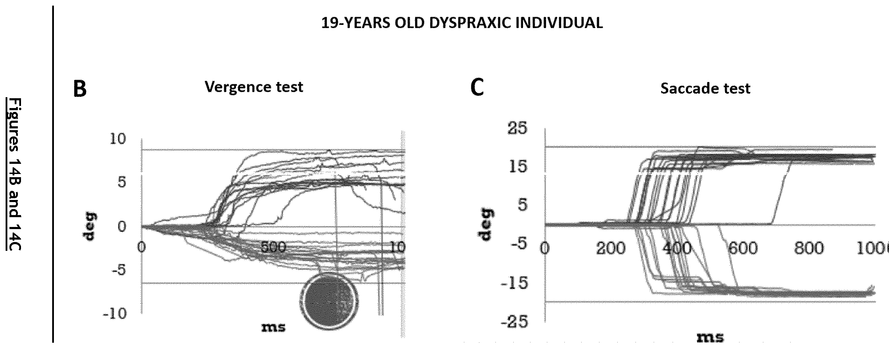

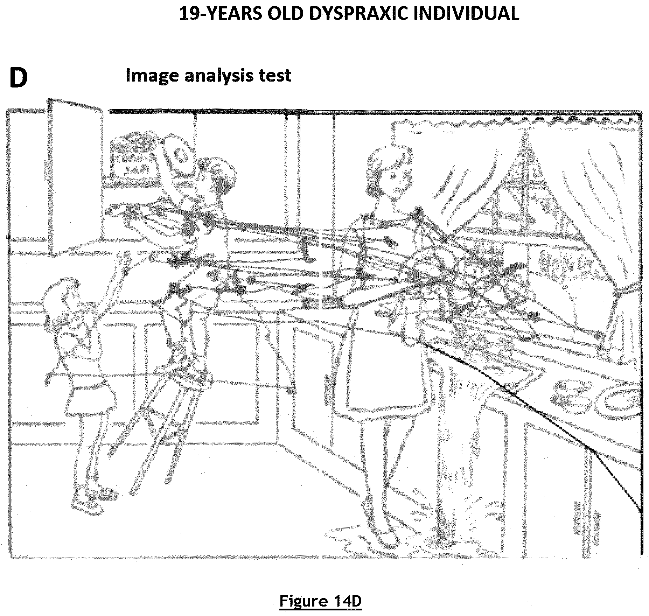

**Figure 14****.** Functional exploration of a 19-years old dyspraxic individual using a vergence test, a saccade test, a reading test and an image analysis test. (A) Curves of the eyes movements during reading. In this figure, on the x-axis we have time; in y the conjugate signal of both eyes in degrees, showing the succession of saccades to the right followed by fixations fixing the words one after the other, then the large saccade to the left to start reading the next line. (B) Vergence trajectories during a vergence test. (C) Saccades trajectories during a saccade test. (D) Eyes movements during an image analysis test.

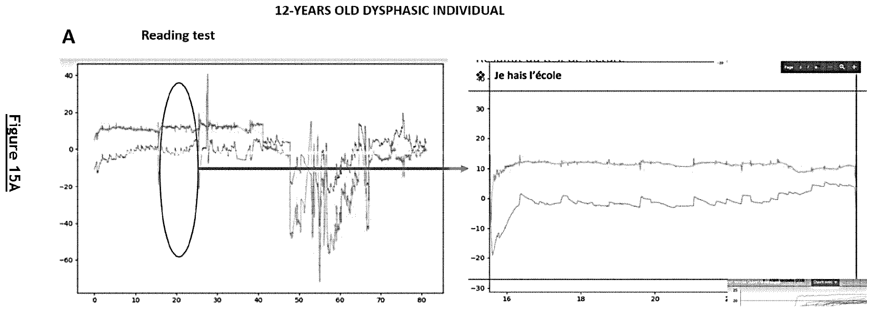

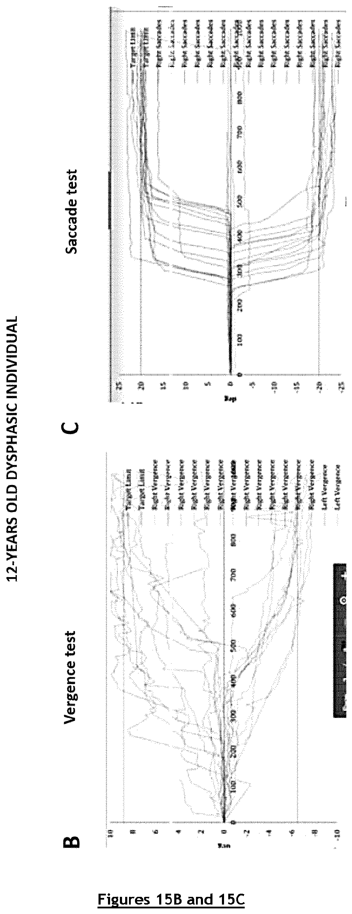

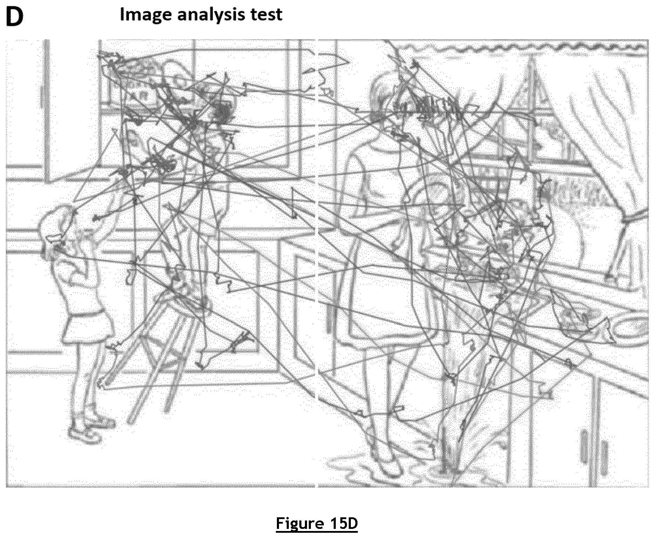

**Figure 15****.** Functional exploration of a 12-years old dysphasic individual using a vergence test, a saccade test, a reading test and an image analysis test. (A) Curves of the eyes movements during reading. In this figure, on the x-axis we have time; in y the conjugate signal of both eyes in degrees, showing the succession of saccades to the right followed by fixations fixing the words one after the other, then the large saccade to the left to start reading the next line. (B) Vergence trajectories during a vergence test. (C) Saccades trajectories during a saccade test. (D) Eyes movements during an image analysis test.

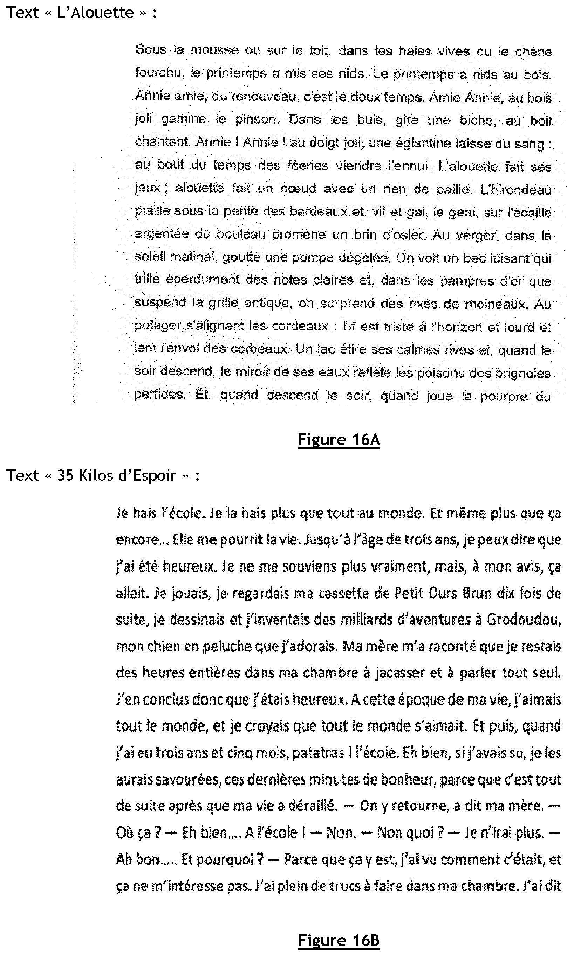

**Figure 16****.** Reference texts useful for the reading test. (A) L'Alouette text. (B) « 35 kg d'espoir » text.

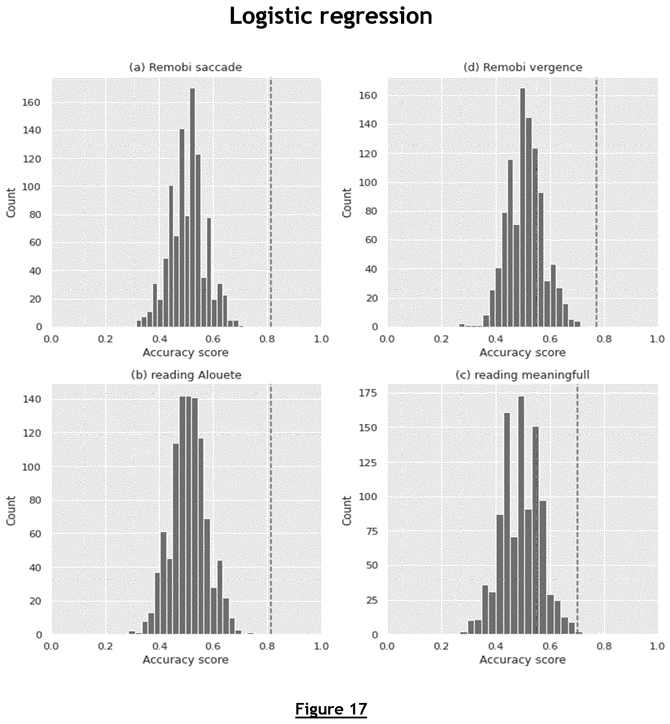

**Figure 17****.** Logistic regression test permutation histograms for Analysis 1, the red line indicated the reported score. (a) the histogram of the Remobi saccade dataset. (b) the histogram of the Remobi vergence dataset. (c) the histogram of the reading Alouette, (d) the histogram of the reading meaningful.

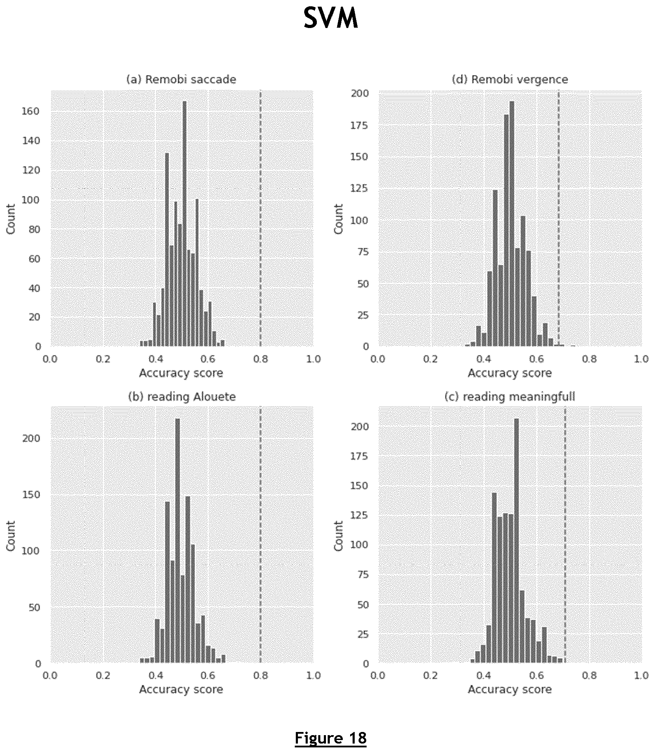

**Figure 18****.** SVM test permutation histograms for Analysis 1; the red line indicates the reported score. (a) The histogram of the Remobi saccade dataset, (b) the histogram of the Remobi vergence dataset, (c) the histogram of the reading Alouette, (d) the histogram of the reading meaningful.

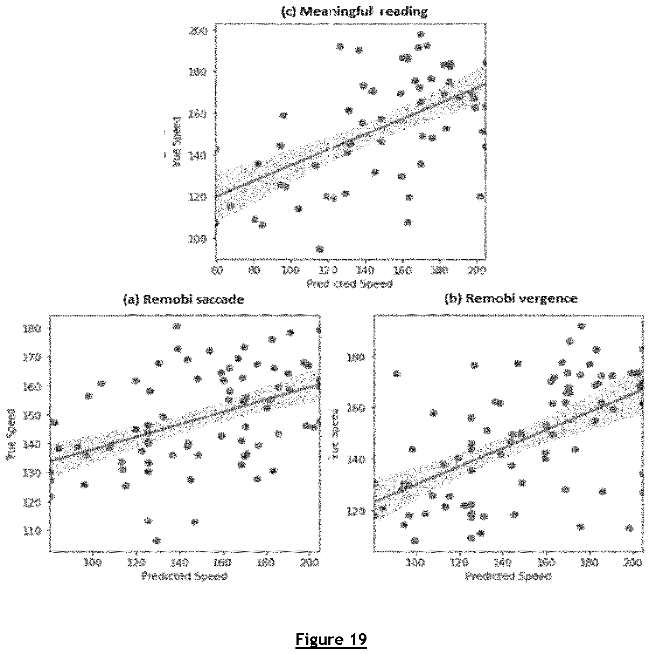

**Figure 19****.** Correlation between true speed vs. predicted speed of the three tasks for the analysis 2: (a) Remobi saccade. (b) Remobi vergence. (c) Meaningful lecture.

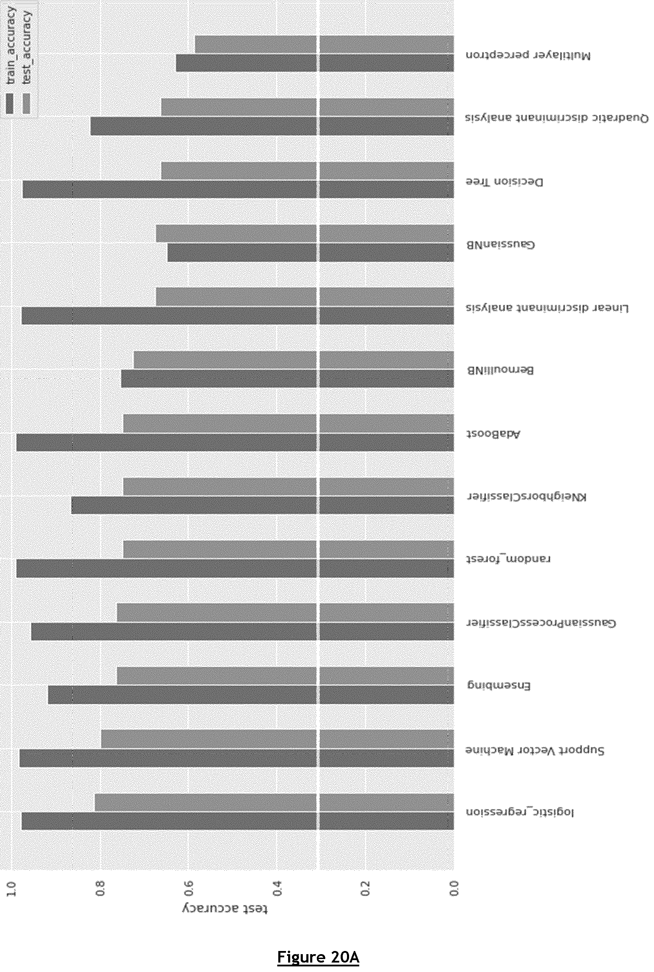

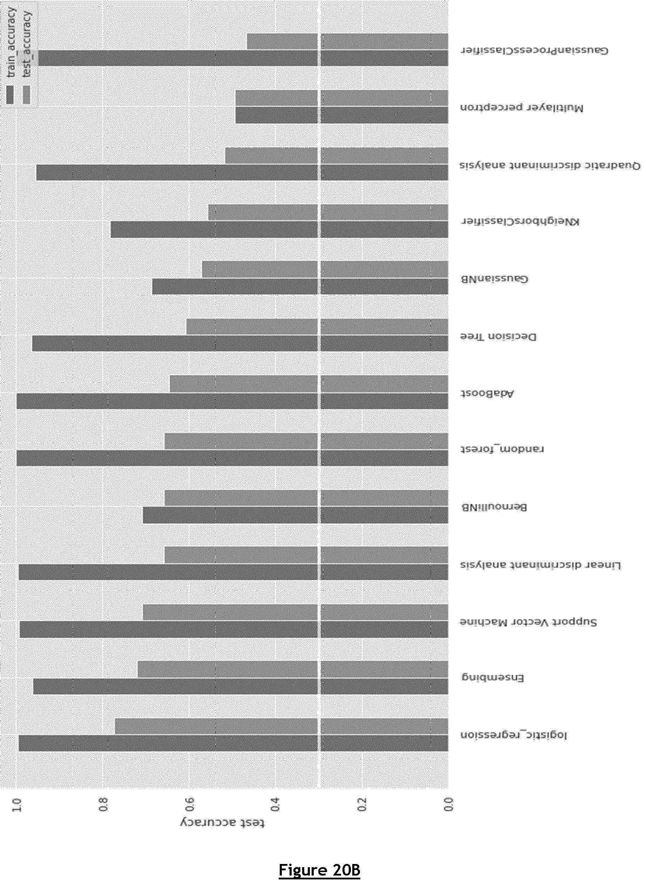

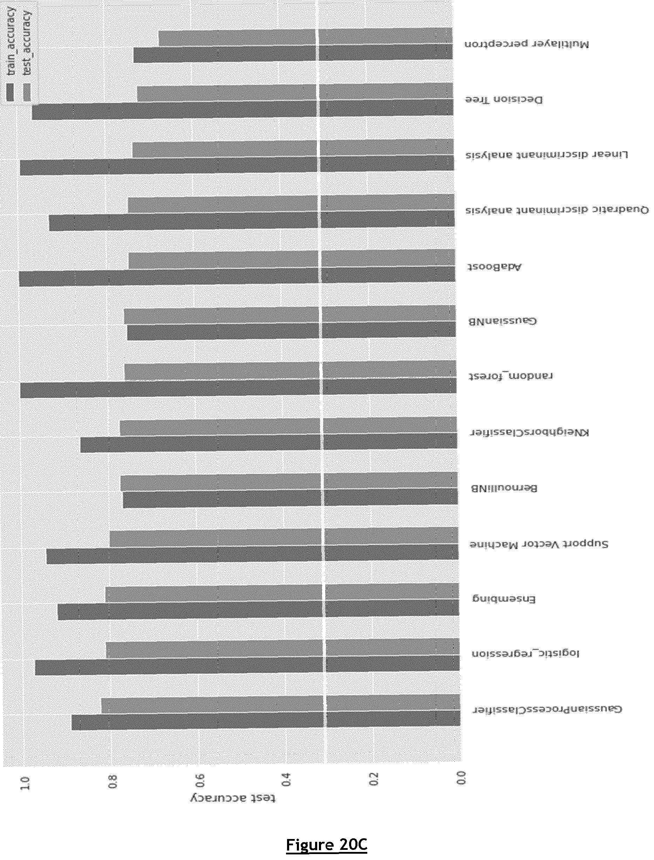

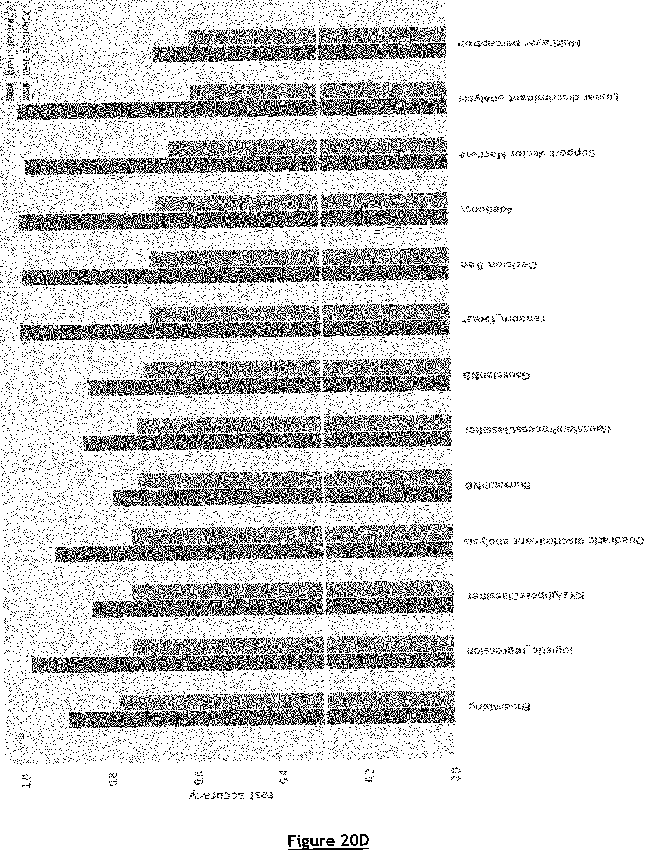

**Figure 20****.** (A) Pair plot of all the score of the analysis 1-Remobi saccade dataset. (B) Pair plot of all the score of the analysis 1-Remobi vergence dataset. (C) Pair plot of all the score of the analysis 1-Remobi Alouette reading dataset. (D) Pair plot of all the score of the analysis 1-Remobi Meaningful reading dataset.

### DETAILED DESCRIPTION OF THE INVENTION

In the context of the present invention, the inventor have measured various binocular coordination parameters, including vergence and saccade parameters, for dyslexic individuals submitted to several ocular motor tests, including a reading test, at least one vergence test and at least one saccade test, the vergence test(s) and the saccade test(s) being performed using the REMOBI^{®} device (see WO2011073288A1), which is a binocular motor stimulation device configured to specifically and physically stimulate vergences and saccades, comprising visual or audiovisual targets placed in real space at eccentricities and depths of a surface, and allows the measurement of intrinsic ocular motor abnormalities.

They found that, compared to non-dyslexic individuals, dyslexic individuals showed ocular motor abnormalities during reading (see Example 1), and also during vergence and saccade tests (see Example 2).

During reading dyslexics performed many more backward saccades fixating the same word several times, the speed of reading (number of words measured per minute) was significantly lower and fixation durations were longer. Reading saccades, also had abnormal velocity profile, e.g., lower average velocity, longer duration and their eyes were poorly coordinated, showing increased disconjugacy during the saccade (see Example 1).

During vergence and saccade tests, the results showed statistically significant abnormalities in vergence and saccades. In vergence, dyslexics displayed a reduced amplitude of the 160 ms component of convergence that follows an initial phasic component and a longer duration in the initial phasic component of divergence. In saccades, dyslexic adolescents demonstrated execution slowness' e.g. longer duration for both left and right saccades. They also showed increased deconjugate drift (convergent or divergent drifts of the eyes) in the first 80 or 160 ms of the fixation following saccades to the right, suggesting poor binocular coordination. For all movements, convergence, divergence, saccades to right or saccades to the left, the peak velocity which is achieved early during the acceleration phase of the movement, was significantly higher in dyslexics. In contrast, the average velocity was slower for dyslexics meaning that the deceleration phase of the movements was slower in dyslexics. Thus, these results indicate peculiar velocity profiles in dyslexics, particularly a slow deceleration phase in both vergence and saccades (see Example 2).

Moreover, using several distinct machine learning algorithms, they found in preliminary experiments that a general population of individuals comprising both healthy (non-dyslexic) and dyslexic individuals could be classified into two groups of non-dyslexic and dyslexic individuals based on vergence and saccade parameters measured in saccade and vergence tests using the REMOBI device with significant sensitivity and specificity, further confirming the relevance of measuring saccade and vergence parameters in saccade and vergence test as an aid for diagnosis and management of dyslexic individuals (see Example 3).They further found that, contrary to healthy individuals, dyslexic individuals do not perform better in vergence and saccade tests when audiovisually stimulated rather than when being stimulated only visually (see Example 4).

When the individual further had an accelerometer fixed to his/her head, they further found increased head motion accompanying the eye movements in saccades and vergence tests or during reading or (see Example 5).

They also found that submitting dyslexic individuals to vergence training sessions with the double step vergence paradigm permits to improve abnormal intrinsic vergence and saccade parameters, which further results in improved reading (see Example 6).

Finally, the same approach was tested on other individuals suffering from distinct learning disorders (dyspraxia, dysphasia) and found to be also useful in these individuals (see Example 7).

On this basis, the invention provides new methods improving the clinical management of binocular eye movements in dyslexic individuals, and more generally individuals suffering from learning disorders, by the measure of binocular motricity parameters specifically altered in most dyslexic individuals in ocular motor tests including not only a reading test but also vergence and saccade tests, the detection of intrinsic vergence and/or saccade abnormalities and the rehabilitation of individuals with such intrinsic vergence and/or saccade abnormalities using vergence training sessions with the double step vergence paradigm.

### Method for measuring binocular motricity parameters in an individual suffering from a learning disorder

In a first aspect, the present invention thus relates to a first method for measuring binocular motricity parameters in an individual suffering from a learning disorder, comprising:
a1) submitting the individual to ocular motor tests comprising at least a reading test, a vergence test and a saccade test;
b1) recording the eye movements of the left eye and the right eye of the individual during each of the ocular motor tests; and
c1) calculating, using a digital processing device, the value of at least one binocular coordination parameter from the records obtained in step b1);

wherein the vergence test and the saccade test are conducted using a binocular motor stimulation device configured to specifically and physically stimulate vergences and saccades, comprising visual or audiovisual targets placed in real space at eccentricities and depths of a surface;
wherein the vergence test comprises 30 to 50 trials of:
   - visually and/or audiovisually stimulating the individual at a point located at the eyes level, in the central axis between the left eye and the right eye, at a first distance, and
   - visually and/or audiovisually stimulating the individual at another point located at the eyes level, in the central axis between the left eye and the right eye, at another distance calling for a convergence movement or a divergence movement, half of the trials calling for a convergence movement, the other half for a divergence movement, the trials calling for convergence and divergence movements being interleaved; and
wherein the saccade test comprises 30 to 50 trials of:
   - visually and/or audiovisually stimulating the individual at a point located at the eyes level, in the central axis between the left eye and the right eye, and
   - visually and/or audiovisually stimulating the individual at another point located at the eyes level, on the left or on the right of the previous point, half of the trials being on the left, the other half on the right, the trials on the left and on the right being interleaved.

### Step a1): submitting the individual to ocular motor tests

In step a1), the individual is submitted to ocular motor tests comprising at least a reading test, a vergence test and a saccade test.

### Reading test(s)

The measure of binocular coordination parameter(s) during a reading test permits to detect the presence of ocular motor abnormalities during reading, which are known to be present in many children suffering from learning disorders, in particular in dyslexic children.

However, the presence of ocular motor abnormalities during reading may be due to intrinsic ocular motor abnormalities but also to other reasons, including language understanding difficulties making the individual going back in the text to try understanding one more time or stress associated to understanding difficulties and leading to involuntary abnormal eye movements.

Combining a reading test with vergence and saccade tests in the method according to the invention permits to conclude whether the ocular motor abnormalities during reading are or not at least partly due to intrinsic ocular motor abnormalities.

In the method for measuring binocular motricity parameters according to the invention, the reading test is preferably performed using a text of 10 to 25 lines, preferably 10 to 20 lines, or 12 to 18 lines, preferably comprising 200 to 300 words, preferably 220 to 280 words. The text may or not make sense. Standardized reading tests (using texts that do or not make sense) are available in the art.

For instance, a well-known example of a French text of 265 words that does not make sense and that is routinely used in dyslexia diagnosis by orthophonist is called "L'Alouette", which is reproduced in **Figure 16A****.**

Non sense texts testing of reading speed exist in all other languages used by speech therapists.

An example of a French text that does make sense and that may be used in methods according to the invention is an excerpt of 15 lines in black text on a white background from a children's book (35 Kilos D'Espoir, Anna Gavalda, Bayard Jeunesse), which is reproduced in **Figure 16B****.** Other similar texts (10 to 20 lines, preferably 15 lines ± 1 or 2 lines) in black text on a white background, taken from a children's book, may be used instead. In addition to its length and presentation as defined in preceding sentence, the selected text that does make sense should preferably engage emotionally the reader.

In some cases, both a text that makes sense and a text that does not make sense may be used.

If only one type of text is used for reading testing, it is preferably a text that does not make sense (for example "L'Alouette" for French-speaking individuals), as the inventors have shown that increased text difficulty due to absence of sense further destabilizes the fragile ocular control of individuals suffering from a learning disorder (see Example 8 below).

### Vergence and saccade tests

As explained above, testing the individual intrinsic ocular motricity is mandatory to be able to conclude whether ocular motor abnormalities during reading are or not at least partly due to intrinsic ocular motor abnormalities.

The vergence and saccade tests to which the individual is submitted in the invention permit to detect intrinsic vergence and/or saccade abnormalities, because they are conducted using a binocular motor stimulation device configured to specifically and physically stimulate vergences and saccades, comprising visual or audiovisual targets placed in real space at eccentricities and depths of a surface. In this case, detected vergence abnormalities may not be due to language understanding difficulties.

Any binocular motor stimulation device configured to specifically and physically stimulate vergences and saccades and comprising visual or audiovisual targets placed in real space at eccentricities and depths of a surface may be used for the vergence test(s). A device for stimulating binocular motricity by sensory stimulation means such as diodes (visual stimulation) and optionally sound diffusion means (audio stimulation), which are arranged along different arcs of iso-vergence (an arc of iso-vergence is such that the vergence angle of both eyes required to fix each point on this arch is the same) in front of a person may notably be used. This device advantageously comprises four arcs of iso-vergence. Preferably, the sensory stimulation means are arranged on a flat support. The means for sensory stimulation are arranged on arcs at a distance of between 20 and 150 cm from an individual and between each means of stimulation placed on the same arc there is an angle of 10 degrees. The support is advantageously mounted on a height-adjustable stand allowing the device to be properly positioned at the level of the face of an individual. The support for the sensory stimulation means is preferably trapezoidal and has suitable dimensions to test the entire natural range of movements of the eyes (saccades at different depths of iso-vergence arcs, vergences along the median plane, movements combining saccades and vergences) and rehabilitate binocular motricity in direction (saccades), in depth (vergences) or combining them. The stimulation offered by this type of device is flexible, it can be audiovisual (thanks to light diodes and sound means), only audio or only visual. The means for visual and audio stimulation constitute targets for the person. Each sensory stimulation medium is represented by a A preferred such device is the REMOBI^{®} device, which structure is notably described in US8851669 and WO2011073288A1, the content of which are herein incorporated by reference. In particular, **Figure 3** of US8851669 and WO2011073288A1 illustrate a bottom view of a device for the rehabilitation and/or training device for the binocular motivity of a patient according to the invention (display means for visual stimuli and means of generating auditory stimuli).

Due to the presence in the REMOBI^{®} device at quasi the same locations of both visual and audio targets (it is then referred to audiovisual targets), it is possible to perform vergence and saccade tests either using audiovisual stimulation (in this case, each stimulation involves both a visual signal and an audio signal) or using visual stimulation only (in this case, each stimulation involves only a visual signal, but no audio signal).

In the case of audiovisual stimulation, the visual signal and the audio signal start preferably simultaneously or very near, typically the audio signal precedes the visual signal by 50 msec and its duration is shorter (about 50-200 ms, preferably about 100 ms) than the visual signal (generally more than 1000 ms).

The inventors showed that, in individuals not suffering from a learning disorder, ocular motor performances in a vergence or saccade test is generally better when the individual is audiovisually stimulated rather than when the individual is only visually stimulated. In particular, audiovisual targets lead to a latency decrease of leftward or rightward saccades; for vergence, the audiovisual targets lead to shortening of movement duration and to increase of their average velocity (see Example 4). However, in individuals suffering from dyslexia, the inventors further showed that audiovisual stimulation does not generally result in better performance in vergence or saccade tests than visual stimulation only (see Example 4). This suggests that comparing performances in a vergence or saccade test using audiovisual stimulation and in a vergence test using visual stimulation only may further permit to detect multisensorial integration difficulties in individuals suffering from a learning disorder, in particular in dyslexia.

Therefore, in a preferred embodiment, the vergence and/or the saccade test(s) is(are) performed twice, once using audiovisual stimulation and once using visual stimulation only. The vergence/saccade test using audiovisual stimulation and the vergence/saccade test using visual stimulation only may be performed in any order.

### Preferred vergence tests

The vergence test comprises 30 to 50 trials of:
- visually and/or audiovisually stimulating the individual at a point located at the eyes level, in the central axis between the left eye and the right eye, at a first distance (illustrated for instance by point F in **Figure 1C**), and
- visually and/or audiovisually stimulating the individual at another point located at the eye's level, in the central axis between the left eye and the right eye, at another distance calling for a convergence movement (illustrated for instance by point T' in **Figure 1C**) or a divergence movement (illustrated for instance by point T in **Figure 1C****),** half of the trials calling for a convergence movement, the other half for a divergence movement, the trials calling for convergence and divergence movements being interleaved.

The vergence test comprises 30 to 50 trials, preferably an even number of trials between 30 and 50, more preferably between 36 and 44 trials, such as 36, 38, 40, 42 or 44 trials, in particular 40 trials.

Each trial of a vergence test preferably comprises:
- visually and/or audiovisually stimulating the individual at a point located at the eyes level, in the central axis between the left eye and the right eye, at a distance of 40 cm ± 5 cm (i.e. between 35 and 45 cm, preferably between 36 and 44 cm, between 37 and 43 cm, between 38 and 42 cm, between 39 and 41 cm, more preferably 40 cm, see point F in **Figure 1C**) for a period varying from 1400ms to 2000ms, and
- visually and/or audiovisually stimulating the individual at another point located at the eyes level, in the central axis between the left eye and the right eye, at a distance of 20 cm ± 5 cm (i.e. between 15 and 25 cm, preferably between 16 and 24 cm, between 17 and 23 cm, between 18 and 22 cm, between 19 and 21 cm, more preferably 20 cm, see point T' in **Figure 1C****)** calling for a convergence movement or at a distance of 70 to 150 cm (in particular 70 cm ± 5 cm, i.e. between 65 and 75 cm, preferably between 66 and 74 cm, between 67 and 73 cm, between 68 and 72 cm, between 69 and 71 cm, more preferably 70 cm; or 100 cm ± 5 cm, i.e. between 95 and 105 cm, preferably between 96 and 104 cm, between 97 and 103 cm, between 98 and 102 cm, between 99 and 101 cm, more preferably 100 cm; or 150 cm ± 5 cm, i.e. between 145 and 155 cm, preferably between 146 and 154 cm, between 147 and 153 cm, between 148 and 152 cm, between 14 and 151 cm, more preferably 150 cm; see point T in **Figure 1C****)** calling for a divergence movement during 1500 to 2500 ms, preferably 2000 ms, half of the trials calling for a convergence movement, the other half for a divergence movement, the trials calling for convergence and divergence movements being interleaved.

As explained above, the individual is preferably submitted twice to one the preferred vergence tests disclosed above, once using audiovisual stimulation and once using visual stimulation only.

### Preferred saccade test(s)

The saccade test comprises 30 to 50 trials of:
- visually and/or audiovisually stimulating the individual at a point located at the eyes level, in the central axis between the left eye and the right eye (illustrated for instance by point F in **Figure 1B**), and
- visually and/or audiovisually stimulating the individual at another point located at the eyes level, on the left (illustrated for instance by point T' in **Figure 1B**) or on the right (illustrated for instance by point T in **Figure 1B**) of the previous point, half of the trials being on the left, the other half on the right, the trials on the left and on the right being interleaved.

The saccade test comprises 30 to 50 trials, preferably an even number of trials between 30 and 50, more preferably between 36 and 44 trials, such as 36, 38, 40, 42 or 44 trials, in particular 40 trials.

Each trial of a saccade test preferably comprises:
- visually and/or audiovisually stimulating the individual at a point located at the eyes level, in the central axis between the left eye and the right eye, at a distance of 20, 40, 70 or 100 cm (see point F in **Figure 1B****)** corresponding to a vergence angle of 17°, 9°, 5.3° or 3.72° for a period varying from 1400ms to 2000ms, and
- visually and/or audiovisually stimulating the individual at another point located at the eyes level at the same isovergence arc, at 15° to 20°, preferably 20° of eccentricity on the left (see point T' in Figure 1B) or on the right (see point T in **Figure 1B**) during 1500 ms to 2000ms, preferably 2000 ms, half of the trials being on the left, the other half on the right, the trials on the left and on the right being interleaved.

As explained above, the individual is preferably submitted twice to one the preferred saccade tests disclosed above, once using audiovisual stimulation and once using visual stimulation only.

### Further optional ocular motor test(s)

In addition to the reading test(s), vergence test(s), and saccade test(s), the individual may be submitted in step a1) to further ocular motor test(s).

In particular, the individual may be submitted to one or more image analysis test(s). In such test, an image is given to the individual and his/her eyes movements are recorded during his/her analysis of the image. The individual may then be questioned about what is represented in the image and what story the image discloses. The sections of the image fixated by the eyes may then be analyzed (the eyes trajectories may be reported on the image to see what parts of the image have been fixated and in which order) and compared to the verbal reports and understanding of the image provided by the individual. This type of test is used typically by neurologists and neuropsychologists for functional exploration of the attention and cognitive executive process. Comparison of verbal reports of the patient with the eye movement exploration enables better understanding on the deployment of the image analysis by the patient (see Examples 7 and 10). Interestingly, some key parts of the image could be not commented by the person while still his/her eyes were fixating or vice versa.

In the field of neurology, orthoptics and neuropsychology, clinicians can use other tests, including Stroop test, Test of Visual Perceptual Skills, EVADYS evaluation of visuo-attentional span, and DEM development eye movement test, all well known in the art. For instance, Daniel & Kapoula have shown the utility of the use of the Stroop test combined with eye movement recording and analysis for evaluating the interplay between quality of binocular motricity and cognition (see e.g., François Daniel, Zoï Kapoula Sci Rep. 2019; 9: 1247 ; François Daniel et al. Front Integr Neurosci. 2016; 10: 33. Published online 2016 Oct 20).

### Further optional presence of an accelerometer on the individual's head in step a1)

In some preferred embodiments, the individual further has an accelerometer mounted on his/her head during step a1), thus measuring the accelerations of its head during one or more (for instance the reading test, the vergence test, the saccade test, or any a combination thereof, preferably all, optionally also during further optional ocular motor tests) of the ocular motor tests.

### Step b1): recording the eye movements of the left eye and the right eye of the individual

In step b1), which occurs simultaneously with step a1), the eye movements of the left eye and the right eye of the individual during the ocular motor tests of step a) are recorded.

This may be performed using any appropriate eye tracker (also referred to as an eye movement acquisition and recording system). The eye movement acquisition and recording system works advantageously with a video-oculography system, i.e. micro-cameras that focus their lenses on both eyes and record their movements when the person is looking at the device to stimulate binocular motricity (vergence and saccade tests) or when he or she is reading (reading test).

Such a system is for example an eyetracker from the company Pupil Labs^{™}, such as the head mounted device Pupil Core, enabling binocular recording at 200 Hz per eye (https://pupil-labs.com/products/core/)

Another type of eye movement acquisition system is the Powref III from the company PlusOptix^{™}. Such a type of system is placed at a distance from the person, for example in front of the person on the REMOBI support. This system allows to measure the eye movement but also, at a distance, the change of eye accommodation for and to plot eye positions but also the plot of the change of eye accommodation. In this regard, one can refer to the document (https://www.medicus.ua/eng/product/ophthalmic-equipment/plusoptiX-R09-PowerRef-3/manufacturer:, 8 "HSOA Journal of Clinical Studies and Medical Case Reports", Kapoula Z, et al, J Clin Stud Med Case Rep 2019, 6: 74).

Other examples of suitable eye trackers include the EyeSeeCam system (University of Munich Hospital, Clinical Neuroscience, Munich, Germany, available in the public domain at http://eyeseecam.com/,) or other remote devices e.g. Tobbii (https://www.tobii.com/), Powref (https://plusoptix.com/home).

Step b1) may optionally further comprise recording the individual's head accelerations during one or more (for instance the reading test, the vergence test, the saccade test, or any a combination thereof, preferably all, optionally also during further optional ocular motor tests) of the ocular motor tests. In this case, an accelerometer is mounted on the individual's head during the test(s) in step a1) and acceleration of the individual's head is recorded simultaneously with the individual's eyes movements. Measure and recording of the individual's head acceleration permits to analyze the individual's head stability during the eye movement tests.

Appropriate accelerometers include, without limitation, i.e. XMPU6050 (accelerometer + gyroscope) Bluetooth module.

### Step c1): Calculating the value of at least one binocular coordination parameter

In step c1), at least one binocular coordination parameter is calculated using a digital processing device.

### Calculating vergence parameters during vergence tests

A vergence is the simultaneous movement of both eyes in opposite directions to obtain or maintain single binocular vision. To look at an object closer by, the eyes rotate towards each other (convergence), while for an object farther away they rotate away from each other (divergence).

Based on the recording of eye movements made by the eye tracker, the effective trajectory corresponding to each vergence is obtained by calculating an unconjugated signal defined by the difference of the position of the left eye with the position of the right eye (i.e., left eye - right eye).

Each vergence effective trajectory is calculated between the time point when a visual or audiovisual stimulation inducing vergence movement starts and the time point when a next visual or audiovisual stimulation inducing vergence movement starts.

In any vergence test, a representative vergence trajectory corresponding to a mean trajectory of all effective trajectories is calculated, and effective trajectory at too much variance with the representative trajectory may be discarded for further analysis.

For each vergence effective trajectory, a corresponding effective velocity trajectory is further calculated by conventional calculation (see Figure 2).

Each effective velocity trajectory first permits to calculate the peak velocity of vergence (divergence or convergence), which is defined as the highest velocity value of the effective velocity trajectory (see Figure 2).

For each vergence effective trajectory and corresponding velocity trajectory, several parts of the vergence effective trajectory and specific time points may then be calculated (see Figure 2):
- Onset of vergence (convergence or divergence):
   This parameter is defined as the time point when the vergence velocity, initially close to zero has increased to reach 8-12%, preferably 10%, of the peak velocity (highest velocity in the effective trajectory), or when the vergence velocity initially close to zero has increased to reach a predefined velocity. For instance, when using a vergence test involving a convergence or divergence of about 7-9°, onset of vergence may be defined as the time point when the vergence velocity reaches 4-6° /s, preferably 5° /s.
- Offset of vergence (convergence or divergence):
   This parameter is defined as the time point when the vergence velocity, after decreasing from the peak velocity reaches only 8-12%, preferably 10%, of the peak velocity, or when the vergence velocity after decreasing from the peak velocity reaches a predefined velocity. For instance, when using a vergence test involving a convergence or divergence of about 7-9° , offset of vergence may be defined as the time point when the vergence velocity once more reaches 4-6° /s, preferably 5° /s after decreasing from peak velocity.
- Latency trajectory part (convergence or divergence):
   This part of the effective vergence trajectory corresponds to the part between the time point when the visual or audiovisual stimulation starts (beginning of the trajectory) and convergence or divergence onset.
- Initial phasic trajectory part of vergence or "initial phasic component":
   This part of the effective vergence trajectory corresponds to the part between convergence or divergence onset and offset.
- 80 ms slower trajectory part of vergence or "80 ms component":
   This part of the effective vergence trajectory corresponds to the 80 ms after convergence or divergence offset.
- 160 ms slower trajectory part of vergence or "160 ms component":
   This part of the effective vergence trajectory corresponds to the 160 ms after convergence or divergence offset.

Based on the above-defined time points and trajectory parts, vergence parameters may be calculated. Vergence parameters include:
- Duration parameters:
   o Latency duration of vergence (convergence or divergence):
      This parameter is defined as the duration between the time point when the visual or audiovisual stimulation starts and convergence or divergence onset.
   ∘ Initial phasic duration of vergence (convergence or divergence):
      This parameter is defined as the duration of the initial phasic trajectory part of vergence, i.e. the duration between convergence or divergence onset and offset.
   ∘ Total duration:
      This parameter is defined as the sum of the initial phasic duration of vergence and the following 160 ms.
- Amplitude parameters:
   o Amplitude of the initial phasic component:
      This parameter is defined as (unconjugated signal at offset - unconjugated at signal onset).
   ∘ Amplitude of the 80 ms component:
      This parameter is defined as (unconjugated signal 80 ms after offset-unconjugated signal at offset).
   ∘ Amplitude of the 160 ms component:
      This parameter is defined as (unconjugated signal 160 ms after offset-unconjugated signal at offset).
   ∘ Total amplitude:
      This parameter is defined as the sum of the amplitude of the initial phasic component and the amplitude of the 160 ms component.
- Velocity parameters:
   ∘ Peak velocity:
      As mentioned above, peak velocity is the highest velocity value of the effective velocity trajectory.
      Peak velocity of specific parts of each vergence effective trajectory may also be calculated, including the peak velocity of the initial phasic component. However, it should be noted that peak velocity is clearly found during the initial phasic component and is thus equal to the peak velocity of the initial phasic component.
   ∘ Average velocity of the initial phasic component:
      This parameter is defined as the ratio between the amplitude and the duration of the initial phasic component.
   ∘ Total average velocity:
      This parameter is defined as the ratio between the total amplitude and the total duration, as defined above.

Vergence parameters are preferably calculated separately for divergence and convergence trajectories.

No matter which vergence parameter(s) is(are) calculated, the calculation may be made using any suitable software able to compute vergence effective trajectories from the recordings made by the eye tracker and calculate vergence parameters. Such software may preferably be the AIDEAL^{®} software disclosed in French patent application filed on May 14, 2020 under number FR2004768, the content of which is herein incorporated by reference.

### Calculating saccade parameters during saccade tests

A saccade is a quick, simultaneous movement of both eyes between two or more phases of fixation in the same direction.

Based on the recording of eye movements made by the eye tracker, the effective trajectory corresponding to a saccade is obtained by calculating a conjugate signal defined by the average of the position of the left eye with the position of the right eye (i.e. left+right eyes positions/2).

Each saccade effective trajectory is calculated between the time point when a visual or audiovisual stimulation inducing saccade movement starts and the time point when a next visual or audiovisual stimulation inducing saccade movement starts.

In any saccade test, a representative saccade trajectory corresponding to a mean trajectory of all effective trajectories is calculated, and effective trajectory at too much variance with the representative trajectory may be discarded for further analysis.

For each saccade effective trajectory, a corresponding effective velocity trajectory is further calculated by conventional calculation (see Figure 3, illustrating this for an individual with a vergence disorder tested for saccades of 40°).

Each effective velocity trajectory first permits to calculate the peak velocity of saccade (right or left), which is defined as the highest velocity value of the effective velocity trajectory (see Figure 3).

For each saccade effective trajectory and corresponding velocity trajectory, several parts of the saccade effective trajectory and specific time points may then be calculated (see Figure 3):
- Onset of saccade (right or left):
   This parameter is defined as the time point when the saccade velocity, initially close to zero has increased to reach 8-12%, preferably 10%, of the peak velocity (highest velocity in the effective trajectory), or when the saccade velocity initially close to zero has increased to reach a predefined velocity. For instance, when using a saccade test involving a right or left saccade of about 20°, onset of saccade may be defined as the time point when the saccade velocity reaches 40-50° /s, preferably 45° /s.
- Offset of saccade (right or left):
   This parameter is defined as the time point when the saccade velocity, after decreasing from the peak velocity reaches only 8-12%, preferably 10%, of the peak velocity, or when the saccade velocity after decreasing from the peak velocity reaches a predefined velocity. For instance, when using a saccade test involving a right or left saccade of about 20°, offset of saccade may be defined as the time point when the saccade velocity once more reaches 40-50° /s , preferably 45° /s after decreasing from peak velocity.
- Latency trajectory part (right or left):
   This part of the effective saccade trajectory corresponds to the part between the time point when the visual or audiovisual stimulation starts (beginning of the trajectory) and right or left saccade onset.
- Initial phasic trajectory part of saccade or "initial phasic component":
   This part of the effective saccade trajectory corresponds to the part between right or left saccade onset and offset.
- 80 ms drift trajectory part of saccade or "80 ms component":
   This part of the effective saccade trajectory corresponds to the 80 ms after right or left saccade offset.
- 160 ms drift trajectory part of saccade or "160 ms component":
   This part of the effective saccade trajectory corresponds to the 160 ms after right or left saccade offset.

Based on the above-defined time points and trajectory parts, saccade parameters may be calculated. Saccade parameters include:
- Duration parameters:
   o Latency duration of saccade (right or left):
      This parameter is defined as the duration between the time point when the visual or audiovisual stimulation starts and right or left saccade onset.
   ∘ Initial phasic duration of saccade (right or left):
      This parameter is defined as the duration of the initial phasic trajectory part of saccade, i.e. the duration between right or left saccade onset and offset.
   ∘ Total duration:
      This parameter is defined as the sum of the initial phasic duration of saccade and the following 160 ms.
- Amplitude parameters:
   ∘ Amplitude of the initial phasic component:
      This parameter is defined as (conjugated signal at offset - conjugated at signal onset).
   ∘ Amplitude of the 80 ms component:
      This parameter is defined as (conjugated signal 80 ms after offset - conjugated signal at offset).
   ∘ Amplitude of the 160 ms component:
      This parameter is defined as (conjugated signal 160 ms after offset - conjugated signal at offset).
   ∘ Total amplitude:
      This parameter is defined as the sum of the amplitude of the initial phasic component and the amplitude of the 160 ms component.
- Velocity parameters:
   ∘ Peak velocity:
      As mentioned above, peak velocity is the highest velocity value of the effective velocity trajectory.
      Peak velocity of specific parts of each vergence effective trajectory may also be calculated, including the peak velocity of the initial phasic component. However, it should be noted that peak velocity is clearly found during the initial phasic component and is thus equal to the peak velocity of the initial phasic component
   ∘ Average velocity of the initial phasic component:
      This parameter is defined as the ratio between the amplitude and the duration of the initial phasic component.
   ∘ Total average velocity:
      This parameter is defined as the ratio between the total amplitude and the total duration, as defined above.

For saccades, to evaluate binocular coordination of saccades, a disconjugate signal (i.e., left eye - right eye, see Figure 3) may also be first calculated and a disconjugacy parameter may then be calculated, defined as the difference in saccade amplitude between the left and right eye signal may be calculated in the initial phasic component (referred to as "disconjugacy during saccade" parameter), or after the initial phasic component (referred to as "disconjugacy after saccade" or "post-saccadic drift disconjugacy"), in particular in the 80 ms component (referred to as "disconjugacy 80 ms after saccade" parameter), in the 160 ms component (referred to as "disconjugacy 160 ms after saccade" parameter).

Saccade (conjugate and disconjugate) parameters are preferably calculated separately for right and left saccades trajectories.

No matter which saccade parameter(s) is(are) calculated, the calculation may be made using any suitable software able to compute saccade effective trajectories from the recordings made by the eye tracker and calculate saccade parameters. Such software may preferably be the AIDEAL^{®} software disclosed in French patent application filed on May 14, 2020 under number FR2004768, the content of which is herein incorporated by reference.

### Calculating saccade parameters during reading

In the case of a reading test, the saccades are treated in the same way as the saccades obtained during a saccade test (after stimulation by means of the REMOBI device for stimulating binocular motricity). The only difference concerning saccade parameters is that the latency is not measurable since there is no target signal and it is the reader himself who decides when to trigger the next saccade.

On the other hand, the duration of fixation, that is, the duration between saccades, is an additional measured parameter. Reading speed, i.e. the number of words read per minute, is also measured as an additional parameter.

In addition, while in the saccade test, only left saccades and right saccades are distinguished, in the case of a reading test, saccades are categorized as saccades to the right (also referred to as "saccades of progression"), saccades to the left on the same line (saccades of regression) and large saccades to the left back to the next line.

Figure 5A illustrates the exploration of the eyes of a reader who does not suffer from any pathology during the reading of a line of text. In this figure, on the x-axis we have time; in y the conjugate signal of both eyes in degrees, showing the succession of saccades to the right (small vertical lines from bottom to top) followed by fixations (horizontal lines) fixing the words one after the other, then the large saccade to the left (large vertical line from top to bottom) to start reading the next line. The trajectory corresponds to the horizontal conjugate signal (left eye + right eye / 2). **Figure 5B** shows an example of the exploration of the eyes of a dyslexic reader during the reading of a line of text. In addition to the alternance of normal right saccades and fixations followed by a large saccade to the left to start reading the next line, the trajectory further comprises regression saccades (saccades to the left, small vertical lines from top to bottom) during reading of the line of text.

### Optional calculation of head movements parameters when the individual's head accelerations are recorded in step b1) using a head mounted accelerometer

The head acceleration signal is double integrated to obtain the head instantaneous position and head rotation: then the gaze signal is estimated, i.e. the head instantaneous position minus the eye instantaneous position (this method is standard, see Guitton D, Volle M. Gaze control in humans: eye-head coordination during orienting movements to targets within and beyond the oculomotor range. J Neurophysiol. 1987 Sep;58(3):427-59). The average amplitude of head rotation (left/right, or up/down) and its variability during the test is then calculated as well as the average gaze amplitude and its variability. Larger head amplitudes are typical in children with reading problems.

### Preferred binocular coordination parameters calculated in step c1)

In an embodiment, all above-defined vergence and saccade parameters may be calculated, in order to perform a complete assessment of the intrinsic vergences and/or saccades abnormalities of the individual.

However, when only a subset of the above-defined vergence and saccade parameters are calculated, then this subset preferably comprises binocular coordination parameters that may specifically be expected to be abnormal in the individual.

Indeed, the inventors found that several binocular coordination parameters are significantly different in dyslexic individuals compared to individuals not suffering from a learning disorder, including both vergence and saccade parameters. One or more of these parameters are thus preferably calculated in step c1).

### Preferred binocular coordination parameters calculated based on vergence and saccade tests

The inventors notably found that the following parameters are significantly different in dyslexic individuals compared to individuals not suffering from a learning disorder during vergence and saccade tests (see Example 2):
- Vergence parameters during vergence test:
   ∘ duration of the initial phasic component of divergences, since these values are increased in dyslexic individuals compared to non-dyslexic individuals (see Example 2, **Table 4,** p-value of 0.00),
   ∘ peak velocity or peak velocity of the initial phasic component (both values are equal) of convergences and divergences, since these values are increased in dyslexic individuals compared to non-dyslexic individuals (see Example 2, **Table 4,** p-values of 0.00 and 0.00 for convergences and divergences respectively),
   ∘ average velocity of the initial phasic component of convergences and divergences, since these values are decreased in dyslexic individuals compared to non-dyslexic individuals (see Example 2, **Table 4,** p-values of 0.00 and 0.00 for convergences and divergences respectively),
   ∘ total average velocity of convergences, since this value is decreased in dyslexic individuals compared to non-dyslexic individuals (see Example 2, **Table 4,** p-value of 0.03),
   ∘ amplitude of the 80 ms and/or 160 ms component of convergences, since these values are decreased in dyslexic individuals compared to non-dyslexic individuals (see Example 2, **Table 4,** p-values of 0.00 and 0.01 for the 80 ms and 160 ms components respectively).
- Saccade parameters during saccade test:
   o duration of the initial phasic component of left and right saccades, since these values are increased in dyslexic individuals compared to non-dyslexic individuals (see Example 2, **Table 5,** p-values of 0.00 and 0.00 for left and right saccades respectively),
   ∘ peak velocity or peak velocity of the initial phasic component (both values are equal) of right and left saccades, since these values are increased in dyslexic individuals compared to non-dyslexic individuals (see Example 2, **Table 5,** p-values of 0.00 and 0.00 for left and right saccades respectively),
   ∘ average velocity of the initial phasic component of right and left saccades, since these values are decreased in dyslexic individuals compared to non-dyslexic individuals (see Example 2, **Table 5,** p-values of 0.00 and 0.00 for left and right saccades respectively),
   o post-saccadic disconjugacy parameters:
      ▪ disconjugacy 80 ms after right saccades, since this value is increased in dyslexic individuals compared to non-dyslexic individuals. With respect to left saccades, disconjugacy 80 ms after left saccades shows a tendency to be increased in dyslexic individuals compared to non-dyslexic individuals (see Example 2, **Table 5,** p-value of 0.03).
      ▪ disconjugacy 160 ms after right saccades, since this value is increased in dyslexic individuals compared to non-dyslexic individuals. With respect to left saccades, disconjugacy 160 ms after left saccades also shows a tendency to be increased in dyslexic individuals compared to non-dyslexic individuals (see Example 2, **Table 5,** p-value of 0.02).

In particular, velocity abnormalities have been observed in dyslexic individuals for both vergence and saccades during vergence and saccade tests, as well as increased disconjugacy 80 ms to 160 ms (such as 80 ms or 160 ms) after right saccades. Notably, it should be noted that, despite non-deficient and even somewhat improved peak velocity (corresponding to a faster initial eye movement), dyslexic are characterized by a decreased and thus deficient average velocity and thus an increased duration of vergences and saccades during vergence and saccade tests, corresponding of a slowing of the vergence or saccade eye movements after an initial normal or even improved start. This slowing of eyes movement after quick initiation of vergences and saccades, resulting in decreased average velocity and increased duration of vergences and saccades, appears to be characteristic of dyslexic individuals.

During saccade test, dyslexic are also characterized by increased disconjugacy 80 ms or 160 ms after right saccades.

Therefore, in a preferred embodiment, in particular when the individual is a dyslexic individual, based on the vergence and saccade tests, step c1) of calculating the value(s) of at least one binocular coordination parameter comprises calculating:
- at least one vergence velocity or duration parameter, preferably average velocity or duration of convergences and/or divergences (preferably both convergences and divergences),
- at least one saccade velocity or duration parameter, preferably average velocity or duration of left and/or right saccades (preferably both left and right saccades),
- at least one post-saccadic disconjugacy parameter, preferably disconjugacy 80 ms or 160 ms after right saccades,
- or any combination thereof.

Step c1) of calculating the value(s) of at least one binocular coordination parameter may notably comprise calculating at least one vergence velocity or duration parameter, at least one saccade velocity or duration parameter, and at least one post-saccadic disconjugacy parameter obtained in vergence and saccade tests. However, the inventors also found that abnormalities in vergence velocity parameters and in post-saccadic disconjugacy parameter were correlated, so that both types of parameters do not need to be calculated simultaneously. For instance, based on the vergence and saccade tests, step c1) of calculating the value(s) of at least one binocular coordination parameter may notably comprise calculating only:
- at least one vergence velocity parameter and at least one saccade velocity parameter, or
- at least one vergence duration parameter and at least one saccade duration parameter, or
- at least one saccade velocity parameter, and at least one post-saccadic disconjugacy parameter.

More particularly, based on the vergence and saccade tests, step c1) of calculating the value(s) of at least one binocular coordination parameter may comprise calculating:
- duration of the initial phasic component of divergences,
- peak velocity or peak velocity of the initial phasic component (both values are equal) of convergences and/or divergences,
- average velocity of the initial phasic component of convergences and/or divergences,
- total average velocity of convergences and/or divergences (preferably of convergences),
- amplitude of the 80 ms and/or 160 ms component of convergences and/or divergences (preferably of convergences),
- duration of the initial phasic component of right and/or left saccades,
- peak velocity or peak velocity of the initial phasic component (both values are equal) of right and/or left saccades,
- average velocity of the initial phasic component of right and/or left saccades,
- disconjugacy 80 ms after right and/or left saccades, preferably after right saccades,
- disconjugacy 160 ms after right and/or left saccades, preferably after right saccades, or
- any combination thereof.

Particular combinations of parameters of interest that should preferably be comprised in the binocular coordination parameters calculated in step c1) based on the vergence and saccade tests include combinations of at least 2, preferably at least 3, at least 4, at least 5, at least 6, at least 7, at least 8, at least 9, or even the 10 above-described preferred parameters.

Particularly preferred combinations of parameters to be calculated based on the vergence and saccade tests include at least:
i) average velocity of convergences, divergences, and left and right saccades,
ii) duration of convergences, divergences, and left and right saccades,
iii) disconjugacy 80 ms and/or 160 ms after right and left saccades, or after right saccades only,
iv) combination of i) and ii),
v) combination of i) and iii),
vi) combination of ii) and iii), and
vii) combination of i), ii) and iii).

As indicated before, these preferred combinations of parameters may be calculated for vergence and saccade tests performed either using only visual stimulation, using only audiovisual stimulation, or using both visual and audiovisual stimulation in two distinct tests.

### Preferred parameters calculated based on reading test

Preferred binocular coordination parameters calculated based on eye movements recorded during a reading test are average velocity of saccades during reading, disconjugacy 80 ms and/or 160 ms after right saccades during reading, and number or proportion of regression saccades.

In addition, another preferred parameter during reading is the reading speed (number of words/minute).

### Preferred general combinations of parameters

When using a head-mounted accelerometer during at least one ocular motor test, average amplitude of head rotation is preferably also calculated.

Therefore, preferred combinations of parameters calculated in step c1) include:
- Preferred binocular coordination parameters calculated based on vergence and saccade tests as defined above (see in particular combinations i) to vii) disclosed above),
- Preferred parameters calculated based on reading test (average velocity of saccades during reading, disconjugacy 80 ms and/or 160 ms after right saccades during reading, number or proportion of regression saccades, and reading speed), and
- Optionally, average amplitude of head rotation when using a head-mounted accelerometer during at least one ocular motor test.

### Individual suffering from a learning disorder

The method for measuring binocular motricity parameters according to the invention may be performed for any individual suffering from a learning disorder.

Learning disorders are a group of neurodevelopmental disorders that cause a discrepancy between potential and actual levels of academic performance as predicted by the person's intellectual abilities. Learning disorders involve impairments or difficulties in concentration or attention, language development, or visual and oral information processing.

Specific learning disorders affect the ability to understand or use spoken language, understand or use written language, understand and use numbers and reason using mathematical concepts, coordinate movements, and/or focus attention on a task, thus involving problems in reading, mathematics, spelling, written expression or handwriting, and understanding or using verbal and nonverbal language.

Learning disorders notably include:
- dyslexia, a reading disorder that emerges in childhood, involving problems with word recognition, decoding words, and spelling, which translates into slower reading, decreased comprehension, and even difficulty in writing;
- dyspraxia, also referred to as developmental co-ordination disorder, a condition affecting physical co-ordination and causing a child to perform less well than expected in daily activities for their age, and appear to move clumsily;
- dysphasia, a type of disorder where a person has difficulties comprehending language or speaking due to some type of damage in the parts of the brain responsible for communication. The symptoms of dysphasia vary based on the region of the brain that was damaged. There are different regions responsible for understanding language, speaking, reading, and writing, though typically they are found in the left side of the brain;
- dyscalculia, involving problems with mathematics and difficulties with problem-solving;
- dysgraphia, involving problems with spelling, written expression, or handwriting;
- attention-deficit/hyperactivity disorder (ADHD), which is characterized by excessive activity, short attention span and impaired inhibitory control.

While it is not clear from the prior art whether they are or not intrinsic, ocular motor abnormalities have been detected in various learning disorders, and the management of any learning disorder may thus benefit from the method for measuring binocular motricity parameters according to the invention.

However, said individual preferably suffers from one or more of the following learning disorders: dyslexia, dyspraxia, dysphasia, dyscalculia, dysgraphia, and ADHD.

More preferably, the individual is a dyslexic individual.

Method for detecting intrinsic ocular motor abnormalities in an individual suffering from a learning disorder

In a second aspect, the present invention also relates to a second method for detecting intrinsic ocular motor abnormalities in an individual suffering from a learning disorder, comprising:
a2) providing recordings of the eye movements of the left eye and the right eye of the individual during ocular motor tests comprising at least a reading test, a vergence test and a saccade test,
b2) calculating, using a digital processing device, the value(s) of at least one binocular coordination parameter from the records provided in step a2);
c2) comparing the value(s) calculated in step b2) to one or more reference value(s) obtained in one or more individual(s) not suffering from a learning disorder; and
d2) concluding to the presence of intrinsic ocular motor abnormalities in the individual suffering from a learning disorder if at least one binocular coordination parameter calculated in step b2) is significantly different from the one or more reference value(s) obtained in one or more individual(s) not suffering from a learning disorder;

wherein the vergence test and the saccade test from which the recordings are provided in step a2) have been conducted using a binocular motor stimulation device configured to specifically and physically stimulate vergences and saccades, comprising visual or audiovisual targets placed in real space at eccentricities and depths of a surface;
wherein the vergence test which recording is provided comprised 30 to 50 trials of:
   - visually and/or audiovisually stimulating the individual suffering from a learning disorder at a point located at the eyes level, in the central axis between the left eye and the right eye, at a first distance, and
   - visually and/or audiovisually stimulating the individual suffering from a learning disorder at another point located at the eyes level, in the central axis between the left eye and the right eye, at another distance calling for a convergence movement or a divergence movement, half of the trials calling for a convergence movement, the other half for a divergence movement, the trials calling for convergence and divergence movements being interleaved; and
wherein the saccade test which recording is provided comprised 30 to 50 trials of:
   - visually and/or audiovisually stimulating the individual suffering from a learning disorder at a point located at the eyes level, in the central axis between the left eye and the right eye, and
   - visually and/or audiovisually stimulating the individual suffering from a learning disorder at another point located at the eyes level, on the left or on the right of the previous point, half of the trials being on the left, the other half on the right, the trials on the left and on the right being interleaved.

### Step a2): providing recordings of the eye movements

The method for detecting intrinsic ocular motor abnormalities in an individual suffering from a learning disorder according to the invention is based on previously obtained recordings of the eye movements of the left eye and the right eye of the individual during ocular motor tests comprising at least a reading test, a vergence test and a saccade test. Such recordings have been made using an eye tracker as defined above for the method for measuring binocular motricity parameters in an individual suffering from a learning disorder according to the invention.

The reading test(s), vergence test(s), saccade test(s) and optional further test(s) to which the individual suffering from a learning disorder has been submitted for obtaining the recordings provided in step a1) have the same general and preferred features and combinations of features as those disclosed above for the method for measuring binocular motricity parameters in an individual suffering from a learning disorder according to the invention.

In particular, in this second method, the vergence test, the saccade test or both the vergence and saccade tests may be performed twice, once using audiovisual stimulation and once using visual stimulation only.

Moreover, step a2) may further comprise providing recordings of an accelerometer mounted on the individual's head during one or more (for instance the reading test, the vergence test, the saccade test, or any a combination thereof, preferably all, optionally also during further optional ocular motor tests) of the ocular motor tests.

### Step b2): calculating the value of at least one binocular coordination parameter

The binocular coordination parameter(s) calculated in step b2) of the method for detecting intrinsic ocular motor abnormalities in an individual suffering from a learning disorder according to the invention also have the same general and preferred features and combinations of features as those disclosed above for step c1) of the method for measuring binocular motricity parameters in an individual suffering from a learning disorder according to the invention.

In particular, based on the vergence and saccade tests, step b2) of calculating the value(s) of at least one binocular coordination parameter preferably comprises (in particular when the individual is a dyslexic individual) calculating:
- at least one vergence velocity or duration parameter, preferably average velocity or duration of convergences and/or divergences (preferably both convergences and divergences),
- at least one saccade velocity or duration parameter, preferably average velocity or duration of left and/or right saccades (preferably both left and right saccades),
- at least one post-saccadic disconjugacy parameter, preferably disconjugacy 80 ms or 160 ms after right saccades, or
- any combination thereof (in particular those disclosed above for step c1) of the method form measuring binocular motricity parameters according to the invention).

More particularly, based on the vergence and saccade tests, step b2) of calculating the value(s) of at least one binocular coordination parameter preferably comprises (in particular when the individual is a dyslexic individual) calculating:
- duration of the initial phasic component of divergences,
- peak velocity or peak velocity of the initial phasic component (both values are equal) of convergences and/or divergences,
- average velocity of the initial phasic component of convergences and/or divergences,
- total average velocity of convergences and/or divergences (preferably of convergences),
- amplitude of the 80 ms and/or 160 ms component of convergences and/or divergences (preferably of convergences),
- duration of the initial phasic component of right and/or left saccades,
- peak velocity or peak velocity of the initial phasic component (both values are equal) of right and/or left saccades,
- average velocity of the initial phasic component of right and/or left saccades,
- disconjugacy 80 ms after right and/or left saccades, preferably after right saccades,
- disconjugacy 160 ms after right and/or left saccades, preferably after right saccades, or
- any combination thereof (in particular those disclosed above for step c1) of the method form measuring binocular motricity parameters according to the invention).

Here also, particular combinations of parameters of interest that should preferably be comprised in the binocular coordination parameters calculated in step b2) ) based on the vergence and saccade tests include combinations of at least 2, preferably at least 3, at least 4, at least 5, at least 6, at least 7, at least 8, at least 9, or even the 10 above-described preferred parameters.

And particularly preferred combinations of parameters to be calculated based on the vergence and saccade tests include at least:
i) average velocity of convergences, divergences, and left and right saccades,
ii) duration of convergences, divergences, and left and right saccades,
iii) disconjugacy 80 ms and/or 160 ms after right and left saccades, or after right saccades only,
iv) combination of i) and ii),
v) combination of i) and iii),
vi) combination of ii) and iii), and
vii) combination of i), ii) and iii).

As indicated before, these preferred combinations of parameters may be calculated for vergence and saccade tests performed either using only visual stimulation, using only audiovisual stimulation, or using both visual and audiovisual stimulation in two distinct tests.

Preferred parameters calculated based on eye movements recorded during a reading test (i.e. average velocity of saccades during reading, disconjugacy 80 ms and/or 160 ms after right saccades during reading, number or proportion of regression saccades, and reading speed) are also preferably calculated in step b2).

When using a head-mounted accelerometer during at least one test, average amplitude of head rotation is also preferably calculated in step b2).

### Step c2): comparing the value(s) calculated in step b2) to one or more reference value(s) obtained in one or more individual(s) not suffering from a learning disorder

In order to detect intrinsic ocular motor abnormalities in the individual suffering from a learning disorder (in particular a dyslexic individual), the value(s) of binocular coordination parameter(s) calculated in step b2) are compared with corresponding reference values obtained when submitting one or more individual(s) not suffering from a learning disorder to the same reading, vergence and saccade tests or with reference values obtained when submitting one or more individual(s) suffering from a learning disorder to the same reading, vergence and saccade tests.

In particular, reference values of healthy individuals not suffering from a learning disorder and of dyslexic individuals for vergence parameters are provided in **Table 1** below for a vergence test comprising 40 trials, each trial comprising:
- visually and/or audiovisually stimulating the individual at a point located at the eyes level, in the central axis between the left eye and the right eye, at a distance of 40 cm corresponding to a vergence angle of 9° for a period varying from 1400ms to 2000ms, and
- visually and/or audiovisually stimulating the individual at another point located at the eyes level, in the central axis between the left eye and the right eye, at a distance of 20 cm calling for a convergence movement of 8° or at a distance of 150 cm calling for a divergence movement of 7° during 2000 ms,
half of the trials calling for a convergence movement, the other half for a divergence movement, the trials calling for convergence and divergence movements being interleaved.

**Table 1. Reference values of healthy individuals not suffering from a learning disorder and of dyslexic individuals for vergence parameters in the above-described vergence test.**

| **Vergence parameter** | **Healthy individuals** | **Dyslexic individuals** |
|---|---|---|
| | **Average value measured in Example 2** | **Average value measured in Example 2** |
| Divergence Initial Amplitude (deg) | 1.22 | 1.47 |
| Convergence Initial Amplitude (deg) | 2.18 | 2.18 |
| Divergence Total Amplitude (deg) | 2.78 | 2.76 |
| Convergence Total Amplitude (deg) | 4.13 | 3.7 |
| Divergence Latency (ms) | 334.27 | 334.02 |
| Convergence Latency (ms) | 314.29 | 336.51 |
| **Divergence Duration (ms)** | **29.64** | **42.61** |
| **Convergence Duration (ms)** | **49.50** | **60.71** |
| **Divergence Peak Velocity (deg/sec)** | **61.95** | **90.68** |
| **Convergence Peak Velocity (deg/sec)** | **70.79** | **99.33** |
| **Divergence Average Velocity (deg/sec)** | **41.32** | **32.65** |
| **Convergence Average Velocity (deg/sec)** | **46.00** | **35.28** |
| Divergence Total Velocity (deg/sec) | 14.53 | 13.43 |
| **Convergence Total Velocity (deg/sec)** | **19.41** | **16.21** |
| Divergence amplitude 80 ms after phasic component (deg) | 0.89 | 0.73 |
| **Convergence Amplitude 80ms after phasic component (deg)** | **1.12** | **0.85** |
| Divergence amplitude 160 ms after phasic component (deg) | 1.56 | 1.29 |
| **Convergence amplitude 160 ms after phasic component (deg)** | **1.95** | **1.52** |

For other specific vergence tests (different numbers of trials and/or different distances of fixation and thus angles of convergence and/or divergence), reference values may be easily obtained by submitting a group of healthy individuals to the vergence test of interest and calculating vergence parameters of the group of individuals on this basis.

Similarly, reference values of healthy individuals not suffering from a learning disorder and of dyslexic individuals for saccade parameters are provided in **Table 2** below for a saccade test comprising 40 trials, each trial comprising:

**Table 2. reference values of healthy individuals not suffering from a learning disorder and of dyslexic individuals for saccade parameters in the above-described saccade test.**

| Saccade parameter | **Healthy individuals** | **Dyslexic individuals** |
|---|---|---|
| | **Average measured in Example 2** | **Average measured in Example 2** |
| Left Amplitude (deg) | 16.685 | 16.76 |
| Right Amplitude (deg) | 16.83 | 16.24 |
| Left Total Amplitude (deg) | 17.44 | 17.11 |
| Right Total Amplitude (deg) | 17.64 | 16.92 |
| Left Latency (ms) | 244.72 | 250.13 |
| Right Latency (ms) | 265.56 | 273.34 |
| Left Duration (ms) | 64.34 | 70.98 |
| Right Duration (ms) | 63.15 | 68.86 |
| Left Peak Velocity (deg/sec) | 280.07 | 387.89 |
| Right Peak Velocity (deg/sec) | 277.18 | 365.06 |
| Left Average Velocity (deg/sec) | 259.83 | 200.56 |
| Right Average Velocity (deg/sec) | 252.71 | 197.19 |
| Left Total Velocity (deg/sec) | 78.22 | 72.08 |
| Right Total Velocity (deg/sec) | 78.86 | 74.14 |
| Left Fixation Disconjugacy 80 ms after saccade (deg) | 0.62 | 0.77 |
| Right Fixation Disconjugacy 80 ms after saccade (deg) | 0.63 | 0.98 |
| Left Fixation Disconjugacy 160 ms after saccade (deg) | 0.74 | 0.97 |
| Right Fixation Disconjugacy 160 ms after saccade (deg) | 0.80 | 1.24 |
| Left Disconjugacy During Saccade (deg) | 2.82 | 2.85 |
| Right Disconjugacy During Saccade (deg) | 2.34 | 2.55 |

For other specific saccade tests (different numbers of trials and/or different eccentricities of right and/or left saccades), reference values may be easily obtained by submitting a group of healthy individuals to the saccade test of interest and calculating saccade parameters of the group of individuals on this basis.

### Step d2): concluding to the presence or absence of intrinsic ocular motor abnormalities in the individual suffering from a learning disorder

Based on the comparison made in step c2), it may be concluded to the presence or absence of intrinsic ocular motor abnormalities in the individual suffering from a learning disorder.

In particular, it is concluded to the presence of intrinsic ocular motor abnormalities in the individual suffering from a learning disorder if at least one binocular coordination parameter calculated in step b2) is significantly different from the corresponding reference value(s) obtained in one or more individual(s) not suffering from a learning disorder.

In contrast, it is concluded to the absence of intrinsic ocular motor abnormalities in the individual suffering from a learning disorder if the binocular coordination parameter(s) calculated in step b2) are not significantly different from the corresponding reference value(s) obtained in one or more individual(s) not suffering from a learning disorder. Notably, reference normal ranges or average values of binocular coordination parameter(s) may be used in step d2). In this case, it may be concluded to:
- absence of intrinsic ocular motor abnormalities in the individual suffering from a learning disorder if all binocular coordination parameters calculated in step b2) are less than 20% above or below the corresponding reference average value in healthy individuals, and
- presence of intrinsic ocular motor abnormalities in the individual suffering from a learning disorder if at least one binocular coordination parameter calculated in step b2) is at least 20% above or below the corresponding reference average value in healthy individuals.

While a conclusion may be drawn based on direct comparison, one by one, of the value(s) of binocular coordination parameter(s) calculated in step b2) with corresponding reference values obtained when submitting one or more individual(s) not suffering from a learning disorder to the same reading, vergence and saccade tests or with reference values obtained when submitting one or more individual(s) suffering from a learning disorder to the same reading, vergence and saccade tests, as disclosed above, it is also possible to use an algorithm (also referred to as a classifier) that will classify the individual as suffering or not suffering from a learning disorder based on the value(s) of binocular coordination parameter(s) calculated in step b2).

The classifier has been previously trained based on the value(s) of binocular coordination parameter(s) calculated in step b2) obtained from a cohort comprising both individuals suffering from a learning disorder and individuals not suffering from a learning disorder (see Example 9 below).

Any suitable classifier may be used, including both linear and non-linear classifiers. Examples of classifiers that maybe used include logistic regression, Support Vector Machine (SVM), Ensembing, GaussianProcessClassifier, random-forest, KNeighborsClassifier, AdaBoost, BernoulliNB, Linear discriminant analysis, GaussianNB, Decision Tree, Quadratic discriminant analysis and Multilayer perceptron.

In the case of classification based on Remobi saccade parameters, preferred classifiers include, by order of preference: logistic regression, Support Vector Machine (SVM), Ensembing, GaussianProcessClassifier, random-forest, KNeighborsClassifier, and AdaBoost (see Example 9 below).

In the case of classification based on Remobi vergence parameters, preferred classifiers include, by order of preference: logistic regression, Ensembing, Support Vector Machine (SVM), Linear discriminant analysis, BernoulliNB, random-forest, and AdaBoost (see Example 9 below).

In the case of classification based on Remobi Alouette reading parameters, preferred classifiers include, by order of preference: GaussianProcessClassifier, logistic regression, Ensembing, Support Vector Machine (SVM), BernoulliNB, KNeighborsClassifier, random-forest, GaussianNB, AdaBoost, Quadratic discriminant analysis, Linear discriminant analysis, and Decision Tree (see Example 9 below).

In the case of classification based on Remobi Meaningful reading, preferred classifiers include, by order of preference: Ensembing, logistic regression, KNeighborsClassifier, Quadratic discriminant analysis, BernoulliNB, GaussianProcessClassifier, GaussianNB, random-forest, Decision Tree and AdaBoost (see Example 9 below).

Because of particularly good performance when using Remobi saccade and vergence parameters, logistic regression, SVM with an RBF kernel, and Ensembing (and in particular logistic regression and SVM with an RBF kernel) are preferred (see Example 9 below).

Depending if intrinsic ocular motor abnormalities are or not detected in an individual suffering from a learning disorder using the method according to the invention, different types of management of the individual may be decided.

***Optional steps c2') and d2'): comparing vergence and*/*or saccade parameters in vergence or saccade test performed using audiovisual stimulation or visual stimulation only and concluding to the presence or absence of multisensorial integration difficulties in the individual***

In healthy individuals, the use of audiovisual stimulation instead of visual stimulation only results in improved binocular coordination, and in particular to a latency decrease of left and right saccades as well as to decreased duration and increased average velocity of convergences and divergences (see Example 4).

However, in dyslexic individuals, such benefits have not been observed (see Example 4), and some binocular parameters measured in vergence and saccade tests even worsen when using audiovisual stimulation instead of visual stimulation only.

In particular, duration of convergences and divergences is even higher when using audiovisual stimulation instead of visual stimulation only (see Example 4 and **Figure 11****)** and average velocity is even lower when using audiovisual stimulation instead of visual stimulation only (see Example 4 and **Figure 11****).**

Concerning saccades, some improvement in the average velocity of both left and right saccades is observed (see Example 4 and **Figure 12A****).**

However, a deficit observed in dyslexic individuals is increased disconjugacy during the 80 or 160 ms component of saccades. Here, no improvement is observed when using audiovisual stimulation instead of visual stimulation only. In contrast, disconjugacy is even higher when using audiovisual stimulation instead of visual stimulation only (see Example 4 and **Figure 12B****).**

Therefore, when the vergence and/or the saccade test(s) is(are) preformed twice, once using audiovisual stimulation and once using visual stimulation only, the method may, in addition to detecting intrinsic ocular motor abnormalities, further detect multisensorial integration difficulties in the individual suffering from a learning disorder (preferably in a dyslexic individual).

In this case, steps c2) and d2) of the method further comprise:
- Step c2') of comparing calculated vergence and/or saccade parameters (depending on which test(s) has(have) been performed twice, once using audiovisual stimulation and once using visual stimulation only) obtained with audiovisual stimulation and with visual stimulation only.
- Step d2') of concluding to the presence or absence of multisensorial integration difficulties in the individual suffering from a learning disorder based on the comparison of step c2').

It is concluded to the presence of multisensorial integration difficulties in the individual suffering from a learning disorder if at least one binocular parameter that improves when using audiovisual stimulation instead of visual stimulation only in healthy individual (duration or average velocity of convergences and divergences) does not improve in the tested individual suffering from a learning disorder when using audiovisual stimulation instead of visual stimulation only.

Conversely, it is concluded to the absence of multisensorial integration difficulties in the individual suffering from a learning disorder if all parameters that improve when using audiovisual stimulation instead of visual stimulation only in healthy individual (duration or average velocity of convergences and divergences) also improves in the tested individual suffering from a learning disorder when using audiovisual stimulation instead of visual stimulation only.

Here also, it is also possible to use an algorithm (also referred to as a classifier) that will classify the individual as suffering or not suffering from a learning disorder based on the value(s) of binocular coordination parameter(s) calculated in step b2), wherein the classifier has been previously trained based on the value(s) of binocular coordination parameter(s) calculated in step b2) obtained from a cohort comprising both individuals suffering from a learning disorder and individuals not suffering from a learning disorder.

### Optional steps c2") and d2"): comparing individual's head accelerations to reference accelerations in healthy individuals

Healthy adults read, make saccades <20° or vergence movements with minimal head rotation (<1-2°). This ability is acquired during development, when they learned to move the eyes with minimal head motion. In young children (6-10 years old), clinician observations (data not shown) indicate large head movements. Increased head movements during reading or other eye movements, signify ongoing incomplete development of eye head coordination and optimization of binocular eye movement control. In older dyslexics or individuals with learning problems, increased head rotation may signify failure of complete development of eye head coordination and/or the presence of infra-clinic problems of binocular vision (double of blurred) resulting from micro-strabismus, vertical or horizontal heterophorias, disorders of the accommodation-vergence synergy.

Therefore, when an accelerometer has been mounted on the individual's head during one or more of the ocular motor tests and recordings of the accelerations are also provided in step a2), the accelerations of the individual suffering from a learning disorder (preferably dyslexia) may be compared in an optional step c2") to reference accelerations of healthy individuals. In particular, the average amplitude of head rotation of the individual suffering from a learning disorder (preferably dyslexia) may be compared to reference average amplitudes of head rotations of healthy individuals.

Based on this comparison, the method may include an optional step d2") in which it is concluded to presence or absence of failure of complete development of eye head coordination and/or the presence of infra-clinic problems of binocular vision in the individual suffering from a learning disorder, provided that this individual is more than 10 years old.

The head accelerations parameters may also be included in an algorithm or classifier.

### Individual suffering from a learning disorder

The method for detecting intrinsic ocular motor abnormalities (and optionally multisensorial integration difficulties) according to the invention may be performed for any individual suffering from a learning disorder, as described above for the method for measuring binocular motricity parameters in an individual suffering from a learning disorder according to the invention.

However, here also, the method is preferably performed for an individual suffering from dyslexia, dyscalculia, dysgraphia, dyspraxia, and ADHD, more preferably for a dyslexic individual.

### Method for treating intrinsic ocular motor abnormalities in an individual suffering from a learning disorder

The inventors have found that most dyslexic children have intrinsic vergence and saccade abnormalities, and that these abnormalities may at least partly be corrected by submitting the dyslexic individual to vergence training sessions.

In a third aspect, the present invention thus also relates to a third method for treating intrinsic ocular motor abnormalities in an individual suffering from a learning disorder, comprising:
a3) submitting the individual to ocular motor tests comprising at least a reading test, a vergence test and a saccade test;
b3) recording the eye movements of the left eye and the right eye of the individual during each of the ocular motor tests; and
c3) calculating, using a digital processing device, the value of at least one binocular coordination parameter from the records obtained in step b3);
d3) comparing the value(s) calculated in step c3) to one or more reference value(s) obtained in one or more individual(s) not suffering from a learning disorder;
e3) concluding to the presence of intrinsic ocular motor abnormalities in the individual if at least one binocular coordination parameter calculated in step b3) is significantly different from the one or more reference value(s) obtained in one or more individual(s) not suffering from a learning disorder,
f3) submitting the individual to vergence training sessions once or twice a week between 2 to 6 weeks; and
g3) repeating steps a3) to e3), and optionally step f3), until some improvement in the intrinsic ocular motor abnormalities of the individual suffering from a learning disorder is found;

wherein the vergence test, the saccade test and the vergence training sessions are conducted using a binocular motor stimulation device configured to specifically and physically stimulate vergences and saccades, comprising visual or audiovisual targets placed in real space at eccentricities and depths of a surface;
wherein the vergence test comprises 30 to 50 trials of:
   - visually and/or audiovisually stimulating the individual suffering from a learning disorder at a point located at the eyes level, in the central axis between the left eye and the right eye, at a first distance, and
   - visually and/or audiovisually stimulating the individual suffering from a learning disorder at another point located at the eyes level, in the central axis between the left eye and the right eye, at another distance calling for a convergence movement or a divergence movement, half of the trials calling for a convergence movement, the other half for a divergence movement, the trials calling for convergence and divergence movements being interleaved; and
wherein the saccade test comprises 30 to 50 trials of:
   - visually and/or audiovisually stimulating the individual suffering from a learning disorder at a point located at the eyes level, in the central axis between the left eye and the right eye, and
   - visually and/or audiovisually stimulating the individual suffering from a learning disorder at another point located at the eyes level, on the left or on the right of the previous point, half of the trials being on the left, the other half on the right, the trials on the left and on the right being interleaved; and
wherein each vergence training session comprises 1 or 2 repetitions of 1 or 2 block(s) of divergence followed by 3 to 5 blocks of convergence, each block comprising 30 to 50 trials of:
   - visually and/or audiovisually stimulating the individual suffering from a learning disorder at a first point located at the eyes level, in the central axis between the left eye and the right eye for a period varying from 1000 to 1600 ms, at a first distance,
   - visually and/or audiovisually stimulating the individual suffering from a learning disorder at a second point located at the eyes level, in the central axis between the left eye and the right eye for a period of150 to 250 ms, 175 to 225 ms, preferably 200 ms, at a second distance calling for a convergence movement in convergence blocks or a divergence movement in divergence blocks, and
   - visually and/or audiovisually stimulating the individual suffering from a learning disorder at a third point located at the eyes level, in the central axis between the left eye and the right eye for a period varying from 1000 to 1600 ms, at a third distance calling for a further convergence movement in convergence blocks or a further divergence movement in divergence blocks.

### Steps a3) to e3)

Steps a3) to c3) of the third method according to the invention (for treating intrinsic ocular motor abnormalities in an individual suffering from a learning disorder) are the same as steps a1) to c1) of the first method according to the invention (for measuring binocular motricity parameters in an individual suffering from a learning disorder) as disclosed above. Similarly, steps d3) and e3) of the third method according to the invention are the same as steps c2) and d2) of the second method according to the invention (for detecting intrinsic ocular motor abnormalities, and optionally multisensorial integration difficulties, in an individual suffering from a learning disorder) as disclosed above.

These steps thus have the same general and preferred features and combinations of features as those disclosed above in the corresponding method.

### Step f3): vergence training sessions

In step f3), the individual suffering from a learning disorder is submitted to vergence training sessions once or twice a week between 2 to 6 weeks, preferably 3 to 5 weeks, such as 3, 4 or 5 weeks. Preferably, the individual is submitted to at least 4 (or at least 5, at least 6, at least 7, at least 8, such as 4, 5 6, 7, 8, 9 or 10) vergence training sessions (also referred to as "vergence rehabilitation sessions" or "vergence rehabilitation training sessions"). The period between two successive vergence training sessions is preferably of 2 to 7 days, such as 2, 3, 4, 5, 6 or 7 days. The period between different successive vergence training sessions may vary, depending on the availabilities of the individual and visual health professional. However, the vergence training sessions are preferably regularly spaced.

Each vergence training session comprises 1 or 2 repetitions of 1 or 2 block(s) of divergence followed by 3 to 5 blocks of convergence. For instance, a vergence training session may comprise:
- 1 block of divergence, followed by 4 blocks of convergence. When each block contains 40 trials, each block is about 2-3 minutes and such a training session has a duration of about 15 minutes.
- 2 blocks of divergence, 3 blocks of convergence, 2 blocks of divergence, 3 blocks of convergence, and a final block of divergence. When each block contains 40 trials, each block is about 2-3 minutes and such a training session has a duration of about 35 minutes.

While all vergence training sessions to which the individual suffering from a learning disorder is submitted in step f3) may be identical, it is also possible for the visual health professional to change the precise content of vergence training sessions during step f3).

Each block of a vergence training session comprises 30 to 50 trials, preferably an even number of trials between 30 and 50, more preferably between 36 and 44 trials, such as 36, 38, 40, 42 or 44 trials, in particular 40 trials.

The trials of the vergence training or treating procedure are not the same as in the vergence testing procedure.

After an initial fixation of an LED presented at various depths along the central line the first target LED stimulus is only activated for a short period (between 150 and 200 ms); following this period, another target stimulus is activated for a period of 800 to 1800 ms. preferably 1300 ms. The vergence rehabilitation training protocol based on sequences of the double step type is more drastic and very effective because it triggers the implementation by the central nervous system of a new adaptive control (generation of a motor command in response to the final stimulus and not in response to the initial transitory stimulus). The technique can be particularly useful as it stimulates natural ocular motor plasticity. During convergence training blocks the second target is closer to the individual's eyes that the first one, and the opposite during the divergence training trials.

Each trial of each block of a vergence training session comprises:
- visually and/or audiovisually stimulating the individual suffering from a learning disorder at a first point located at the eyes level, in the central axis between the left eye and the right eye for a period varying from 1000 to 1600 ms, at a first distance (for instance illustrated by point F in Figures 4B and 4C),
- visually and/or audiovisually stimulating the individual suffering from a learning disorder at a second point located at the eyes level, in the central axis between the left eye and the right eye for a period of 150 to 200 ms, preferably 200 ms, at a second distance calling for a convergence movement in convergence blocks (for instance illustrated by point T1 or T1' in Figure 4C) or a divergence movement in divergence blocks (for instance illustrated by point T1 or T1' in Figure 4B), and
- visually and/or audiovisually stimulating the individual suffering from a learning disorder at a third point located at the eyes level, in the central axis between the left eye and the right eye for a period varying from 800 to 1800 ms, preferably 1000 to 1600 ms, in particular 1300 ms, at a third distance calling for a further convergence movement in convergence blocks (for instance illustrated by point T2 or T2' in Figure 4C) or a further divergence movement in divergence blocks (for instance illustrated by point T2 or T2' in Figure 4B).

This type of protocol is referred to as a vergence double-step protocol, and is based on the fact that the target steps to a second position before the vergence movement completion. Given that the vergence latency is between 160 and 250 ms, and vergence execution lasts between 350 and 550 ms, it is almost certain that the second step of the target occurred before the initial vergence eye movement has been made. This type of protocol is designed to expose the visuo-motor system to an error that cannot be corrected online and leads to adaptive readjustment of the gain of the motor control.

### Step g3)

In step g3), steps a3) to e3) (functional exploration), and optionally step f3) (rehabilitation), are repeated until some improvement in the intrinsic ocular motor abnormalities of the individual suffering from a learning disorder is found.

In many cases, provided that the individual has been submitted to a sufficient number of regularly spaced vergence training sessions, step f3) will not have to be repeated.

However, in some cases, in particular when the intrinsic ocular abnormalities are severe or when numerous abnormalities have been detected in step e3), the visual health professional may choose to submit the individual to a limited number of vergence training sessions (in the range defined above in step f3)), then repeat steps a3) to e3) in order to assess improvements and determine how many further vergence training sessions may be needed, and then repeat step f3) with a suitable number of additional vergence rehabilitation sessions. In any case, functional exploration (steps a3) to e3)) is preferably performed systematically after step f3) (rehabilitation).

### Individual suffering from a learning disorder

The method for treating intrinsic ocular motor abnormalities in an individual suffering from a learning disorder according to the invention may be performed for any individual suffering from a learning disorder, as described above for the method for measuring binocular motricity parameters in an individual suffering from a learning disorder according to the invention.

However, here also, the method is preferably performed for an individual suffering from dyslexia, dyscalculia, dysgraphia, dyspraxia, and ADHD, more preferably for a dyslexic individual.

The following examples merely intend to illustrate the present invention.

### EXAMPLES

### Example 1: Detection of vergence and saccade abnormalities in dyslexic individuals during reading

Dyslexic individuals are known to have troubles when reading. Here, dyslexic and non-dyslexic individuals were submitted to a reading test and vergence and saccade parameters were measured during reading.

### Patients, Materials and Methods

47 dyslexic (18 female, 29 male; mean age 15.47) and 44 non-dyslexic individuals (22 female, 22 male; mean age 14.77) were recruited from schools in Paris (see Example 2 for full description of subjects).

For the reading test, individuals were seated, and their eyes' movements were recorded using a head-mounted video-oculography device, Pupil Core, enabling binocular recording at 220 Hz per eye (Pupil Labs, Berlin) during reading of the text "L'Alouette", which has no sense (Cavalli E, Colé P, Leloup G, Poracchia-George F, Sprenger-Charolles L, El Ahmadi A. Screening for Dyslexia in French-Speaking University Students: An Evaluation of the Detection Accuracy of the Alouette Test. J Learn Disabil. 2018 May/Jun;51(3):268-282).

Based on these measurements, vergence and saccades curves were generated and vergence and saccades parameters measured using the AIDEAL software (as described in details in French patent application filed on May 14, 2020 under number FR2004768, the content of which is herein incorporated by reference).

### Results

Reading results are presented in **Table 3** below:

**Table 3. Reading results of dyslexic versus non-dyslexic individuals.**

| Reading parameter | Dyslexic individual | Healthy individual | P value |
|---|---|---|---|
| Right saccade amplitude | 1.92 | 2.26 | 0.000 |
| Right saccades Duration | 81.49 | 61.80 | 0.001 |
| Right saccades Peak Velocity | 93.98 | 85.29 | 0.005 |
| Right Average Velocity | 43.25 | 63.51 | 0.000 |
| Right saccades post-saccadic drift (80 ms) | 0.50 | 0.49 | 0.915 |
| Right saccades post-saccadic drift (160 ms) | 0.80 | 0.72 | 0.640 |
| Disconjugacy of right saccades | 1.44 | 0.97 | 0.037 |
| (Number of Right Saccades - Number of Left Saccades)/ Number of Right Saccades | 0.5371 | 0.6644 | 0.014 |
| % of saccades of regression | 47% | 30% | |
| Reading Mistakes per word | 0.0819 | 0.0319 | 0.000 |
| Reading speed (words per Minute) | 110.86 | 126.35 | 0.000 |

In dyslexics, the reading speed (number of words read per minute) is slower (110.86 in dyslexic on average, versus 126.35 in healthy individuals, p=0.000), the amplitude of the saccades is smaller (1.92 in dyslexic on average, versus 2.26 in healthy individuals , p=0.000), the reading mistakes per word were higher (0.0819 in dyslexic on average, versus 0.0319 in healthy individuals, p=0.000), and a greater proportion of regression saccades (saccades in back, to the left, 47% in dyslexic on average, versus 30% in healthy individuals) were observed. These oculomotor abnormalities are the consequence of the difficulty of reading in large part.

Reading saccades also had abnormal velocity profile, e.g., lower average velocity (43.25 in dyslexic on average, versus 63.51 in healthy individuals , p=0.000), longer duration (81.49 in dyslexic on average, versus 61.80 in healthy individuals, p=0.001) and their eyes were poorly coordinated, showing increased disconjugacy during the saccade (1.44 in dyslexic on average, versus 0.97 in healthy individuals, p=0.037).

**Figure 5** shows exemplary trajectories of the eyes movements of a healthy (non-dyslexic, **Figure 5A****)** and a dyslexic **(****Figure 5B****)** individual during reading of one line of text.

**Figure 5A** illustrates the exploration of the eyes of a reader who does not suffer from any pathology. In this figure, on the x-axis we have time; in y the conjugate signal of both eyes in degrees, showing the succession of saccades to the right followed by fixations fixing the words one after the other, then the large saccade to the left to start reading the next line. As may be seen, the exploration of the healthy individual is quite regular, and does not comprise many regression saccades.

In contrast, in **Figure 5B** presenting the same curve for a dyslexic individual, the progression of the eyes is not so regular and comprises regression saccades.

### Conclusions

The above data show that dyslexic individuals have troubles when reading, as well as saccade abnormalities.

### Example 2: Detection of intrinsic vergence and saccade abnormalities in dyslexic individuals

While data presented in Example 1 show that dyslexic individuals have vergence and saccade abnormalities during reading, such data does not permit to conclude whether ocular motor abnormalities during reading are or not at least partly due to intrinsic ocular motor abnormalities.

To be able to draw such conclusion, it is necessary to perform further ocular motor tests, in conditions where vergence and saccades are physically stimulated, thus permitting measure of intrinsic ocular motor coordination.

Such tests have been performed in dyslexic versus non-dyslexic individuals.

### Patients, Materials and Methods

### Participants

47 dyslexic adolescents (18 female, 29 male; mean age 15.47) and 44 non-dyslexic adolescents (22 female, 22 male; mean age 14.77) were recruited from schools in Paris. Dyslexic adolescents were selected from a school targeted towards dyslexic adolescents based on physician documentation of a dyslexia diagnosis in their school records. Both dyslexic and non-dyslexic adolescents had no known neurologic or psychiatric abnormalities. Healthy controls had no known neurological or psychiatric abnormalities, no history of reading difficulty, no visual impairment, or difficulty with near vision. The investigation adhered to the principles of the Declaration of Helsinki and was approved by our Institutional Human Experimentation Committee (CPP CNRS 18 011). Written consent was obtained from the adolescents and/or their parents after they were given an explanation about the experimental procedure.

### Clinical Characteristics of the Participants

Adolescents were asked to rate how much they liked doing certain activities (reading, watching movies, going to museums, etc.) on a scale from one to ten, where one meant they did not like doing the activity at all, and ten meant they loved doing the activity. They were also asked how many hours they spent watching TV, using the computer, playing video games, and on their telephone. They also responded to the Convergence Insufficiency Symptom Survey (CISS), a validated questionnaire for quantifying vergence problems in children and adults (Rouse, M. et al. Validity of the convergence insufficiency symptom survey: a confirmatory study. Optom Vis Sci 86, 357-363, doi:10.1097/OPX.0b013e3181989252 (2009)). Their stereoscopic depth discrimination was measured using the Titmus Test (Titmus Stereo Fly Test, Stereo Optical, Essilor Instruments).

### Eye movement recording device

For each adolescent, eye movements were recorded binocularly with a head-mounted video-oculography device, Pupil Core, enabling binocular recording at 220 Hz per eye (Pupil Labs, Berlin).

### Calibration of the Pupil Labs device

The standard Pupil Labs calibration (Pupil Capture) was applied using a target that was presented at viewing distance of 1m. The individual fixated on the center of the target and moved their head rightward, downward, leftward and upward at their own pace. They then repeated the sequence (Kassner, M., Patera, W. & Bulling, A. in Proceedings of the 2014 ACM international joint conference on pervasive and ubiquitous computing: Adjunct publication. 1151-1160*).*

### Ocular motor tests

Adolescents were asked to sit in front of a horizontal visual-acoustic REMOBI device (see patent US8851669 and WO2011073288). The device was placed at eye level, so that the first arc of LEDs was 20 cm from their eyes. Each child is instructed to carefully fixate quickly and accurately on the moving LED, to not attempt to predict a pattern of motion, and to keep their head still during the 2-minute test.

Oculomotor tests were performed in mesopic light conditions. The red LED stimuli were displayed at different distances, laterally or in depth, always in the horizontal plane (0°, Figures 1B and 1C). LED characteristics were: nominal frequency 626nm, intensity 180mCd, and diameter 3 mm. Adjacent to each LED was embedded a buzzer with the following characteristics: nominal frequency approximately 2048Hz, sound pressure level 75dB, diameter 12 mm.

### Vergence test

For each trial the fixation LED (0°) light up at 40cm, creating a required vergence angle of 9° for a period varying from 1200ms to 1800ms. (see Figures 1A and 1C) It was followed randomly by the target LED during 2000ms, appearing always at the central axis (0°) either at 20cm (calling for a convergence movement of 8° , i.e. from 9° to 17°) or 150cm (calling for a divergence movement of 7°, i.e. from 9° to 2). The test contained 40 trials (20 trials of convergence, 20 of divergence, pseudo-randomly interleaved) in an overlap paradigm: after an overlap period of 200 ms following the lighting of the target LED, the fixation LED switched off.

### Saccade test

Each trial started with the fixation central LED lighting at 40cm from the individual for a randomized period ranging from 1200ms to 1800ms; it was followed by the lighting of the saccade target LED for 2000ms at 20° of eccentricity, randomly chosen on the left or on the right. (Figures 1A and 1B) There were 40 trials (20 left, 20 right, pseudo-randomly interleaved) in an overlap paradigm.

### Data Analysis

Data recorded with the Pupil Labs eye tracker were analyzed with AIDEAL, software developed in the IRIS laboratory (see French patent application filed on May 14, 2020 under number FR2004768, DSO2020003510, 2 mars 2020). The vergence signal was derived by calculating the difference between the two eyes from the individual calibrated eye position signals (i.e., left eye - right eye). The beginning and end of the vergence movements were defined as the time point when the eye velocity exceeded or dropped below 5° /s: these criteria are standard and were applied automatically by the AIDEAL software; the program estimated the initial phasic component as the amplitude between these two initial points. It also calculated the amplitude change during the subsequent 80 ms and 160 ms. The total amplitude was calculated as the sum of the amplitude of the initial phasic component plus the 160 ms component. The total duration was calculated as the duration of the phasic component plus the subsequent 160 ms.

For saccade analysis, AIDEAL treated the conjugate signal, e.g. the L+R eye position/2. The onset and the offset of the saccade were defined as the time points where the velocity went above or below 10% of the peak velocity; practically, this corresponded to values above or below 40°/s (as the peak velocity of 20° saccades is typically above 400° /s). The total average velocity was defined as the ratio of total amplitude in degrees divided by time in seconds. To evaluate binocular coordination of saccades, or the disconjugacy during saccadic movements, the difference in amplitude between the left and the right eye signal was calculated. The disconjugate drift, or the difference in drift amplitude during the first 80 or 160 ms of fixation, was calculated. These calculations are standard and have been used in previous experiments (Kapoula, Z. et al. Transl Vis Sci Technol 5, 8, doi:10.1167/tvst.5.2.8 (2016) ; Morize, A., et al. Invest Ophthalmol Vis Sci 58, 329-342, doi:10.1167/iovs.16-19837 (2017)).

Trials with blinks or other artifacts were discarded automatically by AIDEAL. For each adolescent (dyslexic and non-dyslexic) the number of saccades and vergence movements measured in the fixation tasks were counted. The percentage of movements rejected were 0% for vergence and 9% for saccades in dyslexic children and 2% for vergence and 3% for saccades in healthy children.

### Statistical Analysis

As the measured eye movements data were not normally distributed as determined by the Shapiro-Wilk test, the non-parametric Mann-Whitney U test was utilized for means comparison. All hypothesis testing was two-sided and p-values of <0.05 was considered statistically significant. All analyses were performed using SPSS version 25 (IBM Corp. Released 2017. IBM SPSS Statistics for Windows, Version 25.0. Armonk, NY: IBM Corp.).

### Results

### Participant's Characteristics

Dyslexic adolescents reported spending more hours on the computer per week than non-dyslexic adolescents (30.80 vs 13.54, p = 0.000). They also reported liking to read less (4.78 vs 6.57, p = 0.011) and liking to go to theaters and films more than non-dyslexic adolescents (8.12 vs 7.24, p = 0.036).

### Clinical visual examination

Dyslexic adolescents had a lower score on the stereoscopic vision test (57.50 vs 25.86, p < 0.001), indicating difficulty in perceiving depth with very high spatial resolution. They also exhibited higher scores on the Convergence Insufficiency Symptom Survey (24.40 vs 17.68, p = 0.008), indicating symptomatic vergence disorders. A score greater than 21 indicates a symptomatic vergence disorder (Rouse, M. et al. Optom Vis Sci 86, 357-363, doi:10.1097/OPX.0b013e3181989252 (2009)).

### Eye movement results

### Vergence in Dyslexics vs Non-dyslexics

In terms of the initial phasic component of vergence (the time when velocity exceeds or drops below 5°/s), dyslexic adolescents exhibited a longer duration for divergence (42.61ms, SD 21.02ms vs 29.64ms, SD 9.92ms; p < 0.00, p values are all truncated to 2 decimals). They also exhibited an increased peak velocity in both divergence and convergence, meaning their speed during the initial acceleration component of the vergence movement was higher than non-dyslexics (Divergence: 90.68° /s, SD 60.32° /s vs 61.95° /s, SD 76.63° /s; p < 0.00; Convergence: 99.33° /s, SD 63.45° /s vs 70.79° /s, SD 42.68° /s; p < 0.00). Yet, dyslexics displayed a decreased average velocity during this initial phasic component in both divergence and convergence (Divergence: 32.65°/s, SD 11.19° /s vs 41.32° /s, SD 36.89; p < 0.00; Convergence: 35.28° /s, SD 14.20° /s vs 46.00° /s, SD 15.82; p < 0.00). Therefore, despite demonstrating a higher peak velocity during the acceleration phase, the subsequent deceleration phase was significantly slower. When examining the total average velocity (the initial phasic component plus the subsequent 160 ms), dyslexics displayed a significantly lower total average velocity for convergence (0.016° /s, SD 0.007 vs 0.194° /s, SD 0.008, p = 0.03), but not during divergence (0.013° /s, SD 0.005 vs 0.015° /s, SD 0.005; p = 0.29).

In terms of amplitude in the initial phasic component of vergence, there was no significant difference between dyslexics and non-dyslexics (Divergence: 1.47°, SD 0.97 vs 1.22°, SD 0.65; p = 0.25; Convergence: 2.18°, SD 1.52 vs 2.18°, SD 1.35; p = 0.53). Yet, the later phases of vergence measured during the subsequent 80 and 160 ms were both significantly smaller. For convergence only, dyslexic adolescents exhibited decreased amplitude in both the 80 and 160 ms phases of movement (80ms: 0.85°, SD 0.43 vs 1.12°, SD 0.47, p < 0.00; 160ms 1.52°, SD 0.71 vs 1.95°, SD 0.87, p = 0.01), and divergence movements also trended towards decreased values for the dyslexic population (see **Table 4** below).

**Table 4: Means and standard deviations of vergence parameters in dyslexic and non-dyslexic adolescents. P values are all truncated to 2 decimals. Variables with significant differences between the two populations (p < 0.05) are bolded.**

| | **Dyslexic** | | **Non-dyslexic** | | **p-value** |
|---|---|---|---|---|---|
| | Average | **SD** | **Average** | **SD** | |
| Divergence Initial Amplitude (deg) | 1.47 | 0.97 | 1.22 | 0.65 | 0.25 |
| Convergence Initial Amplitude (deg) | 2.18 | 1.52 | 2.18 | 1.35 | 0.53 |
| Divergence Total Amplitude (deg) | 2.76 | 1.13 | 2.78 | 1.06 | 0.71 |
| Convergence Total Amplitude (deg) | 3.70 | 1.91 | 4.13 | 1.84 | 0.23 |
| Divergence Latency (ms) | 334.02 | 62.50 | 334.27 | 69.18 | 0.96 |
| Convergence Latency (ms) | 336.51 | 68.20 | 314.29 | 75.10 | 0.21 |
| **Divergence Duration (ms)** | **42.61** | **21.02** | **29.64** | **9.92** | **0.00** |
| Convergence Duration (ms) | 60.71 | 30.97 | 49.50 | 24.39 | 0.09 |
| **Divergence Peak Velocity (deg/sec)** | **90.68** | **60.32** | **61.95** | **76.63** | **0.00** |
| **Convergence Peak Velocity (deg/sec)** | **99.33** | **63.45** | **70.79** | **42.68** | **0.00** |
| **Divergence Average Velocity (deg/sec)** | **32.65** | **11.19** | **41.32** | **36.89** | **0.00** |
| **Convergence Average Velocity (deg/sec)** | **35.28** | **14.20** | **46.00** | **15.82** | **0.00** |
| Divergence Total Velocity (deg/sec) | 13.43 | 4.46 | 14.53 | 5.07 | 0.29 |
| **Convergence Total Velocity (deg/sec)** | **16.21** | **6.73** | **19.41** | **7.72** | **0.03** |
| Divergence amplitude 80 ms after phasic component (deg) | 0.73 | 0.30 | 0.89 | 0.80 | 0.06 |
| **Convergence Amplitude 80ms after phasic component (deg)** | **0.85** | **0.43** | **1.12** | **0.47** | **0.00** |
| Divergence amplitude 160 ms after phasic component (deg) | 1.29 | 0.59 | 1.56 | 0.68 | 0.06 |
| **Convergence amplitude 160 ms after phasic component (deg)** | **1.52** | **0.71** | **1.95** | **0.87** | **0.01** |

In terms of total amplitude (adding the initial phase and the amplitude over the subsequent 160 ms), there was no statistically significant difference between the two populations (Divergence: 2.76°, SD 1.13 vs 2.78°, SD 1.06; p = 0.710; Convergence: 3.70°, SD 1.91 vs 4.13°, SD 1.84; p = 0.23).

In summary, careful analysis of the vergence trajectory over different periods reveals abnormal vergence execution in dyslexics, e.g. a higher peak velocity but abnormal slowing of the velocity during the deceleration phase. Amplitude analysis reveals decreased values during the later phases of convergence (80 and 160 ms).

### Saccades in Dyslexics vs Nondyslexics

There was also a significant difference in several parameters in saccades between dyslexic and non-dyslexic adolescents. For saccades either to the left or to the right, during the initial phase of the movement dyslexic adolescents displayed an increased duration, meaning it took them longer to reach the target (Left: 70.98ms, SD 14.14ms vs 64.34ms, SD 11.55ms; p < 0.00; Right: 68.86ms, SD 12.01ms vs 63.15ms, SD 8.57; p < 0.00, , p values are all truncated to 2 decimals). Similar to vergence, they also exhibited an increased peak velocity during the initial phase of the movement (Left: 387.89° /s, SD 160.03° /s vs 280.07° /s, SD 95.76° /s; p < 0.00; Right: 365.06° /s, SD 159.20° /s vs 277.18° /s, SD 97.03° /s; p < 0.00) but a decreased average velocity (Left: 200.56° /s, SD 75.15° /s vs 259.83° /s, SD 61.05° /s; p < 0.00; Right: 197.19° /s, SD 80.00° /s vs 252.71 ° /s, SD 60.37° /s; p < 0.00), meaning that, like in vergence, their initial movement towards the target is faster than normal controls, but the subsequent deceleration phase is slowed substantially.

To evaluate binocular coordination during fixation after the saccade, we measured by the disconjugacy of the drifts during the first 80 and 160 ms following the end of the saccade, as these two periods were chosen to represent the two time constants of the ocular movement: the extraocular muscle movement and the stabilization of the eye after the saccade (DA, R., Z, K. & HP, G. in From neuron to action Ch. Holding the eye still after a saccade, 89-96 (Springer, 1990)). For saccades to the right only, dyslexics had an increased disconjugacy during both phases of fixation (80ms: 0.98, SD 0.96 vs 0.63, SD 0.35, p = 0.03; 160ms: 1.24, SD 1.07 vs 0.80, SD 0.80, p = 0.02). Dyslexics displayed an increased disconjugate drift in both phases of fixation for saccades to the left but this observation did not reach statistical significance (see Table 5 below).

**Table 5: Saccadic parameters in dyslexic and non-dyslexic adolescents. P values are all truncated to 2 decimals. Variables with significant differences between the two populations (p < 0.05) are bolded.**

| | **Dyslexic** | | **Non-Dyslexic** | | **P-Value** |
|---|---|---|---|---|---|
| | **Average** | **SD** | **Average** | **SD** | |
| Left Amplitude (deg) | 16.76 | 3.43 | 16.685 | 1.95 | 0.64 |
| Right Amplitude (deg) | 16.24 | 1.52 | 16.83 | 2.03 | 0.22 |
| Left Total Amplitude (deg) | 17.11 | 1.56 | 17.44 | 2.11 | 0.61 |
| Right Total Amplitude (deg) | 16.92 | 1.37 | 17.64 | 2.23 | 0.25 |
| Left Latency (ms) | 250.13 | 49.09 | 244.72 | 53.75 | 0.12 |
| Right Latency (ms) | 273.34 | 65.61 | 265.56 | 63.09 | 0.36 |
| **Left Duration (ms)** | **70.98** | **14.14** | **64.34** | **11.55** | **0.00** |
| **Right Duration (ms)** | **68.86** | **12.01** | **63.15** | **8.57** | **0.00** |
| **Left Peak Velocity (deg/sec)** | **387.89** | **160.03** | **280.07** | **95.76** | **0.00** |
| **Right Peak Velocity (deg/sec)** | **365.06** | **159.20** | **277.18** | **97.03** | **0.00** |
| **Left Average Velocity (deg/sec)** | **200.56** | **75.15** | **259.83** | **61.05** | **0.00** |
| **Right Average Velocity (deg/sec)** | **197.19** | **80.00** | **252.71** | **60.37** | **0.00** |
| Left Total Velocity (deg/sec) | 72.08 | 14.76 | 78.22 | 9.63 | 0.09 |
| **Right Total Velocity (deg/sec)** | **74.14** | **7.28** | **78.86** | **9.49** | **0.04** |
| Left Fixation Disconjugacy 80 ms after saccade (deg) | 0.77 | 0.46 | 0.62 | 0.43 | 0.07 |
| **Right Fixation Disconjugacy 80 ms after saccade (deg)** | **0.98** | **0.96** | **0.63** | **0.35** | **0.03** |
| Left Fixation Disconjugacy 160 ms after saccade (deg) | 0.97 | 0.64 | 0.74 | 0.46 | 0.08 |
| **Right Fixation Disconjugacy 160** ms **after saccade (deg)** | **1.24** | **1.07** | **0.80** | **0.43** | **0.02** |
| Left Disconjugacy During Saccade (deg) | 2.85 | 1.61 | 2.82 | 1.53 | 0.97 |
| Right Disconjugacy During Saccade (deg) | 2.55 | 1.78 | 2.34 | 1.16 | 0.90 |

The binocular coordination during the saccade (i.e. the amplitude difference between left and right eye) was not significantly different between the two groups (see **Table 5).** To determine the relationship between vergence and saccades, we examined the cross-correlation between average velocity and the disconjugate drift after the saccade at 80 ms and 160 ms. For all adolescents, (dyslexic and non-dyslexic) we found the average velocity during vergence is significantly negatively correlated with the deconjugate drift following saccades to the left in the first 80 ms (Convergence: r=-0.223; p = 0.044; Divergence: r=-0.296; p = 0.007) and in the 160 ms following the saccade (Convergence: r=-0.340; p = 0.002; Divergence: r=-0.402; p = 0.000). Therefore, the lower the velocity was during vergence, the higher the deconjugate drift of the saccades.

When looking at dyslexic and non-dyslexic groups individually, a significant negative correlation between average velocity and the disconjugate drift in the following 160 ms after the saccade was found for dyslexics only during convergence following left saccades (r=-0.355; p = 0.027). Correlations for drifts following rightward saccades did not reach significance. Correlations for healthy children only did not reach significance.

An example comparing vergence and saccade trajectories in a dyslexic individual and a non-dyslexic individual is presented in Figure 6.

### Discussion

In this study, we demonstrate a novel technique using REMOBI technology to objectively measure saccade and vergence disorders in dyslexics and non-dyslexics binocularly, without uncoupling vergence accommodation. Our study demonstrates subtle velocity abnormalities for both saccades and vergence and increased deconjugate post-saccadic drifts in dyslexics, suggesting inefficient binocular motor control in the dyslexic population. The tests used in this study enable a differential diagnosis of eye movement problems in dyslexia occurring independently from reading: namely, that dyslexics' reading difficulty could be partially a result of poor eye movement control.

### High peak but slow average vergence velocity in dyslexics

In terms of vergence, as compared to healthy adolescents, dyslexic adolescents show lower average velocities, suggesting that dyslexic adolescents have overall difficulty making prompt vergence movements, i.e. moving from near to far space and vice versa. This could have implications on their ability to rapidly and accurately perceive depth differences between objects in the three-dimensional space. Psychophysical studies have established that depth perceptual ability relies on quality of vergence eye movements: divergence and convergence (Howard, I. P. & Rogers, B. J. in Perceiving in Depth Vol. Volume 2 Stereoscopic Vision (Oxford University Press, 2012)).

Notably, this is the first time the profile of the vergence velocity was studied in the dyslexic population. Interestingly, dyslexic adolescents exhibited an abnormal velocity profile: they demonstrated a more robust initial velocity (peak velocity) but their average velocity is slower; meaning their velocity is abnormally reduced in the later phases of movement for both convergence and divergence. From Hung's model of double control of vergence, we can consider vergence to be modeled as a dual-mode control system (Hung, G. K., Semmlow, J. L. & Ciuffreda, K. J. A dual-mode dynamic model of the vergence eye movement system. IEEE Trans Biomed Eng 33, 1021-1028, doi:10.1109/tbme.1986.325868 (1986)). The vergence response can be dissected into transient and sustaining components. The transient portion-the initial component-is assumed to be an open-loop control component of enhanced speed of a vergence response. The sustaining portion-the slow component-is assumed to be driven by a visual feedback, closed-loop control system. This closed-loop control system provides fine-tuning of the response and enables the extraordinary accuracy seen in binocular fixation.

Our observations are in line with Hung's model as they demonstrate group differences in the initial and subsequent vergence components. The initial velocity movement during the transient, phasic phase in dyslexic adolescents is faster than that of healthy controls. However, the subsequent portion of the velocity including the sustaining portion (subsequent 160 ms), which is visually driven, is slowed. We postulate that this is due to delays in sensory visual processing in dyslexics. In previous studies we have reported a similar phenomenon in a healthy population, in which only the visually driven component of vergence is slowed as a result of age (Yang, Q., Le, T. T. & Kapoula, Z. Aging effects on the visually driven part of vergence movements. Invest Ophthalmol Vis Sci 50, 1145-1151, doi:10.1167/iovs.08-2474 (2009)). In the dyslexic population, this hypothesis of deficits in the visually-driven, sustaining portion of vergence is also consistent with previous theories regarding deficits in the magnocellular visual pathway that is responsible for processing dynamic, rapidly changing binocular disparity (Stein, J. F., Riddell, P. M. & Fowler, S. Disordered vergence control in dyslexic children. Br J Ophthalmol 72, 162-166, doi:10.1136/bjo.72.3.162 (1988)).

Alternatively, abnormal velocity could be related to inappropriate tailoring of the pulse-slide-steps vergence motor command signals sent to extra-ocular muscles rather than to slower visual processing of the disparity and blur of retinal images (Leigh, R. J. & Zee, D. S. Ch. Vergence eye movements, (Oxford University Press, 2015)). In terms of motor control, the velocity of an movement depends on the quality of the movement generator signal located at the brainstem, i.e. in the mesencephalic reticular formation.²⁸ Inefficiency or dysfunction of the mesencephalic reticular formation in dyslexic adolescents could be at the origin of slower average vergence velocity. Whatever the reason, the impact of slower vergence is that clear single vision cannot be obtained as promptly as for non-dyslexics, i.e., dyslexic adolescents experience a delay in obtaining clear vision when moving their eyes from one depth to the other.

### Vergence velocity and clinical tests

It is notable that these vergence results, tested objectively with REMOBI lab equipment, correspond to the clinical testing performed. Dyslexic adolescents tended to have lower scores of stereovision as tested by the Titmus test. They also had higher Convergence Insufficiency Symptom Survey results, indicating symptomatic vergence disorders. As our lab has argued in the past, individualive orthoptic testing can be used as preliminary testing, but it is important to objectively measure vergence, namely any temporal abnormalities, in order to prescribe efficient rehabilitation (Ward, L. M., Gaertner, C., Olivier, L., Ajrezo, L. & Kapoula, Z. Vergence and accommodation disorders in children with vertigo: A need for evidence-based diagnosis. EClinicalMedicine 21, 100323, doi:10.1016/j.eclinm.2020.100323 (2020)).

### Slow average saccade velocity and disconjugate drifts in dyslexics

In addition to vergence differences, dyslexics displayed differences in saccades in the left and right directions as compared to healthy controls. Notably, as in vergence, dyslexic adolescents demonstrated normal latency, but showed a faster initial peak velocity and a slower average velocity than non-dyslexics, leading to a longer total duration to reach their target. As saccade duration lasted on average less than 80 ms (see Table 5), and visual processing is approximately of the same duration, visual feedback is less likely to intervene in the control of the ongoing saccade, which is therefore executed under open loop control. In the case of saccades, this abnormality favors a hypothesis of motor inefficiency. It is possible that the slowing of saccades is due to inefficient vergence involvement leading to not only the subsequent deconjugate drift, but also the slowed saccade itself. If vergence control is lost during the initial part of the saccade, when the eyes are rapidly accelerating, a rapid vergence restoration mechanism is activated to reestablish this loss of vergence (Jainta, S. & Kapoula, Z. Dyslexic children are confronted with unstable binocular fixation while reading. PLoS One 6, e18694, doi:10.1371/journal.pone.0018694 (2011)). Yet, as we have shown vergence velocity is slowed in dyslexics, the saccade would in turn also be slowed, leaving larger vergence drifts during the subsequent fixation period. In fact, what we call disconjugate drift may be physiologically produced by the vergence system that is slowed in dyslexics and fails to restore vergence during the saccade itself. It has been previously shown that, while vergence normally accelerates the saccade, it can also slow it (Kumar, A. N. et al. Tests of models for saccade-vergence interaction using novel stimulus conditions. Biol Cybern 95, 143-157, doi:10.1007/s00422-006-0073-9 (2006) ; Zee, D. S., Fitzgibbon, E. J. & Optican, L. M. Saccade-vergence interactions in humans. J Neurophysiol 68, 1624-1641, doi:10.1152/jn.1992.68.5.1624 (1992)). Vergence, or disconjugate drifts, during fixation harm visual acuity and therefore, single binocular vision (Westheimer, G. & McKee, S. P. Visual acuity in the presence of retinal-image motion. J Opt Soc Am 65, 847-850, doi:10.1364/josa.65.000847 (1975)).

Interestingly, the difference in drifts was not statistically different after saccades to the left. Saccadic Left/Right asymmetries have been the individual of debate as previous studies have shown contradictory results (Tagu, J., Dore-Mazars, K., Lemoine-Lardennois, C. & Vergilino-Perez, D. How Eye Dominance Strength Modulates the Influence of a Distractor on Saccade Accuracy. Invest Ophthalmol Vis Sci 57, 534-543, doi:10.1167/iovs.15-18428 (2016) ; Petit, L. et al. Strong rightward lateralization of the dorsal attentional network in left-handers with right sighting-eye: an evolutionary advantage. Hum Brain Mapp 36, 1151-1164, doi:10.1002/hbm.22693 (2015); Honda, H. Idiosyncratic left-right asymmetries of saccadic latencies: examination in a gap paradigm. Vision Res 42, 1437-1445 (2002); Weber, H. & Fischer, B. Gap duration and location of attention focus modulate the occurrence of left/right asymmetries in the saccadic reaction times of human individuals. Vision Res 35, 987-998, doi:10.1016/0042-6989(94)00186-p (1995)). However, most of these conflicting studies have been conducted in adults, rather than dyslexic adolescents. As rightward saccades are controlled by the contralateral ocular motor areas, involving visual, parietal, and frontal cortexes, perhaps the asymmetry in dyslexics could be attributed to left hemisphere dysfunction. Clinically, this finding could be related to dyslexic difficulty in fixating on words as they progress to the right along a line of text while reading. However, as previous studies on Chinese and Arabic speakers with dyslexia have contributed significantly to our understanding of the interplay between reading patterns and dyslexic pathology, futures studies should consider investigating these findings in dyslexics who read using non-Latin script (Siok, W. T., Perfetti, C. A., Jin, Z. & Tan, L. H. Biological abnormality of impaired reading is constrained by culture. Nature 431, 71-76, doi:10.1038/nature02865 (2004); Beland, R. & Mimouni, Z. Deep dyslexia in the two languages of an Arabic/French bilingual patient. Cognition 82, 77-126, doi:10.1016/s0010-0277(01)00148-2 (2001); Friedmann, N. & Haddad-Hanna, M. Letter position dyslexia in Arabic: from form to position. Behav Neurol 25, 193-203, doi:10.3233/ben-2012-119004 (2012)).

### Vergence velocity and disconjugate post-saccadic drift are correlated

For the first time to our knowledge, this study indicates problems in saccade velocity and high deconjugate drifts following large amplitude saccades in the dyslexic population. While it has previously been shown that dyslexics have deconjugate post-saccadic drifts during reading, the present study suggests this problem is inherent, as it exists for large saccades tested objectively with the REMOBI device. The results favor the hypothesis previously proposed by our lab: that disconjugate drifts arise from poor vergence control (Jainta, S. & Kapoula, Z. Dyslexic children are confronted with unstable binocular fixation while reading. PLoS One 6, e18694, doi:10.1371/journal.pone.0018694 (2011) ; Stein, J. & Kapoula, Z. Ch. Movements of the eyes in 3d space: deficits of vergence and binocular coordination in dyslexia, (Oxford University Press, 2012)). Further, our lab has previously shown that experimental induction of vergence/accommodation mismatch negatively impacts disconjugate post-saccadic drifts (Daniel, F. & Kapoula, Z. Induced vergence-accommodation conflict reduces cognitive performance in the Stroop test. Sci Rep 9, 1247, doi:10.1038/s41598-018-37778-y (2019)). Indeed, the present study argues that these vergence velocity abnormalities and disconjugate post-saccadic drifts are related. To substantiate this hypothesis regarding the physiologic link between vergence and saccades further, we examined the cross-correlation between some parameters. We found that the lower the velocity was during vergence, the higher the deconjugate drift of the saccades. This finding favors our hypothesis of a possible causal link between low vergence velocity capacity and poor binocular coordination after the saccade, or disconjugate drift.

When splitting the data of dyslexics and non-dyslexics, a significant negative correlation between average velocity and the disconjugate drift in the following 160 ms after the saccade was found for dyslexics only during convergence following left saccades. These deficits in saccades and vergence confirm that the dyslexic adolescent population exhibits difficulty in both controlling vergence along the median plane and maintaining vergence stability during fixation after saccades (seen in the increased disconjugate drift).

### No latency abnormality in dyslexics

Contrary to previous studies in our lab, we did not find any latency abnormality in dyslexics for vergence or saccades. However, contrary to our previous experiments, in this study, we used the visual-acoustic REMOBI device, which provides a sound stimulus 50ms before LED onset. Further research on dyslexics comparing eye movements to audiovisual vs visual targets are currently being conducted in our laboratory.

### Baseline Questionnaires

What else, outside of clinical and objective measures, could be associated with these eye movement abnormalities in the dyslexic population? Dyslexic adolescents tended to spend many more hours on the computer per week than non-dyslexic adolescents. It has been thought that dyslexic adolescents perform better when they are able to read and interact with a screen, so this finding may support their preference for screen learning.⁴¹⁻⁴³ Dyslexic adolescents also tended to like to go to theaters and films more than non-dyslexic adolescents, and, perhaps not surprisingly, liked reading less than non-dyslexic adolescents. We postulate that dyslexic adolescents also like to watch films more because they find reading too fatiguing. An open question that remains is to what extend prolonged used of computers aggravates the intrinsic vergence and binocular coordination problems of dyslexics.

### Next Steps

As we have shown that dyslexic adolescents have poor eye movement in both vergence and saccades as compared to non-dyslexic adolescents, how can this new data inform the dyslexic community's efforts to build rehabilitation and alternative learning programs for those affected?

Traditionally, dyslexic adolescents have been assigned to orthoptic rehabilitation, during which they perform exercises over many sessions designed to rehabilitate their eye movements. This helps many adolescents; however, of the 47 dyslexic adolescents studied, 34 had been to an orthoptist or were currently enrolled in orthoptic rehabilitation, yet continued to exhibit objective eye movement deficits and their clinical manifestations of those deficits. Are there any other methods that can be used to help them overcome their persistent deficits? Using computers instead of paper has been one strategy the dyslexic community has used to facilitate easier reading and writing in their population (Berninger, V. W., Nagy, W., Tanimoto, S., Thompson, R. & Abbott, R. D. Computer Instruction in Handwriting, Spelling, and Composing for Students with Specific Learning Disabilities in Grades 4 to 9. Comput Educ 81, 154-168, doi:10.1016/j.compedu.2014.10.005 (2015).; Tanimoto, S., Thompson, R., Berninger, V. W., Nagy, W. & Abbott, R. D. Computerized Writing and Reading Instruction for Students in Grades 4 to 9 With Specific Learning Disabilities Affecting Written Language. J Comput Assist Learn 31, 671-689, doi:10.1111/jcal.12110 (2015); van der Leij, A. Dyslexia and early intervention: what did we learn from the Dutch Dyslexia Programme? Dyslexia 19, 241-255, doi:10.1002/dys.1466 (2013)). However, spending so much time reading on a screen can have some side effects: namely, increasing visual stress, which could accentuate reading problems (Jaiswal, S. et al. Ocular and visual discomfort associated with smartphones, tablets and computers: what we do and do not know. Clin Exp Optom 102, 463-477, doi:10.1111/cxo.12851 (2019); Sheppard, A. L. & Wolffsohn, J. S. Digital eye strain: prevalence, measurement and amelioration. BMJ Open Ophthalmol 3, e000146, doi:10.1136/bmjophth-2018-000146 (2018); Mork, R., Falkenberg, H. K., Fostervold, K. I. & Thorud, H. M. S. Visual and psychological stress during computer work in healthy, young females-physiological responses. Int Arch Occup Environ Health 91, 811-830, doi:10.1007/s00420-018-1324-5 (2018); Collier, J. D. & Rosenfield, M. Accommodation and convergence during sustained computer work. Optometry 82, 434-440, doi:10.1016/j.optm.2010.10.013 (2011)).

On the other hand, perhaps these preliminary results related to eye movement deficits could be useful in planning retraining exercises. Previous studies using a double step vergence paradigm on the REMOBI machine have shown lasting improvements in reading saccades, fixations, and vergence measurements (Daniel, F., Morize, A., Bremond-Gignac, D. & Kapoula, Z. Benefits from Vergence Rehabilitation: Evidence for Improvement of Reading Saccades and Fixations. Front Integr Neurosci 10, 33, doi:10.3389/fnint.2016.00033 (2016); Ward, L. M., Gaertner, C., Olivier, L., Ajrezo, L. & Kapoula, Z. Vergence and accommodation disorders in children with vertigo: A need for evidence-based diagnosis. EClinicalMedicine 21, 100323, doi:10.1016/j.eclinm.2020.100323 (2020) ; Kapoula, Z. et al. Objective Evaluation of Vergence Disorders and a Research-Based Novel Method for Vergence Rehabilitation. Transl Vis Sci Technol 5, 8, doi:10.1167/tvst.5.2.8 (2016); Morize, A., Bremond-Gignac, D., Daniel, F. & Kapoula, Z. Effects of Pure Vergence Training on Initiation and Binocular Coordination of Saccades. Invest Ophthalmol Vis Sci 58, 329-342, doi:10.1167/iovs.16-19837 (2017); Kapoula, Zoï et al. Efficient Rehabilitation of Vergence Accommodation in Adolescents: A Case Study. J Clin Stud Med Case Rep 6: 074). Future research should repeat these experiments to verify audiovisual vergence retraining as a viable new rehabilitation technique. It may even be possible to use this method as a predictive test of dyslexic pathology using machine learning algorithms.

### Conclusion

Dyslexic adolescents exhibit slower vergence and saccades, with a velocity profile that shows a higher peak velocity in the beginning of the movement, but slow deceleration in the subsequent phase of movement towards targets in the three-dimensional space as compared to non-dyslexics. Though other studies have reported differences in saccadic and vergence eye movements in dyslexic adolescents, this study demonstrates, for the first time to our knowledge, an objective difference in the velocity profile of vergence and saccades in the dyslexic adolescent population. Slow average vergence velocity and disconjugate drifts following saccades can be causally related. Our study suggests dyslexics' poor control of vergence dynamics alters saccade speed and induces disconjugate post-saccadic drifts. These deficits in both vergence and saccadic movement provide an oculomotor basis for their reading difficulties: as it is more difficult to accurately and quickly locate the words in the three-dimensional space, it is more difficult to read.

Further, by demonstrating that these deficits persist when making large degree movements to audiovisual stimuli, this study opens up the possibility for novel therapeutic rehabilitation techniques. The proposed testing method assess purely motor problems independently from reading, which could become a useful clinical tool. Notably, the eye movement targets in this experiment mimic movements in everyday life: the targets were placed horizontally in the three-dimensional space at eye level and were preceded by a beep, which provides some warning and intersensory facilitation. This is an exciting area of research: though orthoptic training has been considered the standard of care rehabilitation for the dyslexic population, perhaps there are new avenues to design rehabilitation programs to train dyslexic adolescent eye movements to respond more quickly and accurately to these audiovisual stimuli.

Finally, and most importantly, we would like to highlight that reading is a multisensory activity that is fundamentally based around the visual system, of which eye movement is an integral component. Although we do not claim these eye movement problems represent the sole pathology in dyslexia, we believe the fragile nature of dyslexic adolescents' eye movements provide an inadequate basis on which to build efficient reading skills. These subtle eye movement deficits may represent a symptom of larger motor coordination disorders that may be associated with dyslexia and other learning disabilities. Therefore, we argue that these subtle eye movement testing should be added as differential diagnostic criteria and used for evaluation and rehabilitation in the dyslexic population.

### Example 3: Machine Learning classification of dyslexic vs non-dyslexic individuals on the basis of saccades and vergences parameters tested with REMOBI and analyzed with AIDEAL

In order to further confirm the relevance of vergence and saccade parameters measured during vergence and saccade tests using REMOBI, machine learning classification of dyslexic vs non-dyslexic individuals (29 dyslexic and 32 non-dyslexic adolescents) on the basis of the properties of their eye saccades, i.e. the latency, amplitude, speed, drift etc. parameters produced by AIDEAL after REMOBI stimulation (see Example 2 for details concerning REMOBI stimulation and AIDEAL processing of measured eyes movements) was attempted.

A KNeighborsClassifier was used as machine learning algorithm.

Results are presented in Table 6 below.

**Table 6. Accuracy, specificity and sensitivity of classification of 61 individuals (29 dyslexic and 32 non-dyslexic) using KNeighborsClassifier based on saccade parameters calculated by AIDEAL using eye movements measured during a saccade test using REMOBI.**

| Accuracy | Specificity | Sensitivity | Model | Penalty | C |
|---|---|---|---|---|---|
| 84.17% | 87.54% | 81.33% | KNeighborsClassifier | n.a | n.a |

These preliminary results show that saccade parameters calculated by AIDEAL based on REMOBI saccade test can excellently predict dyslexic individuals with 81% sensitivity (81% of dyslexic individuals are indeed classified as dyslexic) and 88% specificity (88% of non-dyslexic individuals are indeed classified as non-dyslexic).

To refine these results, the Lasso logistic regression was applied; this made it possible to identify that the most discriminating saccade parameters in this limited population are: average velocity of saccades, peak velocity of saccades, and disconjugacy during saccade.

The same approach was used for vergence parameters using two distinct well-known machine learning algorithms have been tested: support vector machine, and logistic regression.

Results are presented in Table 7 below.

**Table 7. Accuracy, specificity and sensitivity of classification of 61 individuals (29 dyslexic and 32 non-dyslexic) using support vector machine or logistic regression based on vergence parameters calculated by AIDEAL using eye movements measured during a vergence test using REMOBI.**

| Accuracy | Specificity | Sensitivity | Model | Penalty | C |
|---|---|---|---|---|---|
| 81.48% | 77.58% | 86.9% | support vector machine | l2 | 1 |
| 81.48% | 78.11% | 86.77% | logistic_regression | l2 | 10 |

The Support Vector machine learning model as well as the logistic regression method show a sensitivity of almost 87% to identify dyslexics and a specificity of about 78% to identify non-dyslexics. The application of the Lasso method indicates that the parameters calculated by AIDEAL that are the most discriminating for vergence are: average velocity of vergences, peak velocity of vergences, and duration of vergences.

These preliminary results confirm the relevance of measuring saccade and vergence parameters (in particular at least one of saccades and vergences average and peak velocity, disconjugacy during saccades and vergences duration) for the functional exploration as a diagnostic aid of dyslexic individuals.

### Example 4: Vergence/saccade tests using audiovisual stimulation or visual stimulation only.

Dyslexic and non-dyslexic individuals were submitted to vergence and saccade tests using REMOBI either with visual stimulation only or using audiovisual stimulation. Audiovisual stimulation was identical to that described in Example 2, and visual stimulation was identical with respect to visual stimulation, only audio stimulation was missing.

Results showed that, in healthy individuals not suffering from a learning disorder, ocular motor performances in a vergence or saccade test is generally better when the individual is audiovisually stimulated rather than when the individual is only visually stimulated. In particular, audiovisual targets lead to a latency decrease of leftward (190+/-34 ms with audiovisual targets versus 200+/-37 ms with visual targets only, Z = 2.5; p = 0.01) or rightward saccades (191+/-35 ms with audiovisual targets versus 203+/-32 ms with visual targets only, Z = 2.05; p = 0.04). The duration and average velocity of saccades is not significantly different in both stimulation conditions.

For vergence, the audiovisual targets lead to shortening of movement duration (convergence: 236+/-50 ms with audiovisual targets versus 274+/-57 ms with visual targets only, Z = 2.67; p = 0.0076; divergence: 284+/-45 ms with audiovisual targets versus 309+/-52 ms with visual targets only, Z = 2.63; p = 0.009) and to increase of their average velocity (convergence: 27+/-5 ° /s with audiovisual targets versus 23+/-4 ° /s with visual targets only, Z = 2.40; p = 0.016; divergence: 16+/-2 ° /s with audiovisual targets versus 15+/-2 ° /s with visual targets only, Z = 2.58; p = 0.009)

Healthy individuals thus better perform in saccade and vergence tests when audiovisually stimulated than when only visually stimulated.

However, in individuals suffering from dyslexia, audiovisual stimulation does not generally result in better performance in vergence or saccade tests than visual stimulation only. This is illustrated by **Figures 8-10****.**

**Figure 7** shows the vergence trajectories of a non-dyslexic individual after visual stimulation only (A) or audiovisual stimulation (B). An improvement of vergence trajectories can be seen when audiovisual stimulation is used instead of visual stimulation only.

**Figure 8** shows the vergence trajectories of a dyslexic individual after visual stimulation only (A) or audiovisual stimulation (B). No improvement of vergence trajectories is observed when audiovisual stimulation is used instead of visual stimulation only.

**Figure 9** further shows analysis of vergence parameters after visual stimulation only or audiovisual stimulation in dyslexic individuals. For vergence movements, dyslexic children had a significantly lower peak velocity in both divergent and convergent movements with multisensory stimulation than with single-sensory stimulation (Divergence: 96.00 vs 142.38, p = 0.005; Convergence 105.15 vs 208.31, p = 0.002).

They had a lower average velocity with multisensory stimulation than with single-sensory stimulation (Divergence: 30.53 vs 62.22; p = 0.000; Convergence 35.14 vs 59.08, p = 0.002). They further exhibited a longer duration during divergence (47.12 vs 29.53; p = 0.001).

Therefore, the inventors show for the first time that, contrary to the improvement of average velocity of vergences in healthy individuals obtained when using audiovisual stimulation instead of visual stimulation only, a further decreased average velocity of vergences is observed in dyslexic individuals.

**Figure 10** further shows analysis of saccade velocity parameters (A) and disconjugacy parameters (B) after visual stimulation only or audiovisual stimulation in dyslexic individuals. For saccadic movements, dyslexic children had a significantly greater peak velocity in both left and right movements with single (visual) stimulation than with multisensory (audiovisual) stimulation (Left: 239.39 vs 369.43, p = 0.003; Right: 249.15 vs 323.93, p = 0.003). Conversely, unlike in vergence movements, the average velocity was much greater in multisensory (audiovisual) stimulation than in single sensory (visual) stimulation for movements in both directions (Left: 242.93 vs 184.81, p = 0.004; Right: 245.85 vs 185.95, p = 0.035). Change vergence, or the disconjugacy, in multisensory (audiovisual) stimulation was much greater than in single stimulus (visual) testing (Left: 2.47 vs 1.09, p = 0.03; Right: 3.28 vs 2.35, p = 0.04). So, for dyslexics, a sound stimulus before the visual target makes the initial movement of the eyes in saccades slower in either direction. It additionally increases disconjugacy of the eyes during the saccadic movement as compared to a single-sensory target.

### Example 5: Comparison of accelerometer data in dyslexic versus non-dyslexic individuals during reading

Dyslexic and healthy (non-dyslexic) individuals were submitted to a reading test with an accelerometer mounted on their head.

On average, dyslexic individuals had at least 3 times more head rotations than non-dyslexic individuals when reading a line of text (data not shown).

### Example 6: Rehabilitation of vergence and saccade abnormalities in dyslexic individuals using vergence training sessions with double step vergence paradigm

It was then tested whether it was possible to improve binocular motricity in dyslexic individuals by vergence training sessions using the double step vergence paradigm, and whether improved binocular motricity resulted or not in improved reading.

### Patients, Materials and Methods

14 dyslexic individuals were submitted to:
i) a first vergence test and a first reading test,
ii) 2 vergence training sessions, and
iii) a second vergence test and a second reading test.

Vergence tests were performed as disclosed in Example 2.

Reading tests were performed as disclosed in Example 1.

Each vergence training session used the following double step vergence paradigm, and contained 5 blocks sequenced as follows: 1 block of divergence, 4 blocks of convergence. Each block contained 40 trials and lasted 2 to 3 minutes. A 1-minute pause was applied between blocks during which the subject was questioned on whether the LEDs were seen single, double, or blurred at each location; subjects were allowed to stand up and relax while resting.

Briefly, the target for convergence or divergence appeared first to a location; after 200ms, presumably before the accomplishment of the vergence eye movement, the target stepped to a second location. Given that the vergence latency is between 160 and 250ms, and vergence execution lasts between 350 and 550ms, it is almost certain that the second step of the target occurred before the initial vergence eye movement has been made. Data obtained during the vergence and reading tests were analyzed using the AIDEAL software as described in Example 2.

### Results and discussion

After only two vergence training sessions using the double step vergence paradigm, a statistically significant improvement for all children is observed, at least for the convergence eye movements. Exemplary vergence trajectories of a dyslexic individual obtained during a vergence test before and after rehabilitation by 2 vergence training sessions are presented in **Figure 11****.** A clear improvement of vergences, in particular for convergence movements, is seen after the two vergence training sessions.

The most interesting data is that following this improvement in convergence there is also an improvement in reading, in particular the speed of reading increases, and the number of regressive saccades decreases. Exemplary curves of the eyes movements of a dyslexic individual before and after the two vergence training sessions is presented in **Figure 12****.** A clear improvement in reading is also observed. In particular, following only two sessions of vergence rehabilitation, the dyslexic individual clearly reads faster, being able to read 3 text lines instead of two for the same duration. Moreover, while not yet comparable to those of a healthy individual (see **Figure 5A****),** progression of eyes movements during reading is more regular than before the two vergence training sessions.

This work can be consolidated with additional sessions. Learning to control the mobility of the gaze when reading is therefore possible and fast in dyslexic children when using a rehabilitation device such as REMOBI, due to the neuroplasticity of vergences present in these children.

### Example 7. Functional exploration of individuals suffering from a learning disorder using the method according to the invention

Functional exploration with a vergence test, a saccade test, a reading test and an image analysis test was performed in several individuals suffering from a learning disorder. Results obtained for a 16-years old dyslexic individual are presented in **Figure 13****:**
- **Figure 13A** shows eyes movements during reading (see Example **1** for description of the reading test).
- **Figure 13B** shows vergence trajectories during a vergence test (see Example 1 for description of the vergence test).
- **Figure 13C** shows saccade trajectories during a vergence test (see Example 1 for description of the saccade test).
- **Figure 13D** shows eyes movements during an image analysis test.

The individual is questioned about what is represented in the image and what story the image discloses. The sections of the image fixated by the eyes are also analyzed (the eyes trajectories may be reported on the image to see what parts of the image have been fixated and in which order) and compared to the verbal reports and understanding of the image provided by the individual. This type of test is used typically by neurologists and neuropsychologists for functional exploration of the attention and cognitive executives process. Comparison of verbal reports of the patient with the eye movement exploration enables better understanding on the deployment of the image analysis by the patient. Interestingly, some key parts of the image could be not commented by the person while still his/her eyes were fixating or vice versa.

**Figure 13** shows that, in this dyslexic individual, vergences and saccades are normal; although eye movements during reading are very disturbed. Here it is shown that the problem of eye movements during reading (back and forth between words) are related to the language disorder and not to intrinsic binocular motricity troubles. In the neurological test of the image, the exploration is normal.

Results obtained for a 19-years old dyspraxic individual are presented in **Figure 14****,** with the same type of data presented in A, B, C and D as in **Figure 13****.**

**Figure 14** shows that, in this dyspraxic individual, vergences and saccades are variable in the REMOBI tests, the reading pattern is near-normal, exploration during the neurological image reading test is very biased and partial.

Results obtained for a 12-years old dysphasic individual are presented in Figure 15, with the same type of data presented in A, B, C and D as in Figure 13.

Figure 15 shows that, in this dysphasic individual, very variable vergences and saccades are observed, as well as disturbed reading (regressive saccades). Moreover, normal semantic exploration of image and risk identification is observed, even if, according to the neurologist, the child sometimes has difficulty finding words.

The above results show that functional exploration based on a reading test, a vergence test, a saccade test, and an image analysis test permits to identify the presence or absence of intrinsic binocular motricity abnormalities, as well as the presence or absence of neurological deficits, thus showing the general usefulness of the methods according to the invention for the clinical management of individuals with learning disorders.

### Example 8. Dyslexics' Fragile Oculomotor Control Is Further Destabilized by Increased Text Difficulty

We compared eye movements while reading two different texts in a dyslexic and non-dyslexic population. It has been shown that a text that provides context clues to make sense of the story is read faster than a text with words placed in random order (Jenkins, J.R.; Fuchs, L.S.; Broek, P.V.D.; Espin, C.; Deno, S.L. Sources of Individual Differences in Reading Comprehension and Reading Fluency. J. Educ. Psychol. 2003, 95, 719-729). Screening of dyslexia, therefore, often uses a text that creates normal reading conditions with a grammatically and syntactically correct text. However, the text lacks any sort of meaning, making it impossible to predict the content of the story moving forward in the text. Therefore, there are no clues the reader could use to compensate for inherent decoding difficulties that might be present in a reader (Torgesen, J.; Rashotte, C.; Alexander, A. Principles of fluency instruction in reading: Relationships with established empirical outcomes. Dyslexia Fluen. Brain 2001, 333-355). In France, the "Alouette Test" is the most commonly used text to diagnose children and adults with dyslexia on the bases of abnormal scores of accuracy, speed, and efficiency and has been validated as a test with a high specificity and sensitivity for screening for dyslexia (Levafrais, P. Test de L'alouette: Manuel; Les Éditions du Centre de Psychologie Appliquée: Paris, France, 1967). We compared eye movements and reading scores while dyslexics and non-dyslexics read this text and an engaging, sensical text extracted from a book targeted towards children of 10 years of age (Gavalda, A. 35 Kilos D'espoir; Éditions des Terres Rouges: Carnoules, France, 2019).

We propose that oculomotor deficit could be altered by the different characteristics of the reading text. A text which requires more decoding skills could exacerbate the fragility of eye movements; while a text that provides more context and predictability may not perturb the eye as much. More specifically, the question remains: which parameters are particularly modulated by the choice of text? Is it reading speed and regressive saccades, or parameters more specifically associated with motor control per se, such as saccade velocity or disconjugacy?

### Materials and Methods

### Participants

A total of 47 dyslexic adolescents (18 female, 29 male; mean age 15.4) and 44 non-dyslexic adolescents (22 female, 22 male; mean age 14.8) were selected from middle and high schools in Paris. Each dyslexic adolescent was given a diagnosis of dyslexia via extensive multidisciplinary testing in specialized centers (including neurological/psychological testing, evaluation of reading, comprehension, and capacity of reading words and pseudowords). Each dyslexic child was admitted to their school on the basis of their diagnosis. In breaking down the types of dyslexia in the population, based on school records, 34.0%(16/47) identified their primary problem was visual/reading based, 4.3% (2/47) was auditory, 2.1% (1/47) was writing, and 59.6 (28/47) were mixed or unknown. As is common in the dyslexic population, many had co-morbid conditions: twelve were diagnosed with dysorthographia, dyscalcula, and/or dyspraxia. As is common in France, 34 had been to an orthoptist or were currently enrolled in orthoptic rehabilitation. All participants had no known neurologic or psychiatric abnormalities. Non-dyslexic adolescents had no difficult with vision, visual impairment, or difficulty reading. The investigation adhered to the principles of the Declaration of Helsinki and was approved by our Institutional Human Experimentation Committee (CPP CNRS 18 011). Written, informed consent was obtained from the adolescents and/or their parents after they were given an explanation about the experimental procedure.

### Eye Movement Recording Device

Binocular eye movements were recorded at 200 Hz per eye with a head-mounted video-oculography device called Pupil Core (Pupil Labs, Berlin, Germany).

### Calibration of the Recording Device

The device was calibrated with the standard calibration software (Pupil Capture, Pupil Labs). The subject fixate on the center of a target presented at a viewing distance of1 m. They then moved their head at their own pace rightward, downward, leftward, and upward twice.

### Texts

All children performed two visual reading tasks while seated comfortably at 40 cm viewing distance from a screen. Each adolescent was instructed to read the text outloud. First, each adolescent viewed binocularly the text L'Alouette, in 16 lines on black lines on a white background. L'Alouette is a text commonly used for evaluation of reading capacity in dyslexia, as the order of the words is unusual and contains uncommon words for children. The second text was an excerpt of 15 lines in black text on a white backgroundfrom a children's book (35 Kilos D'Espoir, Anna Gavalda, Bayard Jeunesse) targeted towards children with a reading age of 10 years. Please see **Figure 16A** and **Figure 16B** for texts.

### Data Analysis

The recorded data were analyzed with a software developed in the IRIS Laboratory called AIDEAL. The software treated the conjugate gaze signal, or the L + R eye position/2.The saccade was define as the time at which the peak velocity was greater or less than 10% of the peak velocity; practically this meant that saccades were above or below 40/s (as the peak velocity of 20 saccades is typically above 40/s). Total average velocity was define as total amplitude divided by time. Disconjugacy during saccadic movements, or the binocular coordination of saccades, was measured by the difference in amplitude between the left and the right eye signal. The difference in drift amplitude during the firs80 or 160 ms of fixation was calculated as the disconjugate drift. These calculations are standard and have been used in previous experiments.

Trials with blinks or other artifacts were discarded automatically by AIDEAL. For each adolescent (dyslexic and non-dyslexic) the number of saccades movements measured in the fixation tasks were counted. The percentage of movements rejected was 1.05% for dyslexic adolescents and 2.27% in healthy adolescents.

### Statistical Analysis

As the measured eye movements data were not normally distributed as determined by the Shapiro-Wilk test, the non-parametric Mann-Whitney U test was utilized for means comparison. In a firs analysis, we compared parameters between the dyslexic and non-dyslexic populations while reading L'Alouette. In a second test, we compared parameters between the two populations while reading an excerpt from 35 Kilos D'Espoir. We then compared parameters between reading Text 1 vs. Text 2 for the entire population. In a fourth test, we compared parameters between reading Text 1 vs. Text 2 in the dyslexic population only. Finally, we compared parameters between reading Text 1 and Text 2 in the healthy control population only. We did not attempt to correct for multiple comparisons.

For all analyses, the statistical significance was set at p 0.05. All analyses were performed using SPSS version 25 (IBM Corp. Released 2017. IBM SPSS Statistics for Windows, Version 25.0. IBM Corp. Armonk, NY, USA).

### Results

### Comparing Reading Performance and Eye Movements in each Text by Population

In comparing dyslexic adolescents to healthy controls while reading Text One (L'Alouette), dyslexics exhibited several abnormalities in eye movement control as compared to non- dyslexics: a smaller amplitude (1.93, SD 0.36 vs. 2.23, SD 0.45; p < 0.001), a longer duration(64.97 ms vs. 44.07 ms, p = 0.001), a higher peak velocity (80.82/ms vs. 66.30/ms ;p = 0.005) yet a lower average velocity (42.13/ms vs. 64.24/ms, p < 0.001), and a larger fixation disconjugacy during the saccade (0.76 vs. 0.61, p-0.037). These finding are consistent with eye movement abnormalities previously found in dyslexics independent of reading using Remobi testing (see Example 2 and Ward, L.M.; Kapoula Z. Differential diagnosis of vergence and saccade disorders in dyslexia. Sci. Rep. 2020, 10, 22116). They also had a larger percentage of regressive saccades (saccades to the left) (35% vs. 28%; p = 0.014), made more mistakes per word of text (0.06 mistakes/word vs. 0.03 mistakes/word, p < 0.001), and read more slowly (92 words per minute vs. 136 words per minute; p < 0.001) (See **Table 8).**

**Table 8. Reading L'Alouette**

| | Dyslexic | | Non-Dyslexic | | p-value |
|---|---|---|---|---|---|
| | Median | SD | Median | SD | |
| Amplitude (deg) | 1.93 | 0.36 | 2.23 | 0.45 | <0.001 |
| Duration (ms) | 64.97 | 67.99 | 44.07 | 48.70 | <0.001 |
| Peak velocity (deg/s) | 80.82 | 35.49 | 66.30 | 41.68 | 0.01 |
| Average velocity (deg/s) | 42.13 | 16.14 | 64.24 | 19.81 | <0.001 |
| Fixation Disconjugacy 80 msec after Saccade (deg) | 0.36 | 0.37 | 0.037 | 0.43 | 0.92 |
| Fixation Disconjugacy 160 msec after Saccade (deg) | 0.61 | 0.64 | 0.56 | 0.60 | 0.64 |
| Disconjugacy During Saccade (deg) | 0.76 | 2.06 | 0.61 | 1.01 | 0.04 |
| Fixation Duration (ms) | 410.28 | 88.08 | 447.58 | 91.49 | 0.05 |
| Percent Regressive Saccades | 35.46 | | 28.24 | | 0.01 |
| Mistakes per Word | 0.06 | 0.06 | 0.026 | 0.02 | <0.001 |
| Words per Minute | 92 | 109 | 136 | 29 | <0.001 |

In comparing the two populations while reading the simpler text, these differences persisted though there were a few notable exceptions. There was no longer any significant difference between the populations in the fixation disconjugacy during the saccade, nor in the percentage of regressive saccades (See **Table 9).**

**Table 9. Reading 35 Kilos D'Espoir.**

| | Dyslexic | | Non-Dyslexic | | p-value |
|---|---|---|---|---|---|
| | Median | SD | Median | SD | |
| Amplitude (deg) | 2.15 | 0.50 | 2.56 | 0.44 | 0.00 |
| Duration (ms) | 55.71 | 21.97 | 40.54 | 111.66 | 0.04 |
| Peak velocity (deg/s) | 77.77 | 18.74 | 69.92 | 38.63 | 0.04 |
| Average velocity (deg/s) | 52.92 | 17.19 | 76.56 | 21.57 | 0.00 |
| Fixation Disconjugacy 80 msec after Saccade (deg) | 0.32 | 0.19 | 0.37 | 0.27 | 0.28 |
| Fixation Disconjugacy 160 msec after Saccade (deg) | 0.50 | 0.36 | 0.54 | 0.50 | 0.72 |
| Disconjugacy During Saccade (deg) | 0.64 | 1.84 | 0.64 | 0.96 | 0.55 |
| Fixation Duration (ms) | 381.66 | 54.99 | 406.15 | 93.06 | 0.10 |
| Percent Regressive Saccades | 31.44 | | 26.56 | | 0.42 |
| Mistakes per Word | 0.03 | 0.03 | 0.015 | 0.01 | 0.00 |
| Words per Minute | 124 | 33 | 176 | 34 | 0.00 |

### Comparing Texts in Each Population

There were also significan differences in the dyslexic population in reading the two texts (see **Table** 10). When reading L'Alouette, dyslexics displayed a smaller amplitude (1.93 vs. 2.15; p = 0.004) yet a faster velocity (42.13/ms vs. 52.92/ms; p = 0.007), as compared to when they were reading the less complicated text, 35 Kilos D'Espoir. This may be expected given one would expect a smaller saccade would necessitate a shorter velocity to make the movement. They also made more regressive saccades (35% vs. 31%,p = 0.036), made more mistakes per word of text (0.06 vs. 0.03, p < 0.001), and read more slowly (92 words per minute vs. 124 words per minute, p < 0.001).

**Table 10. Comparing Texts in the Dyslexic Population.**

| | Text 1 | | Text 2 | | p-value |
|---|---|---|---|---|---|
| | Median | SD | Median | SD | |
| Amplitude (deg) | 1.93 | 0.36 | 2.15 | 0.50 | 0.00 |
| Duration (ms) | 64.97 | 67.99 | 55.71 | 21.97 | 0.07 |
| Peak velocity (deg/s) | 80.82 | 35.49 | 77.77 | 18.74 | 0.22 |
| Average velocity (deg/s) | 42.13 | 16.14 | 52.92 | 17.19 | 0.01 |
| Fixation Disconjugacy 80 msec after Saccade (deg) | 0.36 | 0.37 | 0.32 | 0.19 | 0.33 |
| Fixation Disconjugacy 160 msec after Saccade (deg) | 0.61 | 0.64 | 0.50 | 0.36 | 0.29 |
| Disconjugacy During Saccade (deg) | 0.76 | 2.06 | 0.64 | 1.84 | 0.11 |
| Fixation Duration (ms) | 410.28 | 88.08 | 381.66 | 54.99 | 0.25 |
| Percent Regressive Saccades | 35.46 | | 31.44 | | 0.04 |
| Mistakes per Word | 0.06 | 0.06 | 0.03 | 0.03 | 0.00 |
| Words per Minute | 92 | 109 | 124 | 33 | 0.00 |

Healthy controls, unsurprisingly, also displayed differences in eye movements and reading behavior between the two texts (see **Table 11).** When reading L'Alouette, healthy controls also displayed a smaller amplitude (2.23 vs. 2.56; p = 0.002), made more mistakes per word (0.026 vs. 0.015, p < 0.001), and read more slowly (136 words per minute vs.176 words per minute, p < 0.001). The majority of these differences between reading texts independent of population tested are related to language processing (regressive saccades, mistakes per word, and word per minute) and not to eye movement control perse, reflecting the cognitive aspects of oculomotor control.

**Table 11. Comparing Texts in the Control Population.**

| | Text 1 | | Text 2 | | p-value |
|---|---|---|---|---|---|
| | Median | SD | Median | SD | |
| Amplitude (deg) | 2.23 | 0.45 | 2.56 | 0.44 | 0.00 |
| Duration (ms) | 44.07 | 48.70 | 40.54 | 111.66 | 0.70 |
| Peak velocity (deg/s) | 66.30 | 41.68 | 69.92 | 38.63 | 0.76 |
| Average velocity (deg/s) | 64.24 | 19.81 | 76.56 | 21.57 | 0.06 |
| Fixation Disconjugacy 80 msec after Saccade (deg) | 0.037 | 0.43 | 0.37 | 0.27 | 0.97 |
| Fixation Disconjugacy 160 msec after Saccade (deg) | 0.56 | 0.60 | 0.54 | 0.50 | 0.61 |
| Disconjugacy During Saccade (deg) | 0.61 | 1.01 | 0.64 | 0.96 | 0.82 |
| Fixation Duration (ms) | 447.58 | 91.49 | 406.15 | 93.06 | 0.22 |
| Percent Regressive Saccades | 28.24 | | 26.56 | | 0.91 |
| Mistakes per Word | 0.026 | 0.02 | 0.015 | 0.01 | 0.00 |
| Words per Minute | 136 | 29 | 176 | 34 | 0.00 |

### Discussion

### Velocity Profil during Reading Demonstrate Poor Oculomotor Coordination

Similar to our previous study of dyslexic and non-dyslexic vergence and saccade movements to randomly generated targets, we found that dyslexics' reading saccades also showed abnormal velocity profile (see Example 2 and Ward, L.M.; Kapoula Z. Differential diagnosis of vergence and saccade disorders in dyslexia. Sci. Rep. 2020, 10, 22116). As peak velocity, which is achieved early in the trajectory of the movement, is higher in dyslexics, a slower average velocity indicates slowing of the deceleration in the subsequent phase of movement. We attributed this slowing to poor control of vergence during the saccade that is necessary to keep binocular eye alignment during and after the movement.

There is one important difference in eye movement parameters between the two texts. While reading L'Alouette, the text that required greater use of word decoding skills, dyslexics demonstrated a greater disconjugacy during the saccade, meaning that their eyes were poorly coordinated as they moved from one word to the next. This finding is similar to our previous study using non-reading conditions, which showed a greater disconjugacy of saccades to audiovisual targets in dyslexics (see Example 2).

In the present study, abnormal disconjugacy appeared once again, though only while reading L'Alouette. When adolescents are confronted with a text that relies heavily on word decoding and provides no context clues to help extract meaning, perhaps the binocular motor coordination system, which hypothetically involves saccade-vergence interplay, is less efficient thereby leading to disconjugacy. Reading normally implies a rather automatic motor sequence of the eyes from left to right. Dyslexics' difficult in decoding words to extract meaning exacerbates dyslexics' already fragile oculomotor system, not only potentially revealing abnormal velocity profile and greater disconjugacy during eye movements, but also creating more regressions, more mistakes, and a slower reading time.

In other words, we propose that lack of sense destabilizes both motor aspects and cognitive aspects of eye movement control in dyslexia.

Interestingly, dyslexic children were shown to coordinate their eyes just as well as non-dyslexic children while reading 35 Kilos D'Espoir, the text which provides greater context clues to the reader and decreases reliance on word decoding ability. Perhaps when dyslexics read a text that carries more context clues and does not rely heavily on word decoding skills, they are able to extract meaning and move more smoothly along a line of text similarly to non-dyslexic readers. These observations are in line with our previous studies. Given that these eye movement abnormalities in saccades and vergence exist outside of reading, we propose that the difference between the two reading texts presented here could be understood as evidence for a fragile oculomotor system that is perturbed when attempting to decode a meaningless text.

### Regressive Saccades Provide Insight into How Dyslexics Internalize Reading Text

There are some new insights we can gain into the differences between dyslexic and non-dyslexic eye movements during reading. When reading L'Alouette, the text which requires the reader to rely more heavily on individual word decoding skills, dyslexics exhibited more regressive saccades as compared to non-dyslexics, meaning their eyes moved backwards to the previous word more frequently, indicating difficult with fully internalizing the word. Though we would expect this to be true of how dyslexics readall texts, we found that there was actually no significant difference in regressive saccades between the populations while reading 35 Kilos D'Espoir, a text that provided more context clues and narrative to create a cohesive story.

It is important to note that these regressive saccades more likely represent a cognitive disability as opposed to a pure oculomotor deficit As dyslexics had more difficult during word decoding, they attempted to regress backwards to the previous word in order to search for a coherent story to fin meaning in the text. Non-dyslexics, who do not struggle with word decoding, are more likely to understand the meaning of the word as they progress across the line and do not have to search for context clues via regressive saccades. It is notable that dyslexics did not have more regressive saccades as compared to non-dyslexics when reading 35 Kilos D'Espoir. We postulate this is because the latter text provides more context clues throughout the text to construct a coherent story, so that dyslexics can understand the trajectory of the narrative without needing to focus as much on the meaning of each word. This concept is confirmed in the fact that dyslexics also showed more regressive saccades when reading L'Alouette as compared to reading 35 Kilos D'Espoir. Even though dyslexics have a fragile oculomotor system with poor coordination, they demonstrated more regressive saccades in the text with higher word decoding requirements, confirming that these regressive saccades are more likely secondary to a cognitive issue than a motor one.

Regressive saccades can represent a healthy phenomenon in that it could be the result of a trigger to move backwards when one views a word that is incomprehensible. Therefore, these regressive saccades are not purely or necessarily triggered by pure intrinsic motor control, and it is likely that a portion of these regressive saccades are related to a poor cognitive recognition of the material.

### Cognitive Aspects of Reading Differences

From a cognitive perspective, dyslexics exhibit increased errors and a slower reading speed in both texts, confirming dyslexics do indeed read more slowly and with more difficult, regardless of the text or its cognitive load.

Additionally, there were significant differences in amplitude in each of the comparison groups. Dyslexics demonstrated a smaller amplitude as compared to non-dyslexics while reading both the difficult and the easier text. Previous studies have demonstrated different reading strategies in the dyslexic vs. non-dyslexic population, in which dyslexics preferentially utilize an indirect grapheme/phoneme strategy, breaking words down into smaller pieces and therefore using a smaller amplitude to move across the word in shorter segments. Alternatively, non-dyslexics favor a whole word lexical strategy, which would provide a larger amplitude as the adolescent moves across the whole word (Trauzettel-Klosinski, S.; Koitzsch, A.M.; Dürrwächter, U.; Sokolov, A.N.; Reinhard, J.; Klosinski, G. Eye movements in German-speaking children with and without dyslexia when reading aloud. Acta Ophthalmol. 2009, 88, 681-691). Werth has also found that segmentation of text and longer fixation times improves oculomotor abnormalities (Werth, R. What causes dyslexia? Identifying the causes and effective compensatory therapy. Restor. Neurol. Neurosci. 2019, 37,591-608; Werth, R. Dyslexic Readers Improve without Training When Using a Computer-Guided Reading Strategy. Brain Sci. 2021, 11, 526). It is possible that segmenting the text into smaller portions reduces the need for accommodative effort, and therefore for accommodative vergence, decreasing the stress on the dyslexic child and allowing for more coordinated movements. This is, unfortunately, not sustainable for dyslexic readers and ultimately not representative of how readers view the world around them; by changing the way the subject sees the text, they also change the way the visual system absorbs that text. This demonstrates a more integrative view of how various sensory inputs can influence oculomotor abnormalities and the general presentation of dyslexia; giving a more nuanced picture of the etiology of the condition.

When comparing the texts within each population, both dyslexics and non-dyslexics demonstrated smaller amplitudes while reading the more difficult text. Dyslexics and non-dyslexics both had a larger amplitude while reading the second text, which was easier. Perhaps the easier text has words that dyslexics are able to understand with an easier cognitive load, allowing them to internalize the word as a whole instead of breaking it down into smaller portions.

This modulation in amplitude demonstrates that there may be oculomotor parameters that could be a reflection of alternative cognitive perception in the dyslexic population. Therefore, it appears that one could consider a multidimensional approach to dyslexia, in which oculomotor control and cognition are closely related.

### Limitations

It has been shown that dyslexic oculomotor control can be improved with training exercises and generally improves with age. Many of our dyslexic subjects have participated in orthoptic reeducation, which is common in France. Indeed, our subjects were selected from a school that specializes in educating dyslexics, with specific classes for reading acquisition. Children in these schools are closely monitored at all levels (many years of speech, visual, and/or orthoptic therapy etc.). However, despite their enrollment in these programs, our finding still demonstrated significant differences in their oculomotor profile and reading ability, suggesting that neither speech therapy nor the standard of care orthoptic reeducation are adequately targeted towards improving oculomotor movements and reading skills.

### Conclusions

This study shows that there are both cognitive and oculomotor components to the differences in how dyslexics read compared to their healthy peers. A text can induce word decoding difficulties in its structure that may worsen dyslexics already fragile oculomotor system. From this study, the L'Alouette test, which has been widely used throughout France in screening for dyslexia, proves to be a valid test to tease out not only decreased reading speeds and increased mistakes, but also a fragile oculomotor system in a dyslexic population. In addition to simply counting mistakes and measuring reading time, clinicians can use the velocity profile of saccade and vergence eye movements during and independent of reading in conjunction with the standard dyslexia tests, to measure eye movement fragility. As the L'Alouette test is considered to be predictive of dyslexia, perhaps there could a larger investigation to determine if it is the text or the eye movement abnormality itself caused by such a text that enables differences predictive of dyslexia. Future research should also be conducted in other languages and countries using text analysis and oculomotor monitoring to further understand the relationship between word decoding difficulties and oculomotor deficit in the dyslexic population.

### Example 9. Predicting Dyslexia and Reading Speed in Adolescents from Eye Movements in Reading and Non-Reading Tasks: A Machine Learning Approach

Despite the many machine learning (ML) studies that have been conducted on dyslexic eye movements, they are limited to using data extracted only from eye movements during reading. Therefore, it is difficult to determine how text difficulty may influence ML interpretation of data or how eye movements independent of reading may be used to predict dyslexia. To summarize, there have been no studies that apply ML on a complete physiologically eye movement data set; namely, on the ensemble of eye movements tested both during complex and simple reading tasks as well as during neutral tasks such as large saccades and vergence to LED targets. Given that the predictive value of eye movements during reading has been shown to be dependent on the text, there is a real need to compare ML analysis on non-reading tests. The present study uses the data sets from two studies by Ward and Kapoula: the first testing large saccades and vergence with LED targets, and the second testing reading in two texts of varying difficulty to evaluate the capacity of eye movements to predict dyslexia (Kapoula, Z.; Bucci, M.P.; Jurion, F.; Ayoun, J.; Afkhami, F.; Brémond-Gignac, D. Evidence for frequent divergence impairment in French dyslexic children: Deficit of convergence relaxation or of divergence per se? Graefe's Arch. Clin. Exp. Ophthalmol. 2006, 245, 931-936 ; Bucci, M.P.; Bremond-Gignac, D.; Kapoula, Z. Poor binocular coordination of saccades in dyslexic children. Graefe's Arch. Clin. Exp. Ophthalmol. 2007, 246, 417-428). Eye movements from all tasks were analyzed with the AIDEAL software to produce measurements of multiple physiologically valid spatiotemporal descriptors of eye movements (e.g., latency, amplitude, duration, peak, average velocity, drifts, etc.) We applied several ML models (linear and non-linear) and evaluated their differential accuracy and capacity to predict dyslexia, as well as the reading speed.

### Materials and Methods

### Participants

Given that the data used for the ML analysis is taken from published studies, please see Ward and Kapoula for full details regarding methods and materials (Kapoula, Z.; Bucci, M.P.; Jurion, F.; Ayoun, J.; Afkhami, F.; Brémond-Gignac, D. Evidence for frequent divergence impairment in French dyslexic children: Deficit of convergence relaxation or of divergence per se? Graefe's Arch. Clin. Exp. Ophthalmol. 2006, 245, 931-936 ; Bucci, M.P.; Bremond-Gignac, D.; Kapoula, Z. Poor binocular coordination of saccades in dyslexic children. Graefe's Arch. Clin. Exp. Ophthalmol. 2007, 246, 417-428). In brief, eye movements were recorded during reading and non-reading tasks in 46 dyslexic adolescents (18 female, 28 male; mean age 15.52, SD 2.45) and 41 non-dyslexic adolescents (20 female, 21 male; mean age 14.78 +/- 2.44) recruited from schools in Paris. Dyslexic adolescents were diagnosed in specialized medical centers and were admitted to their schools based on their dyslexia diagnosis. Typically diagnosis by these centers include multiple testing which confers a diagnosis of visual, phonologic dyslexia, mixed dyslexia, etc. We therefore did not have children self-identify their condition. Both dyslexic and non-dyslexic adolescents had no known neurologic or psychiatric abnormalities. Typical readers had no history of reading difficulty, no visual impairment, or difficulty with near vision. Of dyslexics, 34.0% (16/47) identified that their primary issue was visual/reading based, 4.3% (2/47) auditory, 2.1% (1/47) writing, and 59.6 (28/47) were mixed or unknown. Unfortunately, our small sample size limited further analysis by category.

Dyslexia commonly comports other comorbidities: twelve dyslexics were also diagnosed with dysorgraphia, dyscalcula, and/or dyspraxia. A total of 34 had been to an orthoptist or were currently enrolled in orthoptic rehabilitation.

The investigation adhered to the principles of the Declaration of Helsinki and was approved by our Institutional Human Experimentation Committee (CPP CNRS 18 011). Written consent was obtained from the adolescents and/or their parents after they were given an explanation about the experimental procedure. The tests were conducted by two research assistants, who were trained together using the same material and conducted the experiment together for each measurement.

### Eye Movement Recording Device

For each adolescent, eye movements were recorded binocularly with a head-mounted video-oculography device, Pupil Core (Pupila Capture Eye Tracker. Available online: https://pupil-labs.com/), enabling binocular recording at 200 Hz per eye (Pupil Labs, Berlin, Germany).

### Ocular Motor Tests

Adolescents were asked to sit in front of a horizontal visual-acoustic REMOBI device. The device was placed at eye level so that the first arc of LEDs was 20 cm from their eyes. Each child was instructed to carefully fixate quickly and accurately on the moving LED, to not attempt to predict a pattern of motion, and to keep their head still during the 2-min test.

Oculomotor tests were performed in mesopic light conditions. Each test was conducted in a small room in the school that was quiet and free of distractions. The red LED stimuli were displayed at different distances, laterally or in-depth, always in the horizontal plane (0°). LED characteristics were: nominal frequency 626 nm, intensity 180 mCd, and diameter 3 mm. Adjacent to each LED was embedded a buzzer with the following characteristics: nominal frequency approximately 2048 Hz, sound pressure level 75 dB, diameter 12 mm.

### Vergence Test

For each trial the fixation LED (0°) lights up at 40 cm, creating a required vergence angle of 9° for a period varying from 1400 to 2000 ms. It was followed randomly by the target LED during 2000 ms, appearing always at the central axis (0°) either 20 cm (calling for a convergence movement of 8°, i.e., from 9° to 17°) or 150 cm (calling for a divergence movement of 7°, i.e., from 9° to 2). The test contained 40 trials (20 trials of convergence, 20 of divergence, pseudo-randomly interleaved) in an overlap paradigm: after an overlap period of 200 ms following the lighting of the target LED, the fixation LED switched off.

### Saccade Test

Each trial started with the fixation central LED lighting at 40 cm from the subject for a randomized period ranging from 1400 to 2000 ms; it was followed by the lighting of the saccade target LED for 2000 ms at 20° of eccentricity, randomly chosen on the left or the right. There were 40 trials (20 left, 20 right, pseudo-randomly interleaved) in an overlap paradigm.

### Reading Test

All children performed two visual reading tasks with binocular vision while seated comfortably at 40 cm viewing distance from a screen. Each adolescent was instructed to read the text out loud. First, each adolescent viewed the Alouette text, presented in 16 lines on black lines on a white background. Alouette is a text commonly used for the evaluation of reading capacity in dyslexia, as the order of the words is unusual and contains uncommon words for children. The second text was an excerpt of 15 lines in black text on white background from a children's book (35 kilos d'espoir, Anna Gavalda, Bayard Jeunesse) targeted towards children with a reading age of 10 years.

### Data Analysis

Data recorded with the Pupil Labs eye tracker were analyzed with AIDEAL, software developed in the IRIS laboratory. The vergence signal was derived by calculating the difference between the two eyes from the individual calibrated eye position signals (i.e., left eye-right eye). The beginning and end of the vergence movements were defined as the time point when the eye velocity exceeded or dropped below 5° /s: these criteria are standard and were applied automatically by the AIDEAL software; the program estimated the initial phasic component as the amplitude between these two initial points. It also calculated the amplitude change during the subsequent 80 ms and 160 ms. The total amplitude was calculated as the sum of the amplitude of the initial phasic component plus the 160 ms component. The total duration was calculated as the duration of the phasic component plus the subsequent 160 ms.

For saccade analysis, AIDEAL treated the conjugate signal, e.g., the L + R eye position/2. The onset and the offset of the saccade were defined as the time points where the peak velocity went above or below 10% of the peak velocity; practically, this corresponded to values above or below 40° /s (as the peak velocity of 20° saccades is typically above 40° /s). The total average velocity was defined as the ratio of total amplitude in degrees divided by time in seconds. To evaluate binocular coordination of saccades, or the disconjugacy during saccadic movements, the difference in amplitude between the left and the right eye signal was calculated. The disconjugate drift, or the difference in drift amplitude during the first 80 or 160 ms of fixation, was calculated.

**Table 12** presents the results of the two reading tasks as the number of mistakes per word and the reading speed (word per minute).

**Table 12. Dyslexic vs. Non-Dyslexic performance for each reading task.**

| | **Dyslexic** | | **Non-Dyslexic** | |
|---|---|---|---|---|
| | **Median** | **SD** | **Median** | **SD** |
| Mistakes per word (35 Kilos d'Espoir) | 0.03 | 0.03 | 0.015 | 0.01 |
| Words per Minute (35 Kilos d'Espoir) | 124 | 33 | 176 | 34 |
| Mistakes per word (L'Alouette) | 0.06 | 0.06 | 0.026 | 0.02 |
| Words per Minute (L'Alouette) | 92 | 109 | 136 | 29 |

### Eye Movement Descriptors

Amplitude: the amplitude of the movement (in degrees), i.e., the change in eye position between the onset and the offset of the eye movement; the onset and offset of eye movement being determined by velocity threshold criteria as described above using AIDEAL software.

Duration of the phasic component: the time of execution of the movement (in ms), i.e., the time between the onset of the movement and its offset.

Latency: the period between the onset of the LED target and onset of the eye movement (in ms).

P-Velocity: Peak velocity of the eye movement (in degrees per sec); the peak velocity is usually achieved at the first 3rd of the trajectory of the movement.

A-velocity: Average velocity, which is obtained via the ratio of the amplitude of the movement (in degrees) over its duration (in seconds).

Drift 1: is the amplitude (in degrees) of disconjugate drift of the eyes (i.e., subtraction amplitude of the drift of the right eye from that of the left eye) for the 80 ms period starting from the offset of the movement.

Drift 2: Is the amplitude of the disconjugate drift during the 160 ms period following the offset of the movement (in degrees).

Total Amplitude: The addition of the phasic amplitude and the vergence drift during the 160 msec.

Fixation duration: the period (in ms) between two successive fixations.

Percentage of regressive saccades: "backward" saccades to the left calculated as a portion of the number of the rightward, progressive saccades.

The descriptors used for each dataset are presented in **Table 13** below:

**Table 13. List of the descriptor used for each model**

| **Dataset.** | **Eye Movement Descriptors** |
|---|---|
| Remobi Saccade | Amplitude, Duration of the phasic component, Latency, P-Velocity, A-velocity, Drift 1, Drift 2 |
| Remobi Vergence | Amplitude, Duration of the phasic component, Latency, P-Velocity, Drift 1, Drift 2, Total Amplitude, Average velocity |
| Meaningful Reading | Amplitude, Duration of movement, Latency, P-Velocity, A-velocity, Drift 1, Drift 2, Fixation duration, Percentage of regressive saccades |
| Alouette Reading | Amplitude, Duration of movement, Latency, P-Velocity, A-velocity, Drift 1, Drift 2, Fixation duration, Percentage of regressive saccades |

### Preprocessing of the Dataset

For each individual and each test, and each of the above-cited parameters, the AIDEAL software provides the mean value (based on 10 to 20 trials), the standard deviation, the coefficient of the variation (standard deviation/mean × 100), and the total number of trials used for each descriptor.

We considered that outlier values from AIDEAL do not occur randomly and thus convey information on the recorder gaze. For example, aberrant values could be due to blinks or artefacts of eye movement recording apparatus. We therefore chose to give these abberrances a value of 0 instead of the population average.

To remove the outliers, for the classification task (Analysis 1), we replaced the aberrant value by 0. The number of aberrant values in the 1st analysis was 0.29% for meaningful reading, 0.84% for the reading Alouette, 1.10% for the vergence, and 7.3% for the saccade dataset.

For the regression task (Analysis 2), for each dataset and each eye movement parameter, we splitthe dataset by class; then, we replaced values of the first 3% percentile, and the last 3% percentile of each eye movement parameter by its mean value. The number of outliers in the 2nd analysis was 8.7% for the vergence dataset, 9.3% for the saccade dataset, and 4.3% for the meaningful reading dataset.

Finally, we applied a random permutation to change the index of each observation in the dataset.

### Metrics

To evaluate our ML algorithms, for the analysis 1, we used 3 metrics: accuracy, sensitivity, and specificity. In our binary classification, the sensitivity (True Positive rate) measures the proportion of positives that are correctly identified (i.e., the proportion of those who have some condition (affected) who are correctly identified as having the disease). The specificity (True Negative rate) measures the proportion of negatives that are correctly identified (i.e., the proportion of those who do not have the condition or the unaffected who are correctly identified as not having the condition).

Moreover, for the analysis 2, we used the mean absolute percentage error (de Myttenaere, A. et al. Mean Absolute Percentage Error for regression models. Neurocomputing 2016, 192, 38-48, doi:10.1016/j.neucom.2015.12.114) and the Bravais-Pearson correlation metrics.

### Model Fitting

### ANALYSIS 1: Classifying Dyslexia from Eye Movement Descriptors Provided by AIDEAL

In the first ML analysis, the goal was to test the capacity of the model to predict if the person is dyslexic or not dyslexic from eye movement descriptors. Therefore, we used multiple linear classification models such as logistic regression, linear SVM, Gaussian naive Bayes, and linear discriminant analysis. However, linear models's decisions boundaries are linear. Since, in our dataset, the samples were not fully linearly separable, to get a better decision boundary, we additionally used multiple non-linear models such as radial basis function SVM (with gamma = 0.01), k-nearest neighbors, gaussian process classifier, Quadratic discriminant analysis, and multi-layer perceptron. We also used some tree-based algorithms such as random forest classifier and decision tree classifier.

We also added Lasso regularisation whenever possible to reduce overfitting. Overfitting occurs when the models start to adjust their parameters to learn the noise present in the dataset rather than generalizing. To reduce it, one can reduce the complexity of the model by adding regularisation techniques such as the l1 norm (Lasso regularisation) and the l2 norm (Tikhonov regularisation) (Ng, A.Y. Feature selection, L1 vs. L2 regularization, and rotational invariance. In Proceedings of the Twenty-First International Conference on Machine Learning, Banff, Alberta, 4-8 July 2004; doi:10.1145/1015330.1015435)

To summarize, our training procedure, for the four eye movements tests, we used cross-validation (Arlot, S.; Celisse, A. A survey of cross-validation procedures for model selection. Stat. Surv. 2010, 4, 40-79, doi:10.1214/09-ss054) (5 fold). At each fold, the model is fit with the train set, and its generalization ability is evaluated on the test set. We report the average test performance on the five folds. At each fold, the train set and test set were first normalized by subtracting the mean of the training set then dividing by the standard deviation of the training set. Since the size of the dataset did not allow for the creation of a representative validation set, we do not perform any hyperparameter tuning and use the default values of the different models in their scikit-learn implementation. To compare the performance of two models, we have combined the three criteria in the following way; we first use the accuracy to compare the two models. If the accuracy score is the same for two models, then the sensitivity score was used to select the best model. Finally, if the accuracy and the sensitivity score of the two models are equal, the specificity score was used.

### ANALYSIS 2: Predicting Reading Speed Based on Eye Movements Descriptors

In this analysis, we applied a ML algorithm on the eye movement descriptors from the three tests (saccade, vergence, and reading the meaningful text) to predict the reading speed (defined as the number of words read per minute) with a meaningful text.

Recall that reading speed is the number of words read per minute. It is known that reading speed is slower in dyslexics, and the reading speed during the Alouette test has been found to predict dyslexia with high accuracy (Cavalli, E.; et al. Screening for Dyslexia in French-Speaking University Students: An Evaluation of the Detection Accuracy of the Alouette Test. J. Learn. Disabil. 2018, 51, 268-282, doi:10.117710022219417704637; Erratum in 2018, 51, NP1). We used cross-validation to fit linear regression, 2-layers neural networks with 60 parameters, and support vector regression (RBF with gamma 0.01) models by applying the same procedure described in the first analysis to train the models cited above.

We have also made feature selection by keeping only the 10 most correlated features with the reading speed. The feature selection was done at each fold of the cross validation, so that we use only the training set of each iteration to select 10 features that will be used to train the model. We therefore improved the generalization ability of the models by reducing their complexity.

Once the best model was selected based on Mean Absolute Percentage Error, we retrieved its predictions on the test set during each cross-validation iteration, then applied the Bravais-Pearson correlation to test if the predicted reading speed of the child in the Alouette test based on eye movement descriptors was positively correlated with measured reading speed.

### Result

### Analysis 1

For the four oculomotor tests, the best linear classifier (in terms of accuracy) was the Logistic regression. The best non-linear classifier was the SVM with an RBF kernel.

In **Tables 14 and 15** we present the score of each best model so that we can compare the ability of the descriptors extracted from each oculomotor test to predict dyslexia using the family of models that we have tested. Moreover, the accuracy of all the other trained models are presented in **Figures 20A to 20D****.**

The eye movement parameters extracted by AIDEAL can predict dyslexia by using the SVM and logistic regression classifiers with an accuracy of 71.57% and 70.2% when it was trained on the data from the meaningful reading test, 68.42% and 77.3% when it was trained on the REMOBI vergence dataset, 80.0% and 81.25% when it was trained on the REMOBI saccade dataset, and 80.0% and 81.25% when it was trained on the Alouette reading dataset.

The best SVM model in terms of sensitivity is the model trained on the data from the Alouette reading test with a score of 85.0%. The best logistic regression model in terms of sensitivity is also the model trained on the REMOBI saccade test with a score of 86.94%, demonstrating that the Alouette test could also be used to detect dyslexia.

The best model in terms of specificity is the model trained on saccades produced by the Remobi table with a specificity of 82.5%, which means that the Remobi saccade test could be used to detect the non-dyslexic population. Note that the performances we report are obtained without hyper parameter tuning. A larger dataset would likely allow even better performances by tuning the hyper parameters on a separate validation set.

**Table 14. Support Vector Machine scores for Analysis 1.**

| **Metric** | **Reading Alouette** | **Reading Meaningful** | **Remobi Saccade** | **Remobi Vergence** |
|---|---|---|---|---|
| accuracy | 80.0% | 71.57% | 80.0% | 68.42% |
| sensitivity | 82.5% | 54.29% | 82.5% | 67.5% |
| specificity | 77.5% | 76.67% | 77.5% | 69.17% |

**Table 15. Logistic regression scores for Analysis 1.**

| **Metric** | **Reading Alouette** | **Reading Meaningful** | **Remobi Saccade** | **Remobi Vergence** |
|---|---|---|---|---|
| accuracy | 81.25% | 70.2% | 81.25% | 77.3% |
| sensitivity | 85.0% | 62.38% | 80.0% | 80.0% |
| specificity | 77.5% | 76.67% | 82.5% | 74.17% |

To investigate the signification of the reported result, we implemented a label permutation test. For each dataset we have fitted the two selected models after having applied a random permutation to the labels of the entire dataset. We have repeated this procedure 1000 times.

Then, we have computed the probability of getting an accuracy at least equal to the scores presented in the **Tables 14 and 15** in order to estimate the significance of the reported result.

The significance of the reported results are presented in **Table 16** and the histogram of the scores of each model for each dataset are presented in **Figures 17** **and** **18****.**

**Table 16. Significance of reported results for Analysis 1.**

| **Model** | **Dataset** | ***p*-Value** |
|---|---|---|
| Logistic regression | Remobi saccade | 0.000 |
| | Remobi vergence | 0.000 |
| | Reading alouette | 0.000 |
| | Reading meaningfull | 0.002 |
| | Remobi saccade | 0.000 |
| | Remobi vergence | 0.003 |
| SVM | Reading alouette | 0.000 |
| RBF | Reading meaningfull | 0.000 |

### Analysis 2

The best model for predicting the meaningful reading speed from the eye movement parameters of the Remobi saccade test was the RBF SVM. The model achieved a mean absolute percentage error of 26.50%, and the predicted speed was correlated with the real speed of 0.46.

For the second task, which was predicting the meaningful reading speed from the eye movement parameters of the REMOBI Vergence test, the best model was the same as of the previous task. This model achieved a mean absolute percentage error of 16.78%, and the predicted speed was correlated with the real speed with a correlation coefficient of 0.56.

Finally, for the last task, which was predicting the meaningful reading speed from the eye movement parameters of the meaningful reading test itself, the best model was the same as the previous tasks. The model achieved a mean absolute percentage error of 22.2%, and the predicted speed was correlated with the real speed with a correlation coefficient of 0.56.

In **Figure 19****,** we present the correlation between true speed and the predicted speed of the three tasks. The accuracy and the mean percentage error for each of the four tasks are presented in **Table 17:**

**Table 17. Mean percentage error and correlation metrics for the analysis 2.**

| **Metric** | **Remobi Saccade** | **Remobi Vergence** | **Reading Meaningful** |
|---|---|---|---|
| correlation | 0.46 | 0.56 | 0.56 |
| mpe | 20.45% | 16.78% | 22.2% |

For each task, the eye movement parameters selected for at least 4 folds are presented in **Tables 18 and 19,** the correlation is computed on the base of the entire dataset.

**Table 18. The eye movement parameters selected for the Remobi saccade and vergence datasets.**

| **Model** | **Feature Parameters** | **Correlation** | **p-Value** |
|---|---|---|---|
| | Duration left | -0.20 | 0.055 |
| | Duration left std | -0.28 | 0.0062 |
| | Pvelocity left | -0.23 | 0.02616 |
| Remobi Saccade test | Pvelocity left std | -0.21 | 0.0449 |
| | Avelocity left | 0.25 | 0.0137 |
| | Avelocity left std | -0.26 | 0.0112 |
| | Duration right | -0.23 | 0.0248 |
| | Duration std right | -0.33 | 0.0013 |
| | Pvelocity right | -0.21 | 0.0445 |
| | Divergence Duration | -0.26 | 0.0115 |
| | Divergence Avelocity | 0.33 | 0.0012 |
| | Convergence Avelocity | 0.32 | 0.002 |
| | Convergence AVelocity | -0.31 | 0.0026 |
| Remobi Vergence test | CoV | | |
| | Convergence Drift 1 | 0.32 | 0.0018 |
| | Convergence Drift1 CoV | -0.28 | 0.0069 |
| | Convergence Drift 2 | 0.33 | 0.0014 |
| | Convergence Drift2 CoV | -0.25 | 0.0137 |

**Table 19. The eye movement parameters selected for the Alouette and meaningful lecture data sets.**

| **Model** | **Feature Parameters** | **Correlation** | ***p*-Value** |
|---|---|---|---|
| Meaningful lecture test | Reading Left PVelovity CoV | 0.53 | 0.000008 |
| | Reading Left Avelocity | 0.53 | 0.0000000 8 |
| | Reading Left AVelocity CoV | -0.50 | 0.0000005 |
| | Reading Left Drift CoV | -0.40 | 0.00007 |
| | Reading Right Amplitude | 0.47 | 0.000004 |
| | Reading Right Avelocity | 0.52 | 0.0000002 |
| | Reading Total Number Left Sacc | -0.47 | 0.000003 |
| | Reading Total Number Right Sacc | -0.54 | 0.0000000 005 |

### Discussion

ML has been applied to numerous fields including health and neuroscience. Research on the neurology of eye movements has been a pioneering field of cognitive neuroscience for almost a century. Nevertheless, the application of Machine or Deep Learning (DL) in the field of dyslexia and oculomotor movements is rather scarce. This could be related to the fact that eye movement researchers have already extensively characterized many spatio-temporal parameters of eye movements in relation to their specific functions and their neural substrates. Indeed, eye movement control is the best-understood sensorimotor system in neurosciences.

Yet, ML/DL can bring some new insights and advantages: first, such an approach is more data-driven. Second, such methods apply a variety of different models including linear and non-linear. Therefore, our approach was to apply ML on data to quantify more precisely the quality of such eye movement descriptors for dyslexia detection. To this end, we used the descriptors produced from four eye movement tests as input to common ML classifiers to compare their generalization performances.

A review of the existing literature in this field reveals that the few existing prior ML studies use a limited set of data applied to reading concerning only saccades and fixations (Chakraborty, V.; Sundaram, M. Machine learning algorithms for prediction of dyslexia using eye movement. J. Phys. Conf. Ser. 2020, 1427, 012012). Therefore, the present study aims to use ML to further the fields of neuroscience, dyslexia, and ML itself. First, in its very practice, our study unites the fields of computer science and neuroscience. Second, our study uses a complete data set carefully assembled from cohorts diagnosed with dyslexia by medical reference centers in France. Third, the data used covers almost the entire ensemble of physiologic eye movements we make to explore the three-dimensional space, namely, saccades and vergence eye movements to LED targets using the REMOBI device, and reading of two types of text: a meaningful text and the Alouette text used by orthophonists and psychologists in France to diagnose dyslexia. Another advantage of the study is that we have used many biologic descriptors of the eye movements (latency, peak, average velocity, amplitude, duration, coordination, drifts, disconjugacy) that are well established in the field of the neurology of eye movements. Such descriptors enable us to describe not only the eye displacement onset and offset of the saccade and fixation but also the trajectory of the movements themselves. The ML models, pre-processing, and post-evaluation methods were carefully done to provide plausible physiological results, uniting neuro-physiologic and ML expertise.

The results show that an ML approach on such data is useful and provides convincing power to determine dyslexic vs. non-dyslexic classifications. Further, the fact that we were able to create an ML approach that could classify dyslexic from non-dyslexic adolescents based on eye movements during reading is a novel result, shedding light on the importance of eye movements during reading, which is often overlooked by many language-oriented researchers who consider reading solely to be a language issue. It is remarkable that the power of classification is modulated according to text (senseless or meaningful); that the meaningless text stresses dyslexics' more fragile oculomotor system reveals how we can classify dyslexic's eye movements based on their deficits. Perhaps the most remarkable finding is that we were able to powerfully classify large saccades and vergence eye movements to LED targets into dyslexic and non-dyslexic categories. Finally, we were also able to predict reading speed based on eye movement descriptors from tests with LED targets, an approach that has not been previously seen. Our results based on ML classification confirm previous studies that have performed classic group comparison analysis (Kapoula, Z.; Bucci, M.P.; Jurion, F.; Ayoun, J.; Afkhami, F.; Brémond-Gignac, D. Evidence for frequent divergence impairment in French dyslexic children: Deficit of convergence relaxation or of divergence per se? Graefe's Arch. Clin. Exp. Ophthalmol. 2006, 245, 931-936 ; Bucci, M.P.; Bremond-Gignac, D.; Kapoula, Z. Poor binocular coordination of saccades in dyslexic children. Graefe's Arch. Clin. Exp. Ophthalmol. 2007, 246, 417-428). The present study confirms classification power with multiple linear and non-linear models with significant results. Clinically, this study also raises the possibility of a ML diagnostic help assistant, the development of which under progress.

Finally, we should address the physiologic significance of such results. Recall that eye movements are essential for reading during the learning process and exploration of the environment. There is ample evidence in the neuroscience field that eye movements are related to attention and cognition, most frequently referenced via the well-established theory of the motor basis of attention introduced by Rizolatti (Rizzolatti, G.; Riggio, L.; Dascola, I.; Umiltá, C. Reorienting attention across the horizontal and vertical meridians: Evidence in favor of a premotor theory of attention. Neuropsychologia 1987, 25, 31-40). Prior research from our team has expanded this theory to a broader motor basis that includes not only saccades as was introduced by Rizolatti but also vergence eye movements and accommodation (Morize, A.; Brémond-Gignac, D.; Daniel, F.; Kapoula, Z. Effects of Pure Vergence Training on Initiation and Binocular Coordination of Saccades. Investig. Ophthalmol. Vis. Sci. 2017, 57, 329-342 ; Kapoula, Z.; Morize, A.; Daniel, F.; Jonqua, F.; Orssaud, C.; Bremond-Gignac, D. Objective Evaluation of Vergence Disorders and a Research-Based Novel Method for Vergence Rehabilitation. Transl. Vis. Sci. Technol. 2016, 5, 8 ; Daniel, F.; Morize, A.; Bremond-Gignac, D.; Kapoula, Z. Benefits from Vergence Rehabilitation: Evidence for Improvement of Reading Saccades and Fixations. Front. Integr. Neurosci. 2016, 10, 33). As previously mentioned, eye movement control during reading is complex, binocular, and three-dimensional, involving the control of movements of the two eyes in multiple directions (horizontal and vertical) and in depth (vergence). Though the motor theory of visual attention is generally accepted in neurosciences, when it comes to reading, such physiologic approaches are rarely considered in favor of the predominantly phonologic and language-based approach.

Typical arguments against such a physiologic approach are that people with visual problems (for example, the elderly) can oftentimes still read well. Although this is often the case, senior readers who develop problems later in life can usually compensate for visual and motor control issues. People such as dyslexic adolescents, who experience these deficits from an early age during development, before coordination of oculomotor movements is truly learned and cemented, are unable to compensate for these deficits, which can have devastating consequences for reading throughout their lives.

### Conclusions

Our results demonstrate that eye movement parameters from the saccade and vergence Remobi tests can successfully predict both dyslexia diagnosis and reading speed; further studies with larger populations are of interest. The ML results applied to reading itself are highly dependent on the type of text read. The results from ML presented here are in full agreement with our prior studies, showing statistically significant differences in many eye movement parameters with the classic group. The ML approach provides quantification and prediction power, opening the possibility of creating an early diagnostic tool to detect dyslexia independent of reading.

### Example 10. Interest of assessing the visual strategies of children and teenagers with dys-disorders in front of the cookie theft image

### Objectives

The image of the BDAE cookie thief is a classic. We studied eye movements in front of this image in a population of dys- children and adolescents in order to characterize their specific visual strategies and the efficiency of their visual memory.

### Material / Patients and methods

400 dys- children and adolescents from 6 to 20 years old observed the image for 30 seconds. Their eye-scanning movements (fixation, saccades and vergences) were followed by eye-tracker Pupil Labs^{®} and the results were processed by AIDEAL^{®} from Orasis-Ear. In a second step, on a scale from 0 to 15, inspired by the work of Dr Bernard Croisile, we measured a free recall, then, an indexed recall of what they observed.

### Results

Visual strategies (fixation, saccades, and vergences) combined with recall scores show very clear specificities between dyspraxics (poor scanning; weak and sometimes false recalls), dyslexics and dysphasics (normal or rich scanning; normal recalls), ADHD (often out-of-picture scanning; variable recalls), autism spectrum disorders (rich scanning, surprising, sometimes avoidant; often rich recall favoring often innocuous details).

### Discussion - Conclusion

This simple and quick evaluation of the cookie thief allows us to propose a complementary diagnostic tool and, above all, a pedagogical support illustrating the way dys- children and adolescents function.

## Claims

1. A method for measuring binocular motricity parameters in an individual suffering from a learning disorder, comprising:
a1) submitting the individual to ocular motor tests comprising at least a reading test, a vergence test and a saccade test;
b1) recording the eye movements of the left eye and the right eye of the individual during each of the ocular motor tests; and
c1) calculating, using a digital processing device, the value of at least one binocular coordination parameter from the records obtained in step b1);
wherein the vergence test and the saccade test are conducted using a binocular motor stimulation device configured to specifically and physically stimulate vergences and saccades, comprising visual or audiovisual targets placed in real space at eccentricities and depths of a surface;
wherein the vergence test comprises 30 to 50 trials of:
• visually and/or audiovisually stimulating the individual at a point located at the eyes level, in the central axis between the left eye and the right eye, at a first distance, and
• visually and/or audiovisually stimulating the individual at another point located at the eyes level, in the central axis between the left eye and the right eye, at another distance calling for a convergence movement or a divergence movement, half of the trials calling for a convergence movement, the other half for a divergence movement, the trials calling for convergence and divergence movements being interleaved; and
wherein the saccade test comprises 30 to 50 trials of:
• visually and/or audiovisually stimulating the individual at a point located at the eyes level, in the central axis between the left eye and the right eye, and
• visually and/or audiovisually stimulating the individual at another point located at the eyes level, on the left or on the right of the previous point, half of the trials being on the left, the other half on the right, the trials on the left and on the right being interleaved.

2. The method according to claim 1, wherein the reading test of step a1) involves reading of a text of 10 to 20 lines and 200 to 300 words that does make sense and/or of a text of 10 to 20 lines and 200 to 300 words that does make no sense.

3. The method according to claim 1 or claim 2, wherein the vergence test of step a1) comprises 30 to 50 trials of:
• visually and/or audiovisually stimulating the individual at a point located at the eyes level, in the central axis between the left eye and the right eye, at a distance of 40±5 cm for a period varying from 1400ms to 2000ms, and
• visually and/or audiovisually stimulating the individual at another point located at the eyes level, in the central axis between the left eye and the right eye, at a distance of 20±5 cm calling for a convergence movement or at a distance of 70 to 150 cm calling for a divergence movement during 1500 to 2500 ms, preferably 2000 ms, half of the trials calling for a convergence movement, the other half for a divergence movement, the trials calling for convergence and divergence movements being interleaved.

4. The method according to any one of claims 1 to 3, wherein the saccade test of step a1) comprises 30 to 50 trials of:
• visually and/or audiovisually stimulating the individual at a point located at the eyes level, in the central axis between the left eye and the right eye, at a distance of 20, 40, 70 or 100 cm corresponding to a vergence angle of 17°, 9°, 5.3° or 3.72° for a period varying from 1400ms to 2000ms, and
• visually and/or audiovisually stimulating the individual at another point located at the eyes level, at 15° to 20° , preferably 20° of eccentricity on the left or on the right during 1500 to 2000 ms, preferably 2000 ms, half of the trials being on the left, the other half on the right, the trials on the left and on the right being interleaved.

5. The method according to any one of claims 1 to 4, wherein the vergence test, the saccade test or both the vergence and saccade tests of step a1) are performed twice, once using audiovisual stimulation and once using visual stimulation only.

6. The method according to any one of claims 1 to 5, wherein the individual has an accelerometer mounted on his/her head in step a1) and step b1) further comprises recording the individual's head accelerations during one or more of the ocular motor tests.

7. The method according to any one of claims 1 to 6, wherein step c1) of calculating the value(s) of at least one binocular coordination parameter comprises calculating, based on the vergence and saccade tests:
• duration of the initial phasic component of divergences,
• peak velocity or peak velocity of the initial phasic component of convergences and/or divergences,
• average velocity of the initial phasic component of convergences and/or divergences,
• total average velocity of convergences and/or divergences, preferably of convergences,
• amplitude of the 80 ms and/or 160 ms component of convergences and/or divergences, preferably of convergences,
• duration of the initial phasic component of right and/or left saccades,
• peak velocity or peak velocity of the initial phasic component of right and/or left saccades,
• average velocity of the initial phasic component of right and/or left saccades,
• disconjugacy 80 ms after right and/or left saccades, preferably after right saccades,
• disconjugacy 160 ms after right and/or left saccades, preferably after right saccades, or
• any combination thereof.

8. The method according to claim 7, wherein step c1) of calculating the value(s) of at least one binocular coordination parameter comprises calculating, based on the vergence and saccade tests:
• at least one vergence velocity parameter and at least one saccade velocity parameter, or
• at least one vergence duration parameter and at least one saccade duration parameter, or
• at least one saccade velocity parameter, and at least one post-saccadic disconjugacy parameter.

9. The method according to claim 7, wherein step c1) of calculating the value(s) of at least one binocular coordination parameter comprises calculating, based on the vergence and saccade tests:
i) average velocity of convergences, divergences, and left and right saccades,
ii) duration of convergences, divergences, and left and right saccades,
iii) disconjugacy 80 ms and/or 160 ms after right and left saccades, or after right saccades only,
iv) combination of i) and ii),
v) combination of i) and iii),
vi) combination of ii) and iii), and
vii) combination of i), ii) and iii).

10. The method according to any one of claims 1 to 9, wherein step c1) further comprises calculating average velocity of saccades during reading, disconjugacy 80 ms and/or 160 ms after right saccades during reading, number or proportion of regression saccades, and reading speed, and optionally further comprises calculating average amplitude of head rotation when using a head-mounted accelerometer during at least one ocular motor test.

11. A method for detecting intrinsic ocular motor abnormalities in an individual suffering from a learning disorder, comprising:
a2) providing previously obtained recordings of the eye movements of the left eye and the right eye of the individual during ocular motor tests comprising at least a reading test, a vergence test and a saccade test,
b2) calculating, using a digital processing device, the value(s) of at least one binocular coordination parameter from the records provided in step a2);
c2) comparing the value(s) calculated in step b2) to one or more reference value(s) obtained in one or more individual(s) not suffering from a learning disorder; and
d2) concluding to the presence of intrinsic ocular motor abnormalities in the individual suffering from a learning disorder if at least one binocular coordination parameter calculated in step b2) is significantly different from the one or more reference value(s) obtained in one or more individual(s) not suffering from a learning disorder;
wherein the vergence test and the saccade test from which the recordings are provided in step a2) have been conducted using a binocular motor stimulation device configured to specifically and physically stimulate vergences and saccades, comprising visual or audiovisual targets placed in real space at eccentricities and depths of a surface;
wherein the vergence test which recording is provided comprised 30 to 50 trials of:
• visually and/or audiovisually stimulating the individual at a point located at the eyes level, in the central axis between the left eye and the right eye, at a first distance, and
• visually and/or audiovisually stimulating the individual at another point located at the eyes level, in the central axis between the left eye and the right eye, at another distance calling for a convergence movement or a divergence movement, half of the trials calling for a convergence movement, the other half for a divergence movement, the trials calling for convergence and divergence movements being interleaved; and
wherein the saccade test which recording is provided comprised 30 to 50 trials of:
• visually and/or audiovisually stimulating the individual at a point located at the eyes level, in the central axis between the left eye and the right eye, and
• visually and/or audiovisually stimulating the individual at another point located at the eyes level, on the left or on the right of the previous point, half of the trials being on the left, the other half on the right, the trials on the left and on the right being interleaved.

12. The method according to claim 11, wherein the recordings provided in step a2) have been obtained as in step a1) of the method according to any one of claims 1 to 10.

13. The method according to claim 11 or claim 12, wherein:
• steps c2) further comprises steps c2') of comparing vergence and/or saccade parameters in vergence or saccade test performed using audiovisual stimulation or visual stimulation only, and
• step d2) further comprises steps d2') of concluding to the presence or absence of multisensorial integration difficulties in the individual based on the comparison of step c2').

14. The method according to any one of claims 11 to 13, wherein:
• steps c2) further comprises steps c2") of comparing the accelerations of the individual suffering from a learning disorder to reference accelerations of healthy individuals, and
• step d2) further comprises steps d2") of concluding to presence or absence of failure of complete development of eye head coordination and/or the presence of infra-clinic problems of binocular vision based on the comparison of step c2").

## Patentansprüche

1. Verfahren zur Messung von binokularen Motorikparametern bei einer Person, die an einer Lernstörung leidet, umfassend:
a1) Unterziehen der Person okulomotorischen Tests, die mindestens einen Lesetest, einen Vergenztest und einen Sakkadentest umfassen;
b1) Aufzeichnen der Augenbewegungen des linken und des rechten Auges der Person während jedes der okulomotorischen Tests; und
c1) Berechnen des Wertes von mindestens einem binokularen Koordinationsparameter unter Verwendung einer digitalen Verarbeitungsvorrichtung aus den in Schritt b1) erhaltenen Aufzeichnungen;
wobei der Vergenztest und der Sakkadentest unter Verwendung einer binokularen motorischen Stimulationsvorrichtung durchgeführt werden, die speziell und physikalisch Vergenzen und Sakkaden stimuliert und
visuelle oder audiovisuelle Ziele umfasst, die in dem realen Raum an Exzentrizitäten und Tiefen einer Oberfläche platziert sind;
wobei der Vergenztest aus 30 bis 50 Versuchen aus Folgendem besteht:
• visuellem und/oder audiovisuellem Stimulieren der Person an einem Punkt auf Augenhöhe, in der Mittelachse zwischen dem linken und dem rechten Auge, in einer ersten Entfernung,
und
• visuellem und/oder audiovisuellem Stimulieren der Person an einem anderen Punkt auf Augenhöhe, in der Mittelachse zwischen dem linken und dem rechten Auge, in einem anderen Abstand, der eine Konvergenz- oder Divergenzbewegung erfordert, wobei die Hälfte der Versuche eine Konvergenzbewegung und die andere Hälfte eine Divergenzbewegung erfordert, wobei die Versuche, die Konvergenz- und Divergenzbewegungen erfordern, miteinander abgewechselt werden; und
wobei der Sakkadentest aus 30 bis 50 Versuchen aus Folgendem besteht:
• visuellem und/oder audiovisuellem Stimulieren der Person an einem Punkt auf Augenhöhe in der Mittelachse zwischen dem linken und dem rechten Auge und
• visuellem und/oder audiovisuellem Stimulieren der Person an einem anderen Punkt auf Augenhöhe, links oder rechts von dem vorherigen Punkt, wobei die Hälfte der Versuche links, die andere Hälfte rechts und die Versuche links und rechts eingebunden sind.

2. Verfahren nach Anspruch 1, wobei der Lesetest von Schritt a1) das Lesen eines Textes von 10 bis 20 Zeilen und 200 bis 300 Wörtern einschließt, der einen Sinn ergibt, und/oder eines Textes von 10 bis 20 Zeilen und 200 bis 300 Wörtern, der keinen Sinn ergibt.

3. Verfahren nach Anspruch 1 oder Anspruch 2, wobei der Vergenztest in Schritt a1) aus 30 bis 50 Versuchen aus Folgendem besteht:
• visuellem und/oder audiovisuellem Stimulieren der Person an einem Punkt auf Augenhöhe, in der Mittelachse zwischen dem linken und dem rechten Auge, in einem Abstand von 40 ± 5 cm für einen Zeitraum von 1400 ms bis 2000 ms, und
• visuellem und/oder audiovisuellem Stimulieren der Person an einem anderen Punkt auf Augenhöhe, in der Mittelachse zwischen dem linken und dem rechten Auge, in einem Abstand von 20 ± 5 cm, der eine Konvergenzbewegung erfordert, oder in einem Abstand von 70 bis 150 cm, der eine Divergenzbewegung erfordert, über einen Zeitraum von 1500 bis 2500 ms, vorzugsweise 2000 ms, wobei die Hälfte der Versuche eine Konvergenzbewegung und die andere Hälfte eine Divergenzbewegung erfordert, wobei die Versuche, die Konvergenz- und Divergenzbewegungen erfordern, miteinander abwechselt werden.

4. Verfahren nach einem der Ansprüche 1 bis 3, wobei der Sakkadentest in Schritt a1) aus 30 bis 50 Versuchen aus Folgendem besteht:
• visuellem und/oder audiovisuellem Stimulieren der Person an einem Punkt auf Augenhöhe in der Mittelachse zwischen linkem und rechtem Auge in einem Abstand von 20, 40, 70 oder 100 cm, was einem Vergenzwinkel von 17°, 9°, 5,3° oder 3,72° für einen Zeitraum von 1400 ms bis 2000 ms entspricht, und
• visuellem und/oder audiovisuellem Stimulieren der Person an einem anderen Punkt auf Augenhöhe, bei 15° bis 20°, vorzugsweise 20° Exzentrizität nach links oder rechts für 1500 bis 2000 ms, vorzugsweise 2000 ms, wobei die Hälfte der Versuche links und die andere Hälfte rechts durchgeführt werden, wobei die Versuche links und rechts abwechselnd durchgeführt werden.

5. Verfahren nach einem der Ansprüche 1 bis 4, wobei der Vergenztest, der Sakkadentest oder sowohl der Vergenz- als auch der Sakkadentest von Schritt a1) zweimal durchgeführt werden, einmal unter Verwendung der audiovisuellen Stimulation und einmal nur unter Verwendung der visuellen Stimulation.

6. Verfahren nach einem der Ansprüche 1 bis 5, wobei ein Beschleunigungsmesser an dem Kopf der Person in Schritt a1) angebracht ist und Schritt b1) ferner das Aufzeichnen der Kopfbeschleunigungen der Person während eines oder mehrerer der okulomotorischen Tests umfasst.

7. Verfahren nach einem der Ansprüche 1 bis 6, wobei Schritt c1) zum Berechnen des/der Werte(n) mindestens eines binokularen Koordinationsparameters das Berechnen basierend auf den Vergenz- und Sakkadentests umfasst:
• Dauer der anfänglichen Phasenkomponente der Divergenzen,
• Spitzengeschwindigkeit oder Spitzengeschwindigkeit der anfänglichen Phasenkomponente von Konvergenzen und/oder Divergenzen,
• mittlere Geschwindigkeit der anfänglichen Phasenkomponente von Konvergenzen und/oder Divergenzen,
• Gesamtmittelgeschwindigkeit von Konvergenzen und/oder Divergenzen, vorzugsweise von Konvergenzen,
• Amplitude der 80-ms- und/oder 160-ms-Komponente von Konvergenzen und/oder Divergenzen, vorzugsweise von Konvergenzen,
• Dauer der anfänglichen Phasenkomponente der rechten und/oder linken Sakkaden,
• Spitzengeschwindigkeit oder die Spitzengeschwindigkeit der initialen phasischen Komponente der rechten und/oder linken Sakkaden,
• mittlere Geschwindigkeit der anfänglichen Phasenkomponente der rechten und/oder linken Sakkaden,
• Diskonjugation 80 ms nach rechten und/oder linken Sakkaden, vorzugsweise nach rechten Sakkaden,
• Diskonjugation 160 ms nach rechten und/oder linken Sakkaden, vorzugsweise nach rechten Sakkaden, oder
• jede Kombination davon.

8. Verfahren nach Anspruch 7, wobei Schritt c1) zum Berechnen des/der Werte(s) mindestens eines binokularen Koordinationsparameters das Berechnen basierend auf den Vergenz- und Sakkadentests Folgendes umfasst:
• mindestens einen Vergenzgeschwindigkeitsparameter und mindestens einen Sakkadengeschwindigkeitsparameter oder
• mindestens einen Parameter für die Vergenzdauer und mindestens einen Parameter für die Sakkadendauer oder
• mindestens einen Sakkadengeschwindigkeitsparameter und mindestens einen postsakkadelen Diskonjugationsparameter.

9. Verfahren nach Anspruch 7, wobei Schritt c1) zum Berechnen des/der Werte(s) mindestens eines binokularen Koordinationsparameters das Berechnen basierend auf den Vergenz- und Sakkadentests Folgendes umfasst:
i) durchschnittliche Geschwindigkeit der Konvergenzen, Divergenzen und linken und rechten Sakkaden,
ii) Dauer der Konvergenzen, Divergenzen und linken und rechten Sakkaden,
iii) Diskonjugation 80 ms und/oder 160 ms nach rechten und linken Sakkaden oder nur nach rechten Sakkaden,
iv) Kombination aus i) und ii),
v) Kombination aus i) und iii),
vi) Kombination aus ii) und iii), und
vii) Kombination aus i), ii) und iii).

10. Verfahren nach einem der Ansprüche 1 bis 9, wobei Schritt c1) ferner das Berechnen der durchschnittlichen Geschwindigkeit von Sakkaden während des Lesens, der Diskonjugation 80 ms und/oder 160 ms nach rechten Sakkaden während des Lesens, der Anzahl oder des Anteils von Regressionssakkaden und der Lesegeschwindigkeit umfasst und optional ferner das Berechnen der durchschnittlichen Amplitude der Kopfrotation bei Verwendung eines an dem Kopf angebrachten Beschleunigungsmessers während mindestens eines okularen Motoriktests umfasst.

11. Verfahren zum Nachweis von intrinsischen okulomotorischen Anomalien bei einer Person, die an einer Lernstörung leidet, umfassend:
a2) Bereitstellen zuvor erhaltener Aufzeichnungen der Augenbewegungen des linken und des rechten Auges der Person während okulomotorischer Tests, die mindestens einen Lesetest, einen Vergenztest und einen Sakkadentest umfassen,
b2) Berechnen, unter Verwendung einer digitalen Verarbeitungsvorrichtung, des/der Wert(e) mindestens eines binokularen Koordinationsparameters aus den in Schritt a2) bereitgestellten Aufzeichnungen;
c2) Vergleichen der in Schritt b2) berechneten Werte mit einem oder mehreren Referenzwerten, die bei einer oder mehreren Personen ermittelt wurden, die nicht an einer Lernstörung leiden; und
d2) Feststellen des Vorhandenseins von intrinsischen okulomotorischen Anomalien bei der Person, die an einer Lernstörung leidet, wenn mindestens ein in Schritt b2) berechneter binokularer Koordinationsparameter signifikant von dem einen oder den mehreren Referenzwerten abweicht, die bei einer oder mehreren Personen, die nicht an einer Lernstörung leiden, erhalten wurden;
wobei der Vergenztest und der Sakkadentest, aus denen die Aufzeichnungen in Schritt a2) bereitgestellt werden, unter Verwendung einer binokularen motorischen Stimulationsvorrichtung durchgeführt wurden, die so konfiguriert ist, dass sie spezifisch und physikalisch Vergenzen und Sakkaden stimuliert, umfassend visuelle oder audiovisuelle Ziele, die in realem Raum an Exzentrizitäten und Tiefen einer Oberfläche platziert sind;
wobei der Vergenztest, für den eine Aufzeichnung bereitgestellt wird, 30 bis 50 Versuche von Folgendem umfasste:
• visuellem und/oder audiovisuellem Stimulieren der Person an einem Punkt auf Augenhöhe, in der Mittelachse zwischen dem linken und dem rechten Auge, in einer ersten Entfernung,
und
• visuellem und/oder audiovisuellem Stimulieren der Person an einem anderen Punkt auf Augenhöhe, in der Mittelachse zwischen dem linken und dem rechten Auge, in einem anderen Abstand, der eine Konvergenz- oder Divergenzbewegung erfordert, wobei die Hälfte der Versuche eine Konvergenzbewegung und die andere Hälfte eine Divergenzbewegung erfordert, wobei die Versuche, die Konvergenz- und Divergenzbewegungen erfordern, miteinander abgewechselt werden; und
wobei der Sakkadentest, für den eine Aufzeichnung bereitgestellt wird, 30 bis 50 Versuche von Folgendem umfasste:
• visuellem und/oder audiovisuellem Stimulieren der Person an einem Punkt auf Augenhöhe in der Mittelachse zwischen dem linken und dem rechten Auge und
• visuellem und/oder audiovisuellem Stimulieren der Person an einem anderen Punkt auf Augenhöhe, links oder rechts von dem vorherigen Punkt, wobei die Hälfte der Versuche links, die andere Hälfte rechts und die Versuche links und rechts eingebunden sind.

12. Verfahren nach Anspruch 11, wobei die in Schritt a2) bereitgestellten Aufzeichnungen wie in Schritt a1) des Verfahrens nach einem der Ansprüche 1 bis 10 erhalten wurden.

13. Verfahren nach Anspruch 11 oder 12, wobei:
• Schritt c2) ferner Schritt c2') zum Vergleichen von Vergenz- und/oder Sakkadenparametern in einem Vergenz- oder Sakkadentest umfasst, der entweder mit audiovisueller Stimulation oder nur mit visueller Stimulation durchgeführt wird, und
• Schritt d2) ferner Schritt d2') des Schlussfolgerns über das Vorhandensein oder Fehlen von multisensorischen Integrationsschwierigkeiten bei der Person basierend auf dem Vergleich von Schritt c2') umfasst.

14. Verfahren nach einem der Ansprüche 11 bis 13, wobei:
• Schritt c2) ferner Schritt c2") des Vergleichens der Beschleunigungen der an einer Lernstörung leidenden Person mit den Referenzbeschleunigungen gesunder Personen umfasst, und
• Schritt d2) ferner Schritt d2") des Schlussfolgerns über das Vorhandensein oder Fehlen eines Versagens der vollständigen Entwicklung der Kopf-Augen-Koordination und/oder über das Vorhandensein infraklinischer Probleme der binokularen Sicht basierend auf des Vergleichs von Schritt c2") umfasst.

## Revendications

1. Procédé pour mesurer des paramètres de motricité binoculaire chez un individu souffrant d'un trouble de l'apprentissage, comprenant :
a1) la soumission de l'individu à des tests oculomoteurs comprenant au moins un test de lecture, un test de vergence et un test de saccade ;
b1) l'enregistrement des mouvements oculaires de l'œil gauche et de l'œil droit de l'individu lors de chacun des tests oculomoteurs ; et
c1) le calcul, à l'aide d'un dispositif de traitement numérique, de la valeur d'au moins un paramètre de coordination binoculaire à partir des enregistrements obtenus à l'étape b1) ;
dans lequel le test de vergence et le test de saccade sont effectués à l'aide d'un dispositif de stimulation motrice binoculaire configuré pour stimuler spécifiquement et physiquement les vergences et les saccades, comprenant des cibles visuelles ou audiovisuelles placées dans l'espace réel à des excentricités et des profondeurs d'une surface ;
dans lequel le test de vergence comprend 30 à 50 essais de :
• stimulation visuelle et/ou audiovisuelle de l'individu à un point situé au niveau des yeux, dans l'axe central entre l'œil gauche et l'œil droit, à une première distance,
et
• stimulation visuelle et/ou audiovisuelle de l'individu à un autre point situé au niveau des yeux, dans l'axe central entre l'œil gauche et l'œil droit, à une distance différente nécessitant un mouvement de convergence ou un mouvement de divergence, la moitié des essais nécessitant un mouvement de convergence, l'autre moitié un mouvement de divergence, les essais nécessitant des mouvements de convergence et de divergence étant entrelacés ; et
dans lequel le test de saccade comprend 30 à 50 essais de :
• stimulation visuelle et/ou audiovisuelle de l'individu à un point situé au niveau des yeux, dans l'axe central entre l'œil gauche et l'œil droit, et
• stimulation visuelle et/ou audiovisuelle de l'individu à un autre point situé au niveau des yeux, à gauche ou à droite du point précédent, la moitié des essais étant effectués à gauche, l'autre moitié à droite, les essais à gauche et à droite étant entrelacés.

2. Procédé selon la revendication 1, dans lequel le test de lecture de l'étape a1) consiste en la lecture d'un texte de 10 à 20 lignes et de 200 à 300 mots ayant du sens et/ou d'un texte de 10 à 20 lignes et de 200 à 300 mots n'ayant pas de sens.

3. Procédé selon la revendication 1 ou la revendication 2, dans lequel le test de vergence de l'étape a1) comprend 30 à 50 essais de :
• stimulation visuelle et/ou audiovisuelle de l'individu à un point situé au niveau des yeux, dans l'axe central entre l'œil gauche et l'œil droit, à une distance de 40±5 cm pendant une période variant de 1400 ms à 2000 ms, et
• stimulation visuelle et/ou audiovisuelle de l'individu à un autre point situé au niveau des yeux, dans l'axe central entre l'œil gauche et l'œil droit, à une distance de 20±5 cm nécessitant un mouvement de convergence, ou à une distance de 70 à 150 cm nécessitant un mouvement de divergence, pendant 1500 à 2500 ms, de préférence 2000 ms, la moitié des essais nécessitant un mouvement de convergence, l'autre moitié un mouvement de divergence, les essais nécessitant des mouvements de convergence et de divergence étant entrelacés.

4. Procédé selon l'une quelconque des revendications 1 à 3, dans lequel le test de saccade de l'étape a1) comprend 30 à 50 essais de :
• stimulation visuelle et/ou audiovisuelle de l'individu à un point situé au niveau des yeux, dans l'axe central entre l'œil gauche et l'œil droit, à une distance de 20, 40, 70 ou 100 cm correspondant à un angle de vergence de 17°, 9°, 5,3° ou 3,72° pendant une période variant de 1400 ms à 2000 ms, et
• stimulation visuelle et/ou audiovisuelle de l'individu à un autre point situé au niveau des yeux, à une excentricité de 15° à 20°, de préférence 20°, à gauche ou à droite, pendant 1500 à 2000 ms, de préférence 2000 ms, la moitié des essais étant effectués à gauche, l'autre moitié à droite, les essais à gauche et à droite étant entrelacés.

5. Procédé selon l'une quelconque des revendications 1 à 4, dans lequel le test de vergence, le test de saccade ou à la fois les tests de vergence et de saccade de l'étape a1) sont réalisés deux fois, une fois à l'aide d'une stimulation audiovisuelle et une fois à l'aide d'une stimulation visuelle uniquement.

6. Procédé selon l'une quelconque des revendications 1 à 5, dans lequel l'individu présente un accéléromètre monté sur sa tête à l'étape a1), et l'étape b1) comprend en outre l'enregistrement des accélérations de la tête de l'individu pendant un ou plusieurs des tests oculomoteurs.

7. Procédé selon l'une quelconque des revendications 1 à 6, dans lequel l'étape c1) de calcul de la ou des valeurs d'au moins un paramètre de coordination binoculaire comprend le calcul, sur la base des tests de vergence et de saccade :
• de la durée de la composante de phase initiale des divergences,
• de la vitesse maximale ou vitesse maximale de la composante de phase initiale des convergences et/ou divergences,
• de la vitesse moyenne de la composante de phase initiale des convergences et/ou divergences,
• de la vitesse moyenne totale des convergences et/ou divergences, de préférence des convergences,
• de l'amplitude de la composante de 80 ms et/ou 160 ms des convergences et/ou divergences, de préférence des convergences,
• de la durée de la composante de phase initiale des saccades vers la droite et/ou la gauche,
• de la vitesse maximale ou vitesse maximale de la composante de phase initiale des saccades vers la droite et/ou la gauche,
• de la vitesse moyenne de la composante de phase initiale des saccades vers la droite et/ou la gauche,
• de la disconjugaison 80 ms après des saccades vers la droite et/ou la gauche, de préférence après des saccades vers la droite,
• de la disconjugaison 160 ms après des saccades vers la droite et/ou la gauche, de préférence après des saccades vers la droite, ou
• toute combinaison de ceux-ci.

8. Procédé selon la revendication 7, dans lequel l'étape c1) de calcul de la ou des valeurs d'au moins un paramètre de coordination binoculaire comprend le calcul, sur la base des tests de vergence et de saccade :
• d'au moins un paramètre de vitesse de vergence et d'au moins un paramètre de vitesse de saccade, ou
• d'au moins un paramètre de durée de vergence et d'au moins un paramètre de durée de saccade, ou
• d'au moins un paramètre de vitesse de saccade, et d'au moins un paramètre de disconjugaison post-saccade.

9. Procédé selon la revendication 7, dans lequel l'étape c1) de calcul de la ou des valeurs d'au moins un paramètre de coordination binoculaire comprend le calcul, sur la base des tests de vergence et de saccade :
i) de la vitesse moyenne de convergences, de divergences et de saccades vers la gauche et la droite,
ii) de la durée des convergences, divergences et saccades vers la gauche et la droite,
iii) de la disconjugaison 80 ms et/ou 160 ms après des saccades vers la droite et la gauche, ou après des saccades vers la droite uniquement,
iv) de la combinaison de i) et ii),
v) de la combinaison de i) et iii),
vi) de la combinaison de ii) et iii), et
vii) de la combinaison de i), ii) et iii).

10. Procédé selon l'une quelconque des revendications 1 à 9, dans lequel l'étape c1) comprend en outre le calcul de la vitesse moyenne des saccades pendant la lecture, de la disconjugaison 80 ms et/ou 160 ms après les saccades vers la droite pendant la lecture, du nombre ou de la proportion de saccades de régression, ainsi que de la vitesse de lecture, et comprend en outre facultativement le calcul de l'amplitude moyenne de rotation de la tête lors de l'utilisation d'un accéléromètre monté sur la tête pendant au moins un test oculomoteur.

11. Procédé pour détecter des anomalies oculomotrices intrinsèques chez un individu souffrant d'un trouble de l'apprentissage, comprenant :
a2) la fourniture d'enregistrements antérieurement obtenus des mouvements oculaires de l'œil gauche et de l'œil droit de l'individu lors de tests oculomoteurs comprenant au moins un test de lecture, un test de vergence et un test de saccade,
b2) le calcul, à l'aide d'un dispositif de traitement numérique, de la ou des valeurs d'au moins un paramètre de coordination binoculaire à partir des enregistrements fournis à l'étape a2) ;
c2) la comparaison de la ou des valeurs calculées à l'étape b2) à une ou plusieurs valeurs de référence obtenues chez un ou plusieurs individus ne souffrant pas de trouble de l'apprentissage ; et
d2) la conclusion à la présence d'anomalies oculomotrices intrinsèques chez l'individu souffrant d'un trouble de l'apprentissage si au moins un paramètre de coordination binoculaire calculé à l'étape b2) est significativement différent des une ou plusieurs valeurs de référence obtenues chez un ou plusieurs individus ne présentant pas de trouble de l'apprentissage ;
dans lequel le test de vergence et le test de saccade à partir desquels les enregistrements sont fournis à l'étape a2) ont été effectués à l'aide d'un dispositif de stimulation motrice binoculaire configuré pour stimuler spécifiquement et physiquement les vergences et les saccades, comprenant des cibles visuelles ou audiovisuelles placées dans l'espace réel à des excentricités et des profondeurs d'une surface ;
dans lequel le test de vergence dont l'enregistrement est fourni comprenait 30 à 50 essais de :
• stimulation visuelle et/ou audiovisuelle de l'individu à un point situé au niveau des yeux, dans l'axe central entre l'œil gauche et l'œil droit, à une première distance, et
• stimulation visuelle et/ou audiovisuelle de l'individu à un autre point situé au niveau des yeux, dans l'axe central entre l'œil gauche et l'œil droit, à une distance différente nécessitant un mouvement de convergence ou un mouvement de divergence, la moitié des essais nécessitant un mouvement de convergence, l'autre moitié un mouvement de divergence, les essais nécessitant des mouvements de convergence et de divergence étant entrelacés ; et
dans lequel le test de saccade dont l'enregistrement est fourni comprenait 30 à 50 essais de :
• stimulation visuelle et/ou audiovisuelle de l'individu à un point situé au niveau des yeux, dans l'axe central entre l'œil gauche et l'œil droit, et
• stimulation visuelle et/ou audiovisuelle de l'individu à un autre point situé au niveau des yeux, à gauche ou à droite du point précédent, la moitié des essais étant effectués à gauche et l'autre moitié à droite, les essais à gauche et à droite étant entrelacés.

12. Procédé selon la revendication 11, dans lequel les enregistrements fournis à l'étape a2) ont été obtenus comme à l'étape a1) du procédé selon l'une quelconque des revendications 1 à 10.

13. Procédé selon la revendication 11 ou 12, dans lequel :
• l'étape c2) comprend en outre l'étape c2') de comparaison des paramètres de vergence et/ou de saccade dans un test de vergence ou de saccade réalisé à l'aide d'une stimulation audiovisuelle ou stimulation visuelle uniquement, et
• l'étape d2) comprend en outre l'étape d2') de conclusion à la présence ou l'absence de difficultés d'intégration multisensorielle chez l'individu sur la base de la comparaison de l'étape c2').

14. Procédé selon l'une quelconque des revendications 11 à 13, dans lequel :
• l'étape c2) comprend en outre l'étape c2") de comparaison des accélérations de l'individu souffrant d'un trouble de l'apprentissage à des accélérations de référence d'individus sains, et
• l'étape d2) comprend en outre l'étape d2") de conclusion à la présence ou l'absence d'un échec du développement complet de la coordination entre la tête et l'œil et/ou la présence de problèmes infracliniques de vision binoculaire sur la base de la comparaison de l'étape c2").
